# EUROPÄISCHE PATENTSCHRIFT

(11) **EP 4 172 899 B1**
(45) Veröffentlichungstag und Bekanntmachung des Hinweises auf die Patenterteilung: **29.10.2025**
(21) Anmeldenummer: 21786814.0
(22) Anmeldetag: 29.09.2021
(51) Int. Cl.: G06Q 10/06, G06Q 50/26, G16H 50/80

(54) **ELEKTRONISCHES SYSTEM ZUM TRACING VON NUTZERN, INSBESONDERE ZUR BEKÄMPFUNG EINER PANDEMIE**
ELECTRONIC SYSTEM FOR TRACING USERS, IN PARTICULAR FOR CONTROLLING A PANDEMIC
SYSTÈME ÉLECTRONIQUE DE SUIVI DES UTILISATEURS, EN PARTICULIER PERMETTANT DE LUTTER CONTRE UNE PANDÉMIE

(30) Priorität: 29.09.2020 DE 202020105574 U; 29.09.2020 DE 202020105575 U; 26.11.2020 EP 20210115
(43) Veröffentlichungstag der Anmeldung: 03.05.2023
(73) Patentinhaber: culture4life GmbH, 10117 Berlin (DE)
(72) Erfinder: HENNIG, Patrick, 10117 Berlin (DE)
(74) Vertreter: Richardt Patentanwälte PartG mbB
(86) Internationale Anmeldenummer: PCT/EP2021/076810
(87) Internationale Veröffentlichungsnummer: WO 2022/069551

(56) Entgegenhaltungen:
- CN-A- 111 444 279
- STRAUBINGER JUTTA: "Kontakterfassung in der Corona-Pandemie - Hochschule Trier entwickelt digitale Lösung", 27 August 2020 (2020-08-27), XP055854589, Retrieved from the Internet <URL:https://www.hochschule-trier.de/forschung/forschungsprofil/aktuelles/detail/kontakterfassung-in-der-corona-pandemie> [retrieved on 20211025]
- ASHRAF MUHAMMAD USMAN ET AL: "Detection and Tracking Contagion using IoT-Edge Technologies: Confronting COVID-19 Pandemic", 2020 INTERNATIONAL CONFERENCE ON ELECTRICAL, COMMUNICATION, AND COMPUTER ENGINEERING (ICECCE), IEEE, 12 June 2020 (2020-06-12), pages 1 - 6, XP033816840, DOI: 10.1109/ICECCE49384.2020.9179284
- ANDREW TZER-YEU CHEN: "How Fragmentation Can Undermine the Public Health Response to COVID-19", ARXIV.ORG, CORNELL UNIVERSITY LIBRARY, 201 OLIN LIBRARY CORNELL UNIVERSITY ITHACA, NY 14853, 14 September 2020 (2020-09-14), XP081762380

## Beschreibung

### Technisches Gebiet

Die Erfindung betrifft ein elektronisches System zum Tracing von Nutzern, insbesondere zum Tracing von Nutzern in Notfallszenarien wie z.B. bei der Bekämpfung einer Pandemie, wie zum Beispiel Covid-19, sowie ein entsprechendes digitales Speichermedium, auf dem ein Computerprogramm bzw. eine App gespeichert ist.

### Stand der Technik

Zur Begrenzung der Verbreitung von Covid-19 wird bislang so vorgegangen, dass bei Besuch einer öffentlichen Veranstaltung oder einer Gaststätte die betreffende Person ihre persönlichen Daten angeben muss. Die damit einhergehenden Dokumentationspflichten sind für die entsprechenden Veranstalter bzw. Betreiber von Restaurants aufwendig, zumal auch die datenschutzrechtlichen Bestimmungen eingehalten werden müssen.

Außerdem kann mit dem bisherigen Verfahren die Sicherheit der sensiblen, personenbezogenen Daten der Besucher wie z.B. Anschrift und Telefonnummer, nicht in dem Maße gewährt werden, die wünschenswert wäre, da die Betreiber von Restaurants und Kulturveranstaltungsräumen nicht darauf vorbereitet sind, sensible Daten einer Vielzahl von Personen sicher zu speichern. Außerdem ist bereits der Umstand, dass die Adressdaten der Besucher den Betreibern der Veranstaltungslokalitäten offengelegt werden müssen, ein Sicherheitsrisiko, da hierdurch der Betreiber der Lokalität zumindest bezüglich seiner eigenen Räumlichkeiten ein Tracing von Nutzern durchführen kann und er darüber hinaus die Identität der Gäste kennt. Demgegenüber werden erfindungsgemäß ein computerimplementiertes Verfahren, ein elektronisches System sowie einzelne Systemkomponenten geschaffen, welches ein weitgehend automatisiertes Tracing unter Wahrung von datenschutzrechtlichen Vorschriften gewährleistet.

### Technisches Problem und grundlegende Lösungen

Vor diesem Hintergrund besteht ein Bedarf an verbesserten Verfahren zum Tracing von Besuchern von Veranstaltungslokalitäten und anderen räumlichen Bereichen insofern, dass die vorangehend erwähnten Nachteile damit zumindest teilweise vermeidbar sind.

Der Erfindung liegt demgegenüber die Aufgabe zugrunde, ein verbessertes Verfahren und System zum Tracing von Besuchern zu schaffen, welches einen besseren Schutz sensibler personenbezogener Daten bietet.

Die der Erfindung zugrunde liegenden Aufgaben werden jeweils mit den Merkmalen der unabhängigen Patentansprüche gelöst. Ausführungsformen der Erfindung sind in den abhängigen Ansprüchen angegeben. Die im Folgenden aufgeführten Ausführungsformen sind frei miteinander kombinierbar, sofern sie sich nicht gegenseitig ausschließen.

In einem Aspekt betrifft die Erfindung ein elektronisches System mit Nutzergeräten, die jeweils einem Nutzer zugeordnet sind, und mit zumindest einem Server zum Tracing von Nutzern, die sich temporär in verschiedenen räumlichen Bereichen (Venues) aufhalten. Das Tracing der Nutzer wird für eine Entität vorgenommen. Das elektronische System ist zur Durchführung der folgenden Schritte konfiguriert:
- anlässlich einer Registrierung: Speicherung von Nutzerdaten der Nutzer auf dem Server , wobei die Nutzerdaten mit einem für den jeweiligen Nutzer individuellen User-Data-Key (also einem symmetrischen oder asymmetrischen öffentlichen Verschlüsselungsschlüssel für die Verschlüsselung der Nutzerdaten) verschlüsselt sind, wobei insbesondere eine User-ID des betreffenden Nutzers verknüpft mit den verschlüsselten Nutzerdaten auf dem Server gespeichert werden; und Speicherung der User-IDs und eines Nutzer-individuellen zur Entschlüsselung der verschlüsselten Nutzerdaten geeigneten User-Data-Entschlüsselungsschlüssels jeweils in demjenigen der Nutzergeräte, welches dem Nutzer zugeordnet ist, wobei vorzugsweise der User-Data-Key und/oder der User-Data-Entschlüsselungsschlüssel (der User-Data-Key und der User-Data Entschlüsselungsschlüssel können z.B. identische symmetrische Schlüssel sein oder ein asymmetrisches kryptographisches Schlüsselpaar bilden) geschützt in dem Nutzergerät des jeweiligen Nutzer gespeichert wird und dem Server nicht zugänglich ist;
- Im Zuge eines Check-ins eines der Nutzer gegenüber einem der Venues, Erzeugung einer Trace-ID durch das Nutzergerät des einen Nutzers, wobei die Trace-ID repräsentativ ist für eine Kombination aus User-ID des einen Nutzers und einer aktuellen Zeitinformation, und Speicherung der Trace-ID verknüpft mit der Venue-ID des einen Venue auf dem Server, wobei die Trace-ID als Zugriffsschlüssel gespeichert wird,
- Empfang einer Mitteilung beinhaltend die User-ID eines der Nutzer bezüglich welchem das Tracing durchgeführt werden soll; Beispielsweise kann die Mitteilung von einem Entitäts-Gerät der Entität gesendet und von dem Server empfangen werden; In einem anderen Beispiel kann die Mitteilung auch von einem Entitäts-Gerät der Entität gesendet, von dem Nutzergerät empfangen und an den Server weitergeleitet werden,
- Erzeugung von Kandidaten-Trace-IDs für den in der Mitteilung identifizierten Nutzer, wobei die Kandidaten-Trace-IDs mögliche zurückliegende Trace-IDs sind, die innerhalb eines vorgegebenen vergangenen Zeitraums liegen, und
- Verwendung der erzeugten Kandidaten-Trace-IDs als Suchkriterium durch den Server, um entsprechende auf dem Server für den vergangenen Zeitraum gespeicherte Trace-IDs aufzufinden, so dass die Venue-IDs derjenigen Venues gegenüber welchen der Nutzer während des vergangenen Zeitraums einen Check-in durchgeführt hat, ermittelt werden,
- Ausgabe der Venue-IDs der ermittelten Venues zur Identifizierung derjenigen Venues, die der Nutzer während des vergangenen Zeitraums besucht hat, durch den Server.

Dies kann vorteilhaft sein, da auf dem Server zwar die Nutzerdaten der Nutzer sowie die im Zuge von Check-in Ereignissen bei verschiedenen Venues die Venue-IDs verknüpft mit den Trace-IDs der verschiedenen Nutzer gespeichert sind, hierbei aber sichergestellt ist, dass weder der Betreiber des Venue noch der Betreiber des Servers ohne Autorisierung durch den Nutzer auf die auf dem Server gespeicherten Nutzerdaten zugreifen können, da für die Entschlüsselung der Nutzerdaten nötigen Entschlüsselungsschlüssel zum einen nutzerindividuell erstellt wurden und weil zum anderen diese Entschlüsselungsschlüssel geschützt in dem Nutzergerät gespeichert sind. Das bedeutet, ein Bekanntwerden des Nutzerdaten-Entschlüsselungsschlüssels eines Nutzers hat keine Auswirkungen auf die Sicherheit der Nutzerdaten der anderen Nutzer. Die Identitäten der beim Server registrierten Nutzer sind zwar sicher und zentral auf dem Server hinterlegt zusammen mit den Check-in Datensätzen dieser Nutzer. Da die Nutzerdaten, insbesondere die verschlüsselten Ausweis-daten des Nutzers, jedoch gemäß Ausführungsformen der Erfindung verschlüsselt sind, sodass sie der Venue nicht lesen kann (z.B. mit dem UserData-Verschlüsselungsschlüssel und/oder mit einem Entitätenschlüssel der Entität), und da die Trace-ID repräsentativ ist für eine Kombination aus User-ID des einen Nutzers und einer aktuellen Zeitinformation (und also keinen direkten Rückschluss auf die User-ID oder gar den vollen Namen des Nutzers erlaubt), kann weder der Server noch der Venue erkennen, welche Person den Venue besucht hat.

Gemäß mancher Ausführungsformen verschlüsselt der Venue die Check-in Daten (also insbesondere die Trace-ID und optional weitere Daten) mit seinem Venue-spezifischen Venue-Schlüssel. Die weiteren Daten, z.B. die Check-in Zeit, können ebenfalls verschlüsselt werden oder der verschlüsselten Trace-ID als Klartext angefügt sein. Falls der Venue vor dem Hochladen der Check-in Datensätze diese mit seinem Venue-Schlüssel verschlüsselt, kann der Server gemäß Ausführungsformen der Erfindung noch nicht einmal feststellen, wie viele Besucher ein Venue an einem bestimmten Zeitpunkt hatte (falls die Zeitstempel mitverschlüsselt werden). Erst wenn der Venue einer derartigen Auswertung zustimmt und seinen Venue-spezifischen Entschlüsselungsschlüssel bereitstellt, kann der Server die verschlüsselten Trace-IDs entschlüsseln und auswerten.

Ausführungsformen der Erfindung können somit den Vorteil haben, dass eine anonyme und sichere Übertragung und Speicherung von Kontaktinformationen ermöglicht wird, die ein Tracing einzelner Nutzer erlaubt, allerdings nur, wenn der Nutzer dem Tracing auch zustimmt. Besucher und Gäste von öffentlichen oder privaten Veranstaltungen werden dadurch in die Lage versetzt, dem Veranstalter anonym ihre Kontaktdaten mitzuteilen. Nur im Falle einer Infektion wird den örtlichen Gesundheitsbehörden mit Zustimmung des Gastes und vorzugsweise auch mit Zustimmung des Veranstalters der Zugriff auf die Kontaktdaten ermöglicht. Die Kontaktdaten können genutzt werden, um mögliche Ansprechpartner zu informieren.

Beispielsweise kann das System dazu verwendet werden, die Kontaktverfolgung für Veranstaltungsorte, Restaurants und andere räumliche Bereiche (Zugabteil, Flugzeug, Stadien, Konzerthallen, Gebäude, Räume, Konzertflächen im Freien etc.) sicherer und einfacher zu gestalten und insbesondere eine sichere und maschinelle und damit effiziente und skalierbare Tracing-Lösung bereitzustellen.

Das anonyme und sichere Tracing-System bzw. Verfahren kann in verschiedenen Anwendungsszenarien eingesetzt werden. Ein Anwendungsbeispiel ist das Tracing von Infizierten im Falle einer Pandemie wie z.B. Covid-19 oder anderer ansteckender Krankheiten. Ein anderes Anwendungsbeispiel ist die Nachverfolgung von Personen im Falle anderer Arten von Notfällen oder zur Aufklärung von Sicherheitsvorfällen. Beispielsweise könnte ein Besitzer eines räumlichen Bereiches mit hohem Besucherverkehr wie z.B. Zoos, Freizeitparks oder Bahnhöfen das System nutzen, um Besucher beim Betreten des räumlichen Bereiches im Zuge eines Check-ins am Eingang anonym zu registrieren. Das bedeutet, dass der Besitzer zwar die Check-in Daten (die Venue-ID verknüpft mit der Trace-ID der Nutzer und ggf. weiteren Daten) auf dem Server speichert, ohne dass der Besucher hierfür dem Besitzer oder Betreiber des Venue seine Identität offenlegen müsste. Falls jedoch auf dem Venue sich ein Sicherheitsvorfall ereignen sollte, z.B. Besucher verletzt worden sein sollten, kann der Betreiber der Venue in einem öffentlichen Aufruf darum bitten, sich bei der zuständigen Entität (z.B. zuständige Behörde oder kompetente, vertrauenswürdige Organisation) zu melden, um z.B. als Zeuge mitzuwirken (deren Anwesenheit durch Check-in und optional auch Check-Out Ereignisse und serverseitig hinterlegte Datensätze dokumentiert ist.

Nach Ausführungsformen sind die user-IDs Datenwerte, die nutzer-individuell erstellt werden, und die die Identität der Nutzer (insbesondere Name, Kontaktdaten, und/oder Adresse sowie persönliche Attribute wie Kontonummer, Geburtstag, Geburtsort etc.) nicht direkt explizit ohne Kenntnis weiterer Daten offenbaren.

Nach Ausführungsformen enthält die Trace-ID die User-ID nur in kodierter oder verschlüsselter Form, sodass der Server (und/oder andere Akteure wie z.B. der Venue oder sonstige Dritte) anhand der Trace-ID nicht die User-ID des zugehörigen Nutzers erkennen kann. Dies kann beispielsweise bedeuten, dass gemäß mancher Ausführungsformen die Trace-ID so gebildet bzw. berechnet wird, dass aus ihr die User-ID nicht rekonstruiert werden kann.

Nach Ausführungsformen sind die Trace-IDs Datenwerte, die die Identität der Nutzer (insbesondere Name, Kontaktdaten, und/oder Adresse sowie persönliche Attribute wie Kontonummer, Geburtstag, Geburtsort etc.) nicht offenbaren.

Nach Ausführungsformen der Erfindung wird die im Zuge des Check-ins erzeugte Trace-ID mittels einer Ableitfunktion berechnet, nämlich als Funktion der Kombination aus der zu repräsentierenden User-ID und der zu repräsentierenden aktuellen Zeitinformation beim Check-in. Jede der Kandidaten-Trace-IDs wird jeweils mittels der gleichen Ableitfunktion als Funktion der Kombination der in der Mitteilung enthaltenen User-ID und einer der Kandidaten-Zeitinformationen für einen möglichen in der Vergangenheit liegenden Check-in-Zeitpunkt, berechnet. Die Ableitungsfunktion umfasst vorzugsweise eine Rundung der aktuellen bzw. Kandidaten-Zeitinformation auf Zeitpunkte innerhalb vordefinierter Zeitintervalle, wobei die Zeitintervalle insbesondere Minuten oder Stunden oder Tage sind. Beispielsweise kann im Zuge des Check-ins die aktuelle Zeit vom Nutzergerät kontinuierlich erfasst werden, nach der Erfassung auf die volle Minute abgerundet werden, und dann zusammen mit der U-ser-ID als Input an die Ableitfunktion übergeben werden. Die Tracing-ID und die Venue-ID kann sodann mit optionalen weiteren Daten verknüpft und in Form eines QR-Codes kodiert und auf dem Nutzergerät zum Zwecke des Check-ins angezeigt werden. Das Nutzergerät ist dazu konfiguriert, automatisch eine neue Trace-ID und ggf. auch einen neuen QR-Code zu erzeugen, wenn das Zeitintervall (hier z.B. Minute) verstrichen ist und eine aktuellere Zeitinformation (aktuelle Minute, abgerundet) erhalten wurde. Wenn der alte QR-Code bis dahin nicht für den Check-in verwendet wurde, wird die darin enthaltene Trace-ID auch nicht auf dem Server gespeichert und wird vom Nutzergerät verworfen.

Dies kann vorteilhaft sein, da die Trace-ID zeitabhängig gebildet wird und es somit unmöglich ist, die Trace-IDs einzelnen Nutzern zuzuweisen oder auch nur nach Nutzer zu gruppieren. Der Betreiber des Venues und des Servers können also auf Basis der so gebildeten Trace-IDs noch nicht einmal anonyme Bewegungsprofile der einzelnen Besucher erstellen, da die Trace-IDs als Ableitung eines konstanten Datenwerts (User-ID) und eines sich regelmäßig ändernden Zeitwertes gebildet werden. Dies erhöht die Sicherheit der Nutzer vor unberechtigtem Tracing, sollte ein Angreifer sich Zugriff auf das Venue-Gerät, gegenüber welchem das Check-in vorgenommen wird und/oder Zugriff auf den Server verschaffen.

Nach Ausführungsformen der Erfindung ist das Nutzergerät dazu konfiguriert, im Zuge der Registrierung gegenüber dem Server einen Zufallswert ("userTracingSecret") zu erzeugen und zugriffsgeschützt ausschließlich im Nutzergerät zu speichern. Die im Zuge des Check-ins und/oder der Rückverfolgung eines Nutzers zur Erzeugung der Kandidaten-Trace-IDs verwendete Ableitfunktion berechnet die Trace-ID als Funktion (auch) des Zufallswerts ("userTracingSecret"). Das Nutzergerät ist dazu konfiguriert, von dem Benutzer, dem es zugewiesen ist, eine Bestätigung zur Übermittlung des Zufallswerts einzuholen, und erst in Antwort auf die Bestätigung den Zufallswert an den Server zu übermitteln (z.B. innerhalb eines Tracinggeheimnis-Transferobjekts) um diesem die Ableitung der Kandidaten-Trace-IDs zu ermöglichen. Zusätzlich oder alternativ dazu kann das Nutzergerät dazu konfiguriert sein, erst in Antwort auf die Bestätigung den Zufallswert an ein Entitäten-Gerät der Entität zu übermitteln (z.B. innerhalb eines Tracinggeheimnis-Transferobjekt) um der Entität die Ableitung der Kandidaten-Trace-IDs zu ermöglichen.

Je nach Ausführungsformen haben die auf dem Nutzergerät, dem Server und/oder dem Entitäten-Gerät installierten Anwendungsprogramme bzw. Apps die erforderlichen Schnittstellen, um direkt oder indirekt miteinander über ein Netzwerk Daten auszutauschen.

Dies kann vorteilhaft sein, dass ein Angreifer, der die User-ID errät und ggf. sogar die Ableitfunktion in Erfahrung bringt, dennoch nicht die auf dem Server gespeicherten Trace-IDs manipulieren kann dadurch, dass er die Trace-IDs eines Nutzers innerhalb eines bestimmten Zeitpunkts berechnen und manipulieren (löschen und/oder ersetzen oder durch "erfundene" Trace-IDs ergänzen) kann. Der Angreifer müsste nämlich zusätzlich auch in das Nutzergerät des Nutzers eindringen und den dort bei der Registrierung erzeugten und geschützt gespeicherten Zufallswerts ("userTracingSecret") in Erfahrung bringen, um für diesen Nutzer Trace-IDs berechnen und damit erkennen, erzeugen und manipulieren zu können.

Nach Ausführungsformen ist das Nutzergerät dazu konfiguriert, im Zuge der Registrierung gegenüber dem Server einen Zufallswert zu erzeugen, im Folgenden als userVerificationSecret bezeichnet, und zugriffsgeschützt ausschließlich im Nutzergerät zu speichern. Das Nutzergerät ist dazu konfiguriert, im Zuge des Check-ins gegenüber dem Venue das userVerificationSecret zu verwenden, um mit diesem einen Verifikationsdatenwert als Funktion der Trace-ID und der verschlüsselten Ausweis-Daten zu berechnen und verknüpft mit der Trace-ID auf dem Server zu speichern. Die Speicherung auf dem Server kann je nach Ausführungsform direkt erfolgen oder indirekt dadurch, dass die Check-in Daten zunächst von einem Venue-Gerät des Venues erfasst werden und das Venue-Gerät diese Daten dann an den Server weiterleitet, um sie serverseitig zu speichern.

Gemäß Ausführungsformen der Erfindung ist der Entität ein asymmetrisches kryptographisches Schlüsselpaar mit einem öffentlichen und einem privaten Entitäten-schlüssel zugeordnet. Das Nutzergerät des Nutzers ist dazu konfiguriert, im Zuge der Registrierung die bereits mit dem Nutzerdaten-Schlüssel verschlüsselten Nutzerdaten zusätzlich mit dem öffentlichen Entitätenschlüssel zu verschlüsseln.

Zusätzlich oder alternativ dazu ist das Nutzergerät des Nutzers dazu konfiguriert, im Zuge des Check-ins den für die Entschlüsselung dieser verschlüsselten Nutzerdaten erforderlichen Nutzerdaten-Entschlüsselungsschlüssel durch den öffentlichen Entitätenschlüssel zusammen mit der User-ID des Nutzers zu verschlüsseln, um verschlüsselte Ausweis-Daten zu erzeugen. Im Zuge des Check-ins speichert das Nutzergerät die verschlüsselten Ausweis-Daten verknüpft mit der Venue-ID des einen Venue auf dem Server. Diese Speicherung kann direkt erfolgen, wenn z.B. der Venue-Betreiber kein Venue-Gerät besitzt und das Nutzergerät den "Check-in" selbst vornimmt, indem es die Daten des Venues erfasst und zum Check-in verwendet und die Check-in Daten samt Venue-ID an den Server zur Speicherung übermittelt. Die Speicherung kann aber auch indirekt mittels des Venue-Geräts erfolgen, indem das Venue-Gerät die Check-in Daten des Nutzers erfasst, mit der Venue-ID des Venues, dem das Venue-Gerät zugeordnet ist, verknüpft, und zur Speicherung an den Server übermittelt.

Die Verwendung eines Entitäten-schlüssels, z.B. eines öffentlichen Verschlüsselungsschlüssels einer Behörde, z.B. des Gesundheitsamts zu den genannten Zwecken kann vorteilhaft sein, da das Amt, wenn es Zugriff auf den Server erhält, mit seinem zum öffentlichen Entitätenschlüssel korrespondierenden privaten Entitäten-schlüssel die Ausweisdaten, die verknüpft mit der Trace-ID gespeichert sind, entschlüsselt, um hierdurch die User-ID und den zugehörigen Nutzerdaten-Entschlüsselungsschlüssel zu erhalten. Mit dem Nutzerdaten-Entschlüsselungsschlüssel wiederum kann das Amt die verschlüsselten Nutzerdaten entschlüsseln, um die Identität des Nutzers zu erfahren.

Außerdem können diese Merkmale den Vorteil haben, dass die Tracing-Datensätze auf dem Server weniger Speicherplatz verbrauchen und die Tracing-Daten in einem graphischen Code wie z.B. einem QR-Code kodiert werden können: da die Nutzer-ID und der User-Data-Entschlüsselungsschlüssel (Nutzerdaten-Entschlüsselungsschlüssel) zur Erzeugung der verschlüsselten Ausweisdaten verschlüsselt werden, nicht aber die kompletten Nutzerdaten, die auch recht umfangreich sein können, können diese beim Check-in übertragenen Daten problemlos in einem QR Code kodiert werden, was einen automatische Check-in erleichtert.

Nach Ausführungsformen umfasst das elektronische System zumindest ein Entitäts-Gerät, das der Entität zugeordnet ist. Das Entitäts-Gerät ist dazu konfiguriert, nacheinander mehrere asymmetrische kryptographische Schlüsselpaare zu erzeugen, die der Entität zugewiesen sind, wobei bei Erzeugung eines neuen Entitäten-Schlüsselpaares das bisher gültige invalidiert wird, und wobei jedem der Entitäten-Schlüsselpaare eine Schlüsselversionsnummer zugewiesen ist.

Das zumindest eine Entitäts-Gerät ist dazu konfiguriert, den öffentlichen Schlüssel des erzeugten und aktuell gültigen Schlüsselpaares an die Nutzergeräte zu übertragen. Die Nutzergeräte sind dazu konfiguriert, immer nur den jüngsten übertragenen Entitätenschlüssel zur Erzeugung der mehrfach verschlüsselten Nutzerdaten und/oder zur Erzeugung der verschlüsselten Ausweis-Daten zu verwenden.

Bei den Entitäts-Geräten kann es sich z.B. um Datenverarbeitungsgeräte, z.B. Desktop-Computer oder Notebooks, oder sonstige Computer insb. von Mitarbeitern einer Behörde handeln oder um Datenverarbeitungsgeräte von Drittanbietern, die von der Entität zur Erfüllung einer Aufgabe der Entität genutzt wird.

Beispielsweise kann die Erzeugung eines neuen Entitäten-Schlüsselpaares täglich erfolgen, z.B. in Antwort auf das erstmalige Starten einer Entitäten-Tracing-Applikation auf einem der Entitäten-Geräte der Entität durch einen der Mitarbeiter, oder an einem vordefinierten Zeitpunkt (z.B. täglich um 7:00 Uhr morgens an einem vordefinierten Entitäten-Gerät).

Beispielsweise kann die Versionsnummer des jeweils verwendeten Entitäten-schlüssels verknüpft mit den mehrfach verschlüsselten Nutzerdaten und/oder verknüpft mit den verschlüsselten Ausweis-Daten auf dem Server gespeichert werden, sodass das Entitäten-Gerät anhand der Versionsnummer den zur Entschlüsselung geeigneten privaten Entitätenschlüssel mit der gleichen Versionsnummer identifizieren kann.

Die regelmäßige Ausstellung neuer Entitätenschlüsselpaare erhöht die Sicherheit, da ein Angreifer, der einen privaten Entitäten-Entschlüsselungsschlüssel in Erfahrung gebracht haben sollte, mit diesem die allermeisten Check-in bzw. Ausweisdaten auf dem Server nicht entschlüsseln könnte, da diese mit einem anderen (z.B. tagesaktuellen) Entitätenschlüssel verschlüsselt sind. Ohne Kenntnis der Schlüssel-ID wüsste der Angreifer auch nicht, welche Check-in Datensätze er mit dem in Erfahrung gebrachten Entschlüsselungsschlüssel entschlüsseln könnte.

Nach Ausführungsformen der Erfindung besteht das zumindest eine Entitäts-Gerät aus einer Vielzahl von Entitätsgeräten. Dasjenige der Entitäts-Geräte, welches das aktuelle asymmetrische Entitäten-Schlüsselpaar erzeugt, sendet den privaten Schlüssel des aktuell erzeugten asymmetrischen Entitäten-Schlüsselpaares in verschlüsselter Form an alle anderen der Entitäts-Geräte über ein Netzwerk, z.B. das Internet und/oder ein Intranet. Jedes der Entitäts-Geräte ist dazu konfiguriert, eine Mitteilung eines Notfalls mit Bezug zu einem der Nutzer zu erzeugen und an den Server zu senden. Zusätzlich oder alternativ dazu ist das Entitäts-Gerät dazu konfiguriert, mittels eines der bisher empfangenen privaten Entitätenschlüssel die verschlüsselten Nutzerdaten und/oder die verschlüsselten Ausweis-Daten zu entschlüsseln.

Dies kann vorteilhaft sein, da hierdurch sichergestellt ist, dass verschiedene Mitarbeiter der Entität, die verschiedene Entität-Geräte nutzen, dennoch an jedem Tag den gleichen privaten Entitätenschlüssel zur Entschlüsselung der Daten von Nutzern besitzen, bezüglich welcher ein Notfall (z.B. Positiv-Test im Pandemiefall) vorliegt. Somit wird eine Tracing-Lösung bereitgestellt, die sicher und skalierbar ist, da sie eine dezentrale, mehrere Entität-Geräte umfassende IT-Infrastruktur aufbaut, welche regelmäßig (insb. täglich) neu erzeugte Entitäten-Schlüsselpaare zur Ver- und Entschlüsselung von Nutzerdaten und/oder Ausweis-Daten beruht.

Nach Ausführungsformen der Erfindung umfasst das elektronische System mehrere Venue-Geräte. Jedem der Venues ist ein oder mehrere der Venue-Geräte zugewiesen.

Beispielsweise kann es sich bei dem Venue um ein Lokal mit mehreren Eingängen handeln, wobei an jedem Eingang ein Venue-Gerät für den automatischen Check-in und optional auch für den automatischen Check-out von Gästen angebracht ist. Gemäß einem anderen Beispiel ist der Venue ein Zug oder Zugabteil oder ein Flugzeug oder ein einzelner Sitz eines Flugzeugs und an den Ein- und/oder Ausgängen der jeweiligen Venues ist mindestens ein Venue-Gerät angebracht.

Nach Ausführungsformen der Erfindung ist das Nutzergerät dazu konfiguriert, im Zuge des Check-Ins die eigene aktuelle Position zu erfassen. Beispielsweise kann dies dadurch geschehen, dass das Nutzergerät mittels eines GPS-Sensors die eigene Position mittels GPS-Koordinaten bestimmt. In diesem Fall wird die Position des Nutzergeräts in absoluter und expliziter Weise ermittelt. Es ist aber auch möglich, dass die Positionsinformation bezüglich der aktuellen Position des Nutzergeräts nur implizit ermittelt wird, z.B. durch Empfang von WLAN-Signalen, die vom Nutzergerät zur WLAN-basierten Ortung bzw. Ortsbestimmung verwendet werden. WLANbasierte Ortung ist ein Verfahren zur Bestimmung des Standorts des Empfängers. Das Verfahren beruht auf Lateration und berechnet die eigene Position anhand von WLAN-Ausbreitungsmustern. Gerade in Ballungsgebieten senden eine Vielzahl von WLAN-Stationen. Diese Signale stammen von kommerziellen Hotspots, Firmennetzwerken oder von privaten Heimnetzwerken. Die Kenntnis über den Standort dieser Netzwerke (Router) erlaubt so die Berechnung des eigenen Standortes. Je mehr Netzwerksignale empfangen werden, desto exakter kann eine Lateration zur Berechnung des eigenen Standortes erfolgen. Apple verwendet das Verfahren in neueren Mobiltelefonmodellen (iPhone) auf Basis der Technik der Firma Skyhook Wireless. Freie Lösungen sind OpenWLANMap und Mozilla Location Service. Alternativ zur WLAN-basierten Ortsbestimmung kann das System auch dazu konfiguriert sein, ein Nahfeldsignal von einem Sender des Venue zu empfangen und auszuwerten, um eine in dem Nahfeldsignal codierte Venue-ID zu ermitteln. Die zur Ortsbestimmung verwendete Venue-ID muss nicht identisch sein zu der bei dem Server hinterlegten Venue-ID, sie kann ein beliebiger Datenwert sein, der einem Venue eineindeutig zugewiesen ist. Der Empfang eines Nahfeldsignals in dem eine Venue-ID codiert ist fungiert als impliziter Nachweis, dass das Nutzergerät beim Check-in sich in räumlicher Nähe zu dem Venue befindet, da eine hinreichende räumliche Nähe zum Venue Voraussetzung dafür ist, die Venue-ID über ein Nahfeldsignal (z.B. Bluetooth oder ein beliebiger anderer Nahfeldkommunikationsstandard, insb. Funkstandard) empfangen wurde. Das Nahfeldsignal kann z.B. von einem entsprechenden Sender emittiert werden, der von dem Venue am Eingangsbereich zum Venue aufgestellt wurde.

Im Zuge des Check-ins prüft das System gemäß manchen Ausführungsformen, ob das Nutzergerät innerhalb einer maximalen Distanz von der Position des Venues entfernt ist, und führt den Check-in nur dann durch, wenn das Nutzergerät innerhalb der maximalen Distanz von der Position des Venues entfernt liegt.

Beispielsweise kann diese Prüfung vom Venue bzw. einem Datenverarbeitungsgerät des Venue durchgeführt werden. In diesem Fall sind dem Venue bzw. dessen Datenverarbeitungsgerät Referenzdaten bekannt (zugänglich gespeichert), die mit der aktuellen Position bzw. Positionsinformation des Nutzergeräts verglichen werden können, um hinreichende räumliche Nähe zwischen Nutzergerät und Venue feststellen zu können. Falls keine hinreichende Nähe festgestellt wird, wird der Check-in Vorgang abgebrochen.

Gemäß einer alternativen Ausführungsform wird die Prüfung von dem Server durchgeführt. Beispielsweise kann der Server die vom Nutzergerät beim Check-in erfassten Positionsinformation zusammen mit der Venue-ID, der Trace-ID und optional weiteren Check-in Daten von dem Nutzergerät erhalten und diese mit Positionsdaten des Venues vergleichen, die verknüpft mit der Venue-IDs dieses Venues in dem Server gespeichert sind. Gemäß einem weiteren Beispiel sind die Positionsinformationen bezüglich der Position des Venues (z.B. GPS-Daten, Venue-ID zur Ortsbestimmung, WLAN-Name, etc) nicht auf dem Server hinterlegt, sondern werden bei jedem Check-in erneut übertragen, um dem Server die Prüfung zu ermöglichen. Falls keine hinreichende Nähe festgestellt wird, wird der Check-in Vorgang vom Server abgebrochen.

Nach Ausführungsformen der Erfindung umfasst die Erfassung der aktuellen Position des Nutzergeräts eine Erfassung von GPS-Daten des Nutzergeräts mittels eines GPS-Sensors des Nutzergeräts. Die von dem Nutzergerät ermittelten GPS-Daten werden mit Positionsdaten des Venues verglichen, um festzustellen, ob das Nutzergerät beim Check-in innerhalb der maximalen Distanz von dem Venue entfernt ist.

Nach einer alternativen Ausführungsform umfasst die Erfassung der aktuellen Position des Nutzergeräts eine Erfassung eines WLAN-Identifikators eines lokal vorhandenen WLAN-Netzes. Der von dem Nutzergerät ermittelte WLAN-Identifikator fungiert bei der Prüfung als Nachweis, dass das Nutzergerät beim Check-in innerhalb der maximalen Distanz von dem Venue entfernt ist.

Nach einer alternativen Ausführungsform umfasst die Erfassung der aktuellen Position des Nutzergeräts eine Erfassung eines Nahfeldsignals von einem Nahfeldsignalsender des Venues. Das Nahfeldsignal codiert einen Identifikator des Venues. Der von dem Nutzergerät über das Nahfeldsignal ermittelte Identifikator des Venues fungiert bei der Prüfung als Nachweis, dass das Nutzergerät beim Check-in innerhalb der maximalen Distanz von dem Venue entfernt ist.

Diese zusätzliche Prüfung bezüglich hinreichender räumlicher Nähe von Nutzergerät und Venue beim Check-in kann vorteilhaft sein, da hierdurch sichergestellt wird, dass Nutzer nur dann erfolgreich bei einem Venue einchecken können, wenn diese sich auch wirklich in der Nähe des Venues befinden. Beispielsweise kann dadurch verhindert werden, Personen, sie in Besitz eines Bildes von einem QR-Code eines Venues gelangt sind, mit Hilfe dieses Bildes von einem entfernten Ort aus einen Check-in durchzuführen, z.B. um sich ein Alibi zu verschaffen oder um ein Tracing von Besuchern eines Venues durch massenhafte "Fake-Check-ins" zu erschweren. Somit erhöht diese Plausibilitätsprüfung, ob ein Nutzer beim Check-in auch tatsächlich am Veranstaltungsort ist, die Sicherheit des Systems.

Gemäß einer weiteren Ausführungsform sind die Venue-IDs der bei dem Server registrierten Venues auf dem Server verknüpft mit einer aus einer Vielzahl von vordefinierten Gefahrenstufen gespeichert. Die Gefahrenstufe hängt von der Art des Ereignisses ab, für das das Tracing durchgeführt wird, und von verschiedenen den Venue betreffenden Faktoren. Im Falle einer Pandemie können einige der folgenden Faktoren die Gefahrenstufe beeinflussen: Das Vorhandensein und die Güte/Stärke von Belüftungseinrichtungen (Fenster, Klimaanlagen, Luftreiniger), die verfügbare Fläche pro Gast, die Nutzungsart des Venues (für Konzerte, Sport, Innengastronomie, Außengastronomie, etc.). Dieses Merkmal erlaubt es der Entität, z.B. einem Gesundheitsamt, die Besucher eines Venues, sofern diese damit einverstanden sind und ihre Nutzer-ID und optional auch den fraglichen, zu tracenden Zeitraum der Entität gegenüber willentlich offenlegen, in unterschiedlichen Abstufungen zu warnen je nach Gefahrenstufe des jeweils besuchten Venues. Hierfür erzeugt der Server für die Nutzer-ID eines Nutzers, der sein Ansteckungsrisiko z.B. während der letzten 7 Tage in Erfahrung bringen möchte, alle Trace-IDs für dessen Nutzer-ID und die vordefinierten Zeitintervalle innerhalb der 7 Tage. Diese Trace-IDs werden als Zugriffsschlüssel verwendet um in einer Datenbank diejenigen Venues zu identifizieren, mit denen eine identische Trace-ID verknüpft gespeichert ist. Die Venue-ID wiederum kann gemäß Ausführungsformen mit einer Gefahrenstufe verknüpft gespeichert sein. Falls während des fraglichen Zeitraums für einen oder mehrere der von dem interessierten Nutzer besuchten Venues Informationen vorliegen, dass ein Infizierter bzw. Positiv-Getesteter den Venue zur gleichen Zeit besucht hat, so kann für den interessierten Nutzer nicht nur rasch ermittelt werden, dass beim Besuch eines oder mehrerer bestimmter Venues möglicherweise eine Infektion stattgefunden haben könnte, sondern kann optional anhand der Gefahrenstufen der Venues auch automatisch ermittelt werden, wie hoch das Risiko für die Übertragung ist. Somit kann eine automatisierte und dennoch sehr exakte Abschätzung des individuellen Infektionsrisikos durchgeführt werden für jeden Nutzer, der hierfür der Entität seine Nutzer-ID bekannt gibt.

Gemäß Ausführungsformen ist der Server dazu konfiguriert, zu erkennen, wenn eine vordefinierte Mindestanzahl von Nutzern, die sich als Covid-19-positiv-getestet bei der zuständigen Entität (z.B. Gesundheitsamt) gemeldet und ihre Nutzer-ID für das Tracing bereitgestellt haben, innerhalb eines vordefinierten Zeitraums den gleichen Venue besucht haben. Dies könnte einen Hinweis auf ein Super-Spreading-Ereignis bei diesem Venue sein. Dies kann es dem Gesundheitsamt ermöglichen, den Venue zu kontaktieren und diesen auf das Ereignis hinzuweisen, sodass dieser ggf. durch Aushänge seine Besucher dazu auffordern kann, sich bei dem Gesundheitsamt zu melden, falls sie im fraglichen Zeitraum Gast des Venues waren. Anstatt für das Tracing von Covid-19 Infektionen kann das System auch für andere Notfallsituationen und andere Entitäten verwendet werden.

Nach einer Ausführungsform ist die Venue-Applikation, also ein Anwendungsprogramm, das auf einem dem Venue-Betreiber zugewiesenen Datenverarbeitungsgerät instanziiert ist, mit einer auf dem Nutzergerät instanziierten Nutzerstatus-App interoperabel und ist dazu konfiguriert, beim Check-In des Nutzers nutzerbezogene Daten automatisch zu empfangen und im Zuge des Check-ins auszuwerten, sofern der Nutzer dem Auslesen dieser Daten zuvor zugestimmt hat. Beispielsweise kann es sich bei der Nutzerstatus-App um eine Applikation handeln, die den Impfstatus des Nutzers in Bezug auf eine Infektionskrankheit wie z.B. Covid spezifiziert. Hierdurch wird es dem Venue-Betreiber ermöglicht, den Check-In Vorgang noch effizienter und sicherer zu machen.

Nach Ausführungsformen der Erfindung führt der Nutzer den Check-in gegenüber einem der Venue-Geräte durch, wobei das eine Venue-Gerät eine Kommunikationsschnittstelle zur direkten Kommunikation mit dem Nutzergerät eines eincheckenden Nutzers beinhaltet. Das Nutzergerät und das eine Venue-Gerät sind so konfiguriert, dass im Zuge des Check-ins Folgendes erfolgt:
- Erzeugung des Check-in Datenpakets durch das Nutzergerät, wobei das Check-in Datenpaket die Trace-ID und vorzugweise auch die verschlüsselten Ausweis-daten kodiert;
- Übertragung des Check-in Datenpakets vom Nutzergerät über die direkte Kommunikationsschnittstelle an das Venue-Gerät; und
- Dekodieren des empfangenen Check-in Datenpakets durch das Venue-Gerät, um die Trace-ID und vorzugsweise auch die verschlüsselten Ausweisdaten des eincheckenden Nutzers zu erhalten und diese verknüpft mit der Venue-ID des Venue, dem das Venue-Gerät zugewiesen ist, auf dem Server zu speichern;

Bei der direkten Kommunikationsverbindung kann es sich z.B. um eine Nahfeld-Kommunikationsverbindung oder um einen optischen Datenübertragungskanal handeln. Die Datenübertragung über den optischen Datenübertragungskanal umfasst eine Erfassung eines graphischen Codes, der das Check-in Datenpaket kodiert und auf einer Anzeige des Nutzergeräts angezeigt wird, durch einen optischen Sensor des Venue-Geräts. Das Venue-Gerät dekodiert die in dem Code (z.B. QR-Code oder Barcode) enthaltenen Daten, darunter z.B. die Trace-ID und optional weitere Daten wie z.B. die verschlüsselten Ausweisdaten, Verifikationswerte, QR-Code-Versionsnummer, Zeitstempel des Check-ins und/oder weitere Daten, und speichert diese verknüpft mit der in dem Venue-Gerät hinterlegten Venue-ID auf dem Server. In einer Ausführungsform ist das Venue-Gerät das Smartphone des Betreibers des Venues, auf welchem z.B. eine Venue-App eines Tracing-Dienstanbieters installiert ist, die die Venue-Gerät-seitigen Schritte des Tracingverfahrens implementiert.

Bei der Nahfeld-Kommunikationsverbindung kann es sich z.B. um eine Funkverbindung, eine Infrarot-Verbindung, eine Bluetooth-Verbindung oder andere Verbindungsformen im Nahfeld des Venue-Geräts handeln.

Diese Ausführungsvariante kann vorteilhaft sein, da geeignete Venue-Geräte den Check-in Prozess stark vereinfachen und beschleunigen können und hierbei eine nahtlose Integration der erfassten Check-in Daten in das Tracingsystem ohne Zusatzaufwand für den Besucher oder Venue-Betreiber ermöglicht wird.

Gemäß einer anderen Ausführungsform umfasst das elektronische System zumindest ein Trägerobjekt mit einem graphischen Venue-Code eines der Venues. Der Venue-Code kodiert eine Venue-ID, unter welcher dieser Venue bei dem Server registriert ist. Der Nutzer nimmt den Check-in gegenüber diesem Venue vor, wobei das Nutzergerät so konfiguriert ist, dass im Zuge des Check-ins Folgendes erfolgt:
- Erfassung des graphischen Venue-Codes mit einer Kamera des Nutzergerätes; und
- Dekodierung des Venue-Codes durch das Nutzergerät, um die Venue-ID des Venues zu erhalten.

Die Übertragung der Trace-ID und vorzugsweise auch der Ausweisdaten des eincheckenden Nutzers erfolgt an den Server durch das Nutzergeräts, wobei hierbei auch die dekodierte Venue-ID verknüpft mit der Trace-ID auf dem Server gespeichert wird. Bei dem Venue-Code vorzugsweise um einen QR-Code oder Barcode handelt, der auf einem Aufdruck oder einer Digitalanzeige sichtbar ist, handelt und/oder wobei es sich bei dem Trägerobjekt vorzugsweise handelt um einen Tischaufsteller oder ein Türschild zu einem Fahrzeugwagen, Fahrzeugabteil, Gebäude oder Zimmer handelt.

Dies kann vorteilhaft sein, da der Betreiber des Venues kein eigenes Venue-Gerät vorhalten oder installieren muss, um den Besuchern das sichere Tracingverfahren nach Ausführungsformen der Erfindung zur Verfügung zu stellen. Es ist beispielsweise ausreichend, dass der Betreiber eines Restaurants Tischaufsteller auf den Tischen platziert, wobei auf jedem Tischaufsteller die Venue-ID der Gaststädte und optional weitere Daten (wie z.B. Tischnummer) in Form eines QR-Codes kodiert ist. Ein Gast muss also nur mit der Kamera seines Nutzergerätes den QR-Code auf dem Tischaufsteller erfassen, um den oben beschriebenen "Check-in Prozess" innerhalb des gleichen Nutzergeräts durchzuführen. Beispielsweise können die in dem Venue-Code kodierten Daten vom Nutzergerät verwendet werden, ein simuliertes Venue-Gerät mit der ID und ggf. weiteren Daten des Venues zu "personalisieren" und sodann den Check-in mit diesem simulierten Venue-Gerät durchzuführen. Die Trace-ID wird verknüpft mit der Venue-ID und ggf. weiteren Daten von dem Nutzergerät direkt zum Server zur Speicherung auf dem Server übermittelt.

Nach Ausführungsformen der Erfindung ist das elektronische System so konfiguriert, dass von dem Nutzergerät des einen der Nutzer oder von dem Venue-Gerät eine Check-out Zeit an den Server übertragen wird, wenn der Nutzer den Venue verlässt. Die Check-out Zeit wird der zuvor für diesen Nutzer empfangenen Trace-ID zugeordnet, so dass für den Nutzer der Aufenthaltszeitraum an dem Venue in Form einer Kombination der Check-in Zeit und der Check-Out Zeit gespeichert wird.

Nach Ausführungsformen der Erfindung ist das elektronische System konfiguriert zur:
- Erzeugung eines Tracinggeheimnis-Transferobjekts durch das Nutzergerät, wobei das Tracinggeheimnis-Transferobjekt zumindest die User-ID und den Nutzerdaten-Entschlüsselungsschlüssel des Nutzers, dem das Nutzergerät zugewiesen ist, enthält;
- Verschlüsselung des Tracinggeheimnis-Transferobjekts mit einem öffentlichen Schlüssel der Entität durch das Nutzergerät, wobei es sich bei dem öffentlichen Schlüssel insbesondere um den jüngsten aus einer Reihe nacheinander erzeugter öffentlicher Entitätenschlüssel handelt; beispielsweise kann es sich bei dem Entitätenschlüssel um den öffentlichen Teil des für den aktuellen Tag gültigen Entitäten-Schlüsselpaares handeln;
- Übertragung des verschlüsselten Tracinggeheimnis-Transferobjekts über ein Netzwerk von dem Nutzergerät an den Server;
- In Antwort auf den Erhalt des verschlüsselten Tracinggeheimnis-Transferobjekts, Erzeugen einer TAN und Speicherung der TAN verknüpft mit dem verschlüsselten Tracinggeheimnis-Transferobjekt durch den Server, und Ausgabe der TAN an den Nutzer;
- Empfang der TAN von dem Nutzer durch ein der Entität zugewiesenes Entitäts-Gerät;
- Verwenden der TAN durch das Entitäts-Gerät, um in Interoperation mit dem Server die verschlüsselten Nutzerdaten des Nutzers zu empfangen und zu entschlüsseln.

Vorzugsweise wird die Erzeugung des Tracinggeheimnis-Transferobjekts durch das Nutzergerät ausschließlich durch ein explizites Kommando des Nutzers, dem das Nutzergerät zugewiesen ist, initiiert, beispielsweise dadurch, dass der Nutzer auf einer Tracing-App auf seinem Nutzergerät die Funktion "Identität und Besuchshistorie freigeben" auswählt. Beispielsweise kann der Nutzer dies nach Erhalt eines positiven Befundes für eine Infektionskrankheit nach einer Aufforderung des Gesundheitsamts (z.B. per Mail, Brief oder Telefonanruf) tun.

Dies kann vorteilhaft sein, da ohne die explizite Freigabe durch den Nutzer die Entität die Kandidaten-Trace-IDs nicht erzeugen und somit weder die besuchten Venues noch potenzielle Kontaktpersonen des Nutzers bei diesen Venues identifizieren kann. Wenn der betreffende Nutzer, der getraced werden soll, dem Amt seine Tracinggeheimnisse (also alle Daten, die zur Erzeugung der Kandidaten-Trace-IDs für einen bestimmten Nutzer erforderlich sind) z.B. mittels eines Tracinggeheimnis-Transferobjekts übermittelt, versetzt dies das Amt zwar in die Lage, die Identität der potentiellen Kontakte zu erkennen (deren Nutzerdaten können mit den User-DataKeys dieser Nutzer, die Bestandteil der Check-in Daten der besuchten Venues im Fraglichen Zeitraum sind, vom Amt entschlüsselt werden), nicht jedoch, diese potentiellen Kontaktpersonen zu tracen, da diese hierfür deren Tracinggeheimnis an die Entität übermitteln müssen.

Nach Ausführungsformen der Erfindung umfasst das Verwenden der TAN:
- Senden einer ersten Nutzerdatenanfrage beinhaltend die TAN an den Server durch das Entitätsgerät;
- Empfang einer ersten Nutzerdatenanfrage beinhaltend die TAN durch den Server von dem Entitätsgerät;
- In Antwort auf den Empfang der Nutzerdatenanfrage, senden des verschlüsselten Tracinggeheimnis-Transferobjekts, das mit der TAN verknüpft gespeichert ist, von dem Server an das Entitäts-Gerät;
- Entschlüsseln des Tracinggeheimnis-Transferobjekts mit dem privaten Entitäten-schlüssel, der zu dem öffentlichen Schlüssel der Entität korrespondiert, um die U-ser-ID und den zur Entschlüsselung der verschlüsselten Nutzerdaten des Nutzers erforderlichen User-Data-Entschlüsselungsschlüssel zu erhalten, durch das Entitäten-Gerät;
- Verwenden der entschlüsselten User-ID, um eine zweite Nutzerdatenanfrage beinhaltend die User-ID zu erzeugen und vom Entitäten-Gerät an den Server zu senden;
- Empfang der verschlüsselten Nutzerdaten des Nutzers vom Server durch das Entitäten-Gerät in Antwort auf die zweite Anfrage; und
- Entschlüsselung der verschlüsselten Nutzerdaten mittels des in dem Tracinggeheimnis-Transferobjekt enthaltenen Nutzerdaten-Entschlüsselungsschlüssels durch das Entitäten-Gerät.

Nach Ausführungsformen der Erfindung enthält das Tracinggeheimnis-Transferobjekt zusätzlich einen im Zuge der Registrierung des gegenüber dem Server erzeugten und zugriffsgeschützt ausschließlich im Nutzergerät gespeicherten Zufallswert ("userTracingSecret"). Der Server ist dazu konfiguriert, die Kandidaten-Trace-IDs mittels dieses Zufallswerts auf Basis einer Vielzahl von Zeitpunkten innerhalb des zurückliegenden Zeitraums und der User-ID zu berechnen.

Dies kann vorteilhaft sein, da das Amt vor Empfang des Tracinggeheimnis-Transferobjekts mit dem userTracingSecret die Kandidaten-Trace-IDs nicht berechnen und somit auch nicht als Suchwert in den Daten des Servers verwenden kann, um Check-in Datensätze zu identifizieren, die zu dem betreffenden Nutzer gehören. Somit kann das Amt ohne die willentliche Freigabe und Bereitstellung des Tracinggeheimnis-Transferobjekts nicht ermittelt, wann und bei welchen Venues der Nutzer einen Check-in durchgeführt hat.

Nach Ausführungsformen enthält das Tracinggeheimnis-Transferobjekt zusätzlich ein im Zuge der Registrierung des Nutzers des gegenüber dem Server erzeugten und zugriffsgeschützt ausschließlich im Nutzergerät gespeicherten Zufallswert, der im Folgenden als userVerificationSecret bezeichnet wird. Der Server ist dazu konfiguriert:
- die auf dem Server gespeicherten Trace-IDs, die identisch zu einer der Kandidaten-Trace-IDs sind, zu ermitteln;
- das im Tracinggeheimnis-Transferobjekt enthaltene userVerificationSecret zu verwenden, um mit diesem einen Verifikationsdatenwert für jede der ermittelten Trace-IDs als Funktion der Trace-ID und der mit diesen ermittelten Trace-IDs verknüpft gespeicherten verschlüsselten Ausweis-Daten (408) zu berechnen;
- den für eine jede der ermittelten Trace-IDs jeweils berechneten Verifikationsdatenwert mit einem Verifikationsdatenwert zu vergleichen, der im Zuge des Check-ins verknüpft mit der ermittelten Trace-ID gespeichert wurde; und
- ein Ergebnis dieses Vergleichs an das zumindest eine Entitäten-Gerät zu kommunizieren.

Das zumindest eine Entitäten-Gerät ist dazu konfiguriert, im Falle des Ergebnisses, dass der Vergleich eine fehlende Übereinstimmung der Verifikationsdatenwerte ergibt, die Zuweisung der Trace-ID an den Nutzer als manipuliert zu behandeln.

Die Erstellung des userVerificationSecret im Zuge des Registrationsprozesses des Nutzers, die Verwendung dieses userVerificationSecrets zur Berechnung eines Verifikationsdatenwertes, der als Bestandteil eines Check-in Nachricht bzw. QR-Codes direkt oder indirekt vom Nutzergerät an den Server übermittelt wird und die Bereitstellung des userVerificationSecret als Bestandteil des Tracinggeheimnis-Transferobjekts ans Amt kann den Vorteil haben, dass dem Amt die Prüfung ermöglicht wird, ob ein bestimmter Check-in Datensatz vom gleichen Nutzergerät (und damit dem gleichen Nutzer) erzeugt wurde, welches aktuell auch das Tracinggeheimnis-Transferobjekt übermittelt hat. Falls also eine Trace-ID von einem Angreifer bei der Übertragung abgefangen und auf einem Nutzergerät des Angreifers für einen Check-in unter falscher Identität verwendet werden sollte, würde dies auffallen, denn das Nutzergerät des Angreifers könnte zwar die gestohlene Trace-ID nochmals für den betrügerischen Check-in verwenden, aber es hätte ein anderes userVerificationSecret als das "berechtigte" Nutzergerät (oder auch gar kein userVerificationSecret). Falls es dem Angreifer also gelingen sollte, einen Check-in Vorgang mit der gestohlenen Trace-ID durchzuführen, wäre diesen falschen Check-in Daten auf dem Server ein falscher Verifikationsdatenwert zugeordnet (oder gar keiner) und ein Vergleich mit dem Verifikationsdatenwert aus dem Tracinggeheimnis-Transferobjekt würde fehlende Übereinstimmung ergeben.

Nach Ausführungsformen der Erfindung ist das elektronische System, und insbesondere der Server, so konfiguriert ist, dass diejenigen Nutzer ermittelt werden, die ebenfalls einen oder mehrere der ermittelten Venues während des vergangenen Zeitraums und während einer Aufenthaltsdauer des Nutzers besucht haben. Hierzu werden diejenigen der Check-in Datensätze ermittelt (sowie deren Trace-IDs), die eine Venue-ID und einen Check-in Zeitstempel enthalten aus welchen hervorgeht, dass sich die jeweiligen Check-in-Ereignisse an einem der ermittelten Venues während eines Zeitraums ergeben haben, an dem sich auch der zu tracende Nutzer in der Venue aufgehalten hat.

Durch Entschlüsselung der mit dem Entitätenschlüssel verschlüsselten Ausweisdaten dieser ermittelten Check-in Datensätze erlangt die Entität die Entschlüsselungsschlüssel für die Nutzerdaten der möglichen Kontaktpersonen, und somit an die Kontaktdaten der möglichen Kontaktpersonen. Optional haben die Venue-Geräte die Ausweisdaten in den von diesen erzeugten Check-in Datensätzen zusätzlich mit einem Schlüssel des Venue-Geräts verschlüsselt, sodass in diesem Fall der Server zunächst die ermittelten Check-in Datensätze an das durch die Venue-ID identifizierte Venue-Gerät senden muss um dieses die Ausweis-Daten entschlüsseln zu lassen und die entschlüsselten Ausweisdaten, die immer noch mit dem Entitäten-schlüssel verschlüsselt sind, über den Server dem Entitäten-Gerät zur Verfügung zu stellen.

In einem weiteren Aspekt betrifft die Erfindung ein elektronisches System, das nur eine oder einige der Komponenten des oben beschriebenen Systems umfasst, z.B. nur den Server, nur das Nutzergerät, nur ein Entitätengerät, nur ein Venue-Gerät, oder z.B. eine Kombination aus dem Server und einem Nutzergerät, eine Kombination aus dem Server und dem Venue-Gerät, eine Kombination aus dem Server und dem Entitäten-Gerät, oder eine Kombination aus dem Venue-Gerät oder dem Venue-Objekt und dem Nutzergerät.

In einem weiteren Aspekt betrifft die Erfindung ein Computerprogramm, ein Computerprogrammprodukt oder ein digitales Speichermedium mit von einem Prozessor eines Nutzergeräts ausführbaren Programminstruktionen zur Durchführung derjenigen Schritte, zu deren Durchführung des elektronische System nach einer der hier beschriebenen Ausführungsformen der Erfindung konfiguriert ist, welche das Nutzergerät betreffen.

In einem weiteren Aspekt betrifft die Erfindung ein Computerprogramm, ein Computerprogrammprodukt oder ein digitales Speichermedium mit von einem Prozessor ausführbaren Programminstruktionen zur Durchführung derjenigen Schritte zu deren Durchführung des elektronische System nach einer der hier beschriebenen Ausführungsformen der Erfindung konfiguriert ist, welche den Server betreffen.

In einem weiteren Aspekt betrifft die Erfindung ein Computerprogramm, ein Computerprogrammprodukt oder ein digitales Speichermedium mit von einem Prozessor ausführbaren Programminstruktionen zur Durchführung derjenigen Schritte zu deren Durchführung des elektronische System nach einer der hier beschriebenen Ausführungsformen der Erfindung konfiguriert ist, welche das zumindest eine Venue-Gerät betreffen.

In einem weiteren Aspekt betrifft die Erfindung ein digitales Speichermedium mit von einem Prozessor ausführbaren Programminstruktionen zur Durchführung derjenigen Schritte zu deren Durchführung des elektronische System nach einer der hier beschriebenen Ausführungsformen der Erfindung konfiguriert ist, welche das zumindest eine Entitäten-Gerät betreffen.

In einem weiteren Aspekt betrifft die Erfindung ein Nutzergerät eines Nutzers, das dazu verwendet werden kann um den Nutzer, der sich temporär in verschiedenen räumlichen Bereichen (Venues) aufhalten kann, für eine Entität (246) zu tracen, wobei das Nutzergerät dazu ausgebildet ist:
- anlässlich einer Registrierung des Nutzers: Verschlüsselung von Nutzerdaten des Nutzers mit einem für den Nutzer individuellen User-Data-Key; und Speicherung der verschlüsselten Nutzerdaten verknüpft mit einer User-ID des Nutzers auf dem Server zu speichern; und Speicherung der User-ID und eines Nutzer-individuellen Entschlüsselungsschlüssels, der zur Entschlüsselung der verschlüsselten Nutzerdaten erforderlich ist (kann im symmetrischen Fall der UserDataKey oder im asymmetrischen Fall ein zu diesem korrespondierender privater Schlüssel sein) in dem Nutzergerät, wobei vorzugsweise dieser Schlüssel geschützt gespeichert wird und dem Server nicht zugänglich ist;
- Im Zuge eines Check-ins des der Nutzer gegenüber einem der Venues, Erzeugung einer Trace-ID, wobei die Trace-ID repräsentativ ist für eine Kombination aus der User-ID des einen Nutzers und einer aktuellen Zeitinformation, und Übermittlung eines Check-in Datensatzes mit der Trace-ID an ein Venue-Gerät oder Übermittlung des Check-in Datensatzes beinhaltend eine Venue-ID des einen Venue auf den Server, um die Speicherung der Trace-ID mit den mit ihr verknüpften Daten (Check-in Daten) auf dem Server zu ermöglichen, wobei vorzugsweise die Trace-ID als Zugriffsschlüssel gespeichert wird,
- Optional eine Funktion zum Erfassen eines graphischen Codes auf einem Trägerobjekt mit einer Kamera des Nutzergeräts zur Anbahnung eines Check-ins, zur Extraktion einer Venue-ID aus dem Code und zur Verknüpfung der extrahierten Venue-ID mit der Trace-ID, wobei der Check-in Datensatz mit der extrahierten Venue-ID direkt an den Server gesendet wird;
- Optional eine Funktionalität zur Erstellung eines Tracinggeheimnis-Transferobjekts in Reaktion auf eine Nutzer-Interaktion mit dem Nutzergerät und zur Übermittlung des Tracinggeheimnis-Transferobjekts an den Server;
- Optional eine Schnittstelle zum Server zum Erhalt einer TAN, die mit dem Tracinggeheimnis-Transferobjekt verbunden ist, von dem Server, wobei das Nutzergerät zur Anzeige oder anderweitigen Ausgabe der empfangenen TAN an den Nutzer konfiguriert ist.

In einem weiteren Aspekt betrifft die Erfindung einen Server, das dazu verwendet werden kann um einen jeden von einer Vielzahl von Nutzern, die bei dem Server registriert sind, der sich temporär in verschiedenen räumlichen Bereichen (Venues) aufgehalten hat, für eine Entität zu tracen, wobei der Server ausgebildet ist zum:
- Speichern von verschlüsselten Nutzerdaten, die zumindest mit einem nutzerindividuellen Verschlüsselungsschlüssel und optional auch mit einem EntitätenSchlüssel verschlüsselt sind, im Zuge der Registrierung jedes der Nutzer in einer Datenbank;
- Empfang eines Check-in Datensatzes im Zuge des Check-ins des einen Nutzers gegenüber einem Venue-Gerät von dem Nutzergerät des Nutzers oder von dem Venue-Gerät, wobei der Check-in Datensatz eine Trace-ID umfasst sowie eine Venue-ID des Venues sowie Ausweisdaten, wobei zumindest die Ausweisdaten mit einem Schlüssel der Entität verschlüsselt sind, wobei die Ausweisdaten zumindest die User-ID des Nutzers sowie einen nutzerspezifischen Schlüssel zur Entschlüsselung der Nutzerdaten dieses Nutzers beinhalten, wobei die Trace-ID repräsentativ ist für eine Kombination aus der User-ID des Nutzers und einer aktuellen Check-in Zeitinformation,
- Speichern des empfangenen Check-in Datensatzes in der Datenbank, wobei vorzugsweise die Trace-ID als Zugriffsschlüssel gespeichert wird.

Nach Ausführungsformen ist der Server ferner ausgebildet zum:
- Empfang einer Mitteilung beinhaltend die User-ID eines der Nutzer, bezüglich welchem das Tracing durchgeführt werden soll;
- Erzeugung von Kandidaten-Trace-IDs für den in der Mitteilung identifizierten Nutzer, wobei die Kandidaten-Trace-IDs mögliche zurückliegende Trace-IDs sind, die innerhalb eines vorgegebenen vergangenen Zeitraums liegen; vorzugsweise enthält die Mitteilung aller zur Berechnung der Kandidaten-Trace-IDs nötigen Daten, also insb. die UserID und das TracingSecret, und vorzugsweise auch eine Spezifikation des Zeitraums, für welchen die Kandidaten-Tracing-IDs berechnet werden sollen; Gemäß einer alternativen Ausführungsform empfängt der Server die Kandidaten-Trace-IDs stattdessen von einem Entitäten-Gerät der Entität;

- Verwendung der erzeugten oder empfangenen Kandidaten-Trace-IDs als Suchkriterium , um entsprechende auf dem Server für den vergangenen Zeitraum gespeicherte Trace-IDs dieses Nutzers aufzufinden, so dass die Venue-IDs derjenigen Venues gegenüber welchen der zu tracende Nutzer während des vergangenen Zeitraums einen Check-in durchgeführt hat, ermittelt werden, und
- Ausgabe der Venue-IDs der ermittelten Venues zur Identifizierung derjenigen Venues, die der zu tracende Nutzer während des vergangenen Zeitraums besucht hat, durch den Server.

In einem weiteren Aspekt betrifft die Erfindung ein Datenverarbeitungsgerät, das einer Entität, insbesondere einem Amt oder einer Behörde, zugewiesen ist, und als Entitäten-Gerät bezeichnet wird. Das Entitäten-Gerät kann dazu verwendet werden, einen jeden von einer Vielzahl von Nutzern, die bei einem Tracing-Server registriert sind, und die sich temporär in verschiedenen räumlichen Bereichen (Venues) aufgehalten haben, für die Entität zu tracen, wobei das Entitäten-Gerät ausgebildet ist zum:
- Empfang einer Mitteilung beinhaltend die User-ID eines der Nutzer, bezüglich welchem das Tracing durchgeführt werden soll;
- Erzeugung von Kandidaten-Trace-IDs für den in der Mitteilung identifizierten Nutzer, wobei die Kandidaten-Trace-IDs mögliche zurückliegende Trace-IDs sind, die innerhalb eines vorgegebenen vergangenen Zeitraums liegen; vorzugsweise enthält die Mitteilung aller zur Berechnung der Kandidaten-Trace-IDs nötigen Daten, also insb. die NutzerID und das TracingSecret, und vorzugsweise auch eine Spezifikation des Zeitraums, für welchen die Kandidaten-Tracing-IDs berechnet werden sollen;
- Senden der erzeugten oder empfangenen Kandidaten-Trace-IDs als Bestanteil einer Suchanfrage an den Server, wobei die Anfrage die Kandidaten-Trace-IDs als Suchkriterium enthält, um entsprechende auf dem Server für den vergangenen Zeitraum gespeicherte Trace-IDs dieses Nutzers aufzufinden, so dass die Venue-IDs derjenigen Venues gegenüber welchen der zu tracende Nutzer während des vergangenen Zeitraums einen Check-in durchgeführt hat, ermittelt werden, und
- Empfang der Venue-IDs der ermittelten Venues zur Identifizierung derjenigen Venues, die der zu tracende Nutzer während des vergangenen Zeitraums besucht hat, von dem Server über ein Netzwerk.

In einem weiteren Aspekt betrifft die Erfindung ein Datenverarbeitungsgerät, das einem Venue, also einem räumlichen Bereich, in dem sich Nutzer aufhalten können, zugewiesen ist, und als Venue-Gerät bezeichnet wird. Das Venue-Gerät kann dazu verwendet werden, einem jeden von einer Vielzahl von Nutzern, die bei einem Tracing-Server registriert sind, und die sich temporär in verschiedenen räumlichen Bereichen (Venues) aufhalten können, einen automatischen oder semi-automatischen Check-in in den Venue zu ermöglichen, wobei der Besuch des Venues für die Entität tracebar ist, wobei das Venue-Gerät eine Schnittstelle zur lokalen Datenübertragung mit einem Nutzergerät eines Nutzers, der einen Check-in gegenüber dem Venue vornehmen will, beinhaltet und ausgebildet ist zum:
- Empfang eines Check-in Signals (z.B. QR Code oder Nahfeldsignal) von dem Nutzergerät des Nutzers, wobei das Check-in Signal einen Check-in Datensatz beinhaltet, wobei der Check-in Datensatz zumindest eine Trace-ID, eine Check-in Zeitinformation und verschlüsselte Ausweisdaten des Nutzers beinhaltet, wobei die Ausweisdaten mit einem Verschlüsselungsschlüssel der Entität verschlüsselt sind und zumindest die User-ID des Nutzers und einen für diesen Nutzer spezifischen Entschlüsselungsschlüssel für die Nutzerdaten dieses Nutzers beinhalten;
- Optional: Prüfung des Alters der Zeitinformation in dem Check-in Datensatz und Weiterverarbeitung der Check-in Datensatzes nur dann, wenn die Zeitinformation ein Maximalalter nicht überschreitet;
- Ergänzung der empfangenen Check-in Daten um die Venue-ID des Venue;
- Optional: Verschlüsseln zumindest eines Teils des empfangenen und ergänzten Check-in Datensatzes mit einem Venue-spezifischen Verschlüsselungsschlüssel, wobei die TraceID unverschlüsselt bleibt;
- Übermittlung der ergänzten und optional mit dem Venue-Schlüssel verschlüsselten Check-in Daten über das Netzwerk an den Server.

In einem weiteren Aspekt betrifft die Erfindung ein (computerimplementiertes) Verfahren zum Tracing von Nutzern, die sich temporär in verschiedenen räumlichen Bereichen (Venues) aufhalten, für eine Entität, insbesondere für Notfallszenarien wie in einer Pandemie. Das Verfahren umfasst:
- anlässlich einer Registrierung eines jeden der Nutzer: Speicherung von Nutzerdaten des Nutzers auf einem Server, wobei die Nutzerdaten von einem dem Nutzer zugeordneten Nutzergerät mit einem für den Nutzer individuellen User-Data-Key verschlüsselt werden, wobei insbesondere eine User-ID des Nutzers verknüpft mit den verschlüsselten Nutzerdaten auf dem Server gespeichert werden; und Speicherung der User-ID und eines Nutzer-individuellen zur Entschlüsselung der verschlüsselten Nutzerdaten erforderlichen User-Data-Entschlüsselungsschlüssels in dem Nutzergerät, wobei vorzugsweise der U-ser-Data-Key und/oder der User-Data-Entschlüsselungsschlüssel geschützt gespeichert werden und dem Server nicht zugänglich sind;
- Im Zuge eines Check-ins eines der Nutzer gegenüber einem der Venues, Erzeugung einer Trace-ID durch das Nutzergerät des einen Nutzers, wobei die Trace-ID repräsentativ ist für eine Kombination aus der User-ID des einen Nutzers und einer aktuellen Zeitinformation, und Speicherung der Trace-ID verknüpft mit der Venue-ID des einen Venue auf dem Server, wobei die Trace-ID als Zugriffsschlüssel gespeichert wird,
- Empfang einer Mitteilung beinhaltend die User-ID eines der Nutzer, bezüglich welchem das Tracing durchgeführt werden soll;
- Erzeugung von Kandidaten-Trace-IDs für den in der Mitteilung identifizierten Nutzer, wobei die Kandidaten-Trace-IDs mögliche zurückliegende Trace-IDs sind, die innerhalb eines vorgegebenen vergangenen Zeitraums liegen,
- Verwendung der erzeugten Kandidaten-Trace-IDs als Suchkriterium durch den Server, um entsprechende auf dem Server für den vergangenen Zeitraum gespeicherte Trace-IDs aufzufinden, so dass die Venue-IDs derjenigen Venues gegenüber welchen der zu tracende Nutzer während des vergangenen Zeitraums einen Check-in durchgeführt hat, ermittelt werden, und
- Ausgabe der Venue-IDs der ermittelten Venues zur Identifizierung derjenigen Venues, die der zu tracende Nutzer während des vergangenen Zeitraums besucht hat, durch den Server.

Nach Ausführungsformen umfasst das Verfahren weitere Schritte, die von ein oder mehreren Komponenten eines elektronischen Systems zum Tracing von Nutzern ausgeführt werden, und die bereits im Hinblick auf diese Ausführungsvarianten des elektronischen Systems beschrieben wurden.

In einem weiteren Aspekt betrifft die Erfindung ein elektronisches System mit Nutzergeräten, die jeweils einem Nutzer zu-geordnet sind, Venue-Geräten und zumindest einem Server zum Tracing von Nutzern für eine Entität, der ein asymmetrisches kryptographisches Schlüsselpaar mit einem öffentlichen und einem privaten Entitätenschlüssel zugeordnet ist, wobei das elektronische System zur Durchführung der folgenden Schritte konfiguriert ist:
- Speicherung von Nutzerdaten der Nutzer anlässlich einer Registrierung auf einem Server, wobei die Nutzerdaten durch den öffentlichen Entitätenschlüssel verschlüsselt sind und das Resultat dieser Ver-schlüsselung mit einem jeweiligen U-ser-Data-Key des betreffenden Nutzers verschlüsselt ist,
- Speicherung in jedem der Nutzergeräte eines User-Data-Key, eines User-Secret und einer User-ID, wobei das Nutzergerät über eine Netzwerkschnittstelle Zugriff auf eine aktuelle Zeitinformation hat und das Nutzergerät konfiguriert ist zur Erzeugung einer Trace-ID, die repräsentativ ist für eine Kombination aus User-ID, User-Secret und der aktuellen Zeitinformation sowie zur Erzeugung eines Chiffrats aus dem User-Data-Key und der User-ID, wobei der öffentliche Entitäten-schlüssel zur Erzeugung des Chiffrats verwendet wird, und wobei das Nutzergerät eine Kommunikationsschnittstelle zur direkten Kommunikation mit einem Venue-Gerät aufweist, über welche die Trace-ID und das Chiffrat an das Venue-Gerät übertragen werden,

- Erfassung eines der Nutzer durch ein Venue-Gerät mit Hilfe des Nutzergeräts des Nutzers, wobei das Venue-Gerät eine Schnittstelle für die direkte Kommunikation mit der entsprechenden Schnittstelle des Nutzergeräts aufweist, und mit einer Netzwerkschnittstelle zur Kommunikation mit dem Server, wobei das Venue-Gerät dazu konfiguriert ist, zur Erfassung des Nutzers die Trace-ID und das Chiffrat von dem Nutzergerät zu empfangen und zu prüfen, ob die Zeitinformation einer empfangenen Trace-ID hinreichend aktuell ist, und wenn dies der Fall ist, die Trace-ID mit einer dem Venue-Gerät zugeordneten Venue-ID und dem Chiffrat an den Server zu übertragen,
- Speicherung einer empfangenen Trace-ID und dem Chiffrat mit der Zeitinformation als Zugriffsschlüssel durch den Server,
- Empfang einer Mitteilung der User-ID und des User-Secrets von einem der Nutzer an die Entität,
- Erzeugung von möglichen zurückliegenden Trace-IDs für den betreffenden Nutzer, die innerhalb eines vorgegebenen vergangenen Zeitraums liegen und Verwendung der erzeugten Trace-IDs als Suchkriterium, um entsprechende auf dem Server für den vergangenen Zeitraum gespeicherte Trace-IDs aufzufinden, so dass die Venue-IDs derjenigen Venues von denen der Nutzer während des vergangenen Zeitraums erfasst wurde, ermittelt werden,
- Ausgabe der Venue-IDs der ermittelten Venues zur Identifizierung derjenigen Venues, die der Nutzer während des vergangenen Zeitraums besucht hat.

Nach Ausführungsformen ist das elektronische System so konfiguriert, dass von dem Nutzergerät des einen der Nutzer eine Check-out Zeit an den Server übertragen wird, wenn der Nutzer den Venue verlässt, wobei die Check-out Zeit der zuvor für diesen Nutzer empfangenen Trace-ID zugeordnet wird, so dass für den Nutzer der Aufenthaltszeitraum an dem Venue gespeichert wird.

Nach Ausführungsformen ist das elektronische System so konfiguriert, dass diejenigen Nutzer ermittelt werden, die ebenfalls einen oder mehrere der ermittelten Venues während des vergangenen Zeitraums und während einer Aufenthaltsdauer des Nutzers besucht haben, wozu diejenigen der Trace-IDs, die von den ermittelten Venues während des vergangenen Zeitraums zu dem Server übertragen wurden, ermittelt werden.

In einem weiteren Aspekt betrifft die Erfindung ein digitales Speichermedium mit von einem Prozessor eines Datenverarbeitungsgeräts ausführbaren Programminstruktionen zur Durchführung derjenigen Schritte zu deren Durchführung des elektronische System nach einer der hier beschriebenen Ausführungsformen, welche das Datenverarbeitungsgerät betreffen, wobei es sich bei dem Datenverarbeitungsgerät um das Nutzergerät, ein Entitäten-Gerät, ein Venue-Gerät oder um den Server handelt.

In einem weiteren Aspekt betrifft die Erfindung ein elektronisches System mit Nutzergeräten, die jeweils einem Nutzer zugeordnet sind, Venue-Geräten und zumindest einem Server zum Tracing von Nutzern für eine Entität, der ein asymmetrisches kryptographisches Schlüsselpaar mit einem öffentlichen und einem privaten Entitätenschlüssel zugeordnet ist, wobei das elektronische System zur Durchführung der folgenden Schritte konfiguriert ist:
- Speicherung von Nutzerdaten der Nutzer anlässlich einer Registrierung auf einem Server, wobei die Nutzerdaten durch den öffentlichen Entitätenschlüssel verschlüsselt sind,
- Speicherung einer User-ID in jedem der Nutzergeräte, wobei das Nutzergerät über eine Netzwerkschnittstelle Zugriff auf eine aktuelle Zeitinformation hat und das Nutzergerät konfiguriert ist zur Erzeugung einer Trace-ID, die repräsentativ ist für eine Kombination aus User-ID und der aktuellen Zeitinformation und wobei das Nutzergerät eine Kommunikationsschnittstelle zur direkten Kommunikation mit einem Venue-Gerät aufweist, über welche die Trace-ID an das Venue-Gerät übertragen werden,

- Erfassung eines der Nutzer durch ein Venue-Gerät mit Hilfe des Nutzergeräts des Nutzers, wobei das Venue-Gerät eine Schnittstelle für die direkte Kommunikation mit der entsprechenden Schnittstelle des Nutzergeräts aufweist, und mit einer Netzwerkschnittstelle zur Kommunikation mit dem Server, wobei das Venue-Gerät dazu konfiguriert ist, zur Erfassung des Nutzers die Trace-ID von dem Nutzergerät zu empfangen und zu prüfen, ob die Zeitinformation einer empfangenen Trace-ID hinreichend aktuell ist, und wenn dies der Fall ist, die Trace-ID mit einer dem Venue-Gerät zugeordneten Venue-ID an den Server zu übertragen,
- Speicherung einer empfangenen Trace-ID mit der Zeitinformation als Zugriffsschlüssel durch den Server,
- Empfang einer Mitteilung der User-ID von einem der Nutzer an die Entität,
- Erzeugung von möglichen zurückliegenden Trace-IDs für den betreffenden Nutzer, die innerhalb eines vorgegebenen vergangenen Zeitraums liegen und Verwendung der erzeugten Trace-IDs als Suchkriterium, um entsprechende auf dem Server für den vergangenen Zeitraum gespeicherte Trace-IDs aufzufinden, so dass die Venue-IDs derjenigen Venues von denen der Nutzer während des vergangenen Zeitraums erfasst wurde, ermittelt werden,
- Ausgabe der Venue-IDs der ermittelten Venues zur Identifizierung derjenigen Venues, die der Nutzer während des vergangenen Zeitraums besucht hat.

Nach Ausführungsformen ist das elektronische System so konfiguriert, dass von dem Nutzergerät des einen der Nutzer oder von dem Venue-Gerät eine Check-out Zeit an den Server übertragen wird, wenn der Nutzer den Venue verlässt, wobei die Check-out Zeit der zuvor für diesen Nutzer empfangenen Trace-ID zugeordnet wird, so dass für den Nutzer der Aufenthaltszeitraum an dem Venue gespeichert wird.

Nach Ausführungsformen ist das elektronische System so konfiguriert, dass diejenigen Nutzer ermittelt werden, die ebenfalls einen oder mehrere der ermittelten Venues während des vergangenen Zeitraums und während einer Aufenthaltsdauer des Nutzers besucht haben, wozu diejenigen der Trace-IDs, die von den ermittelten Venues während des vergangenen Zeitraums zu dem Server übertragen wurden, ermittelt werden.

In einem weiteren Aspekt betrifft die Erfindung ein digitales Speichermedium mit von einem Prozessor eines Datenverarbeitungsgeräts ausführbaren Programminstruktionen zur Durchführung derjenigen Schritte zu deren Durchführung des elektronische System nach einer der hier beschriebenen Ausführungsformen, welche das Datenverarbeitungsgerät betreffen, wobei es sich bei dem Datenverarbeitungsgerät um das Nutzergerät, ein Entitäten-Gerät, ein Venue-Gerät oder um den Server handelt.

Unter einem "Nutzergerät" wird hier ein portables Datenverarbeitungssystem verstanden, insbesondere ein batteriegetriebenes Datenverarbeitungssystem wie z.B. ein Mobilfunkgerät, ein Notebook, ein Tablet-Computer oder ein integriertes Gerät, das spezifisch für das automatische Check-in und Tracing von Besuchern an einem Venue konzipiert wurde und/oder genutzt wird.

Unter einem "Mobilfunkgerät" wird hier ein portables elektronisches System, insbesondere ein batteriegetriebenes Telekommunikationsgerät verstanden, welches über zumindest einen Prozessor zur Ausführung von Programminstruktionen verfügt, insbesondere zur Ausführung von sogenannten Apps. Ein Mobilfunkgerät hat eine Netzwerkschnittstelle zum Aufbau von Kommunikationsverbindungen über ein Telekommunikationsnetzwerk, welches nach einem Mobilfunkstandard ausgebildet sein kann.

Das Nutzergerät kann optional über weitere Kommunikationsschnittstellen verfügen, wie zum Beispiel eine Anzeigevorrichtung, das heißt ein sogenanntes Display, für eine optische Kommunikation mit dem Nutzer oder auch für eine maschinelle Kommunikation beispielsweise durch Anzeigen optischer Muster, wie zum Beispiel eines QR-Codes. Über diese weitere Kommunikationsschnittstelle erfolgt die Kommunikation direkt, das heißt ohne Zwischenschaltung eines Netzwerks. Beispielsweise weist das Nutzergerät hierzu auch ein oder mehrere Nahfeldkommunikationsschnittstellen auf, beispielsweise nach dem Bluetooth- und/oder NFC-Standard. Das Nutzergerät verfügt ferner über zumindest einen Speicher, in dem Daten gespeichert werden können.

Ein Nutzergerät ist einem Nutzer eindeutig zugeordnet. Dies erfolgt oftmals, insbesondere bei Mobilfunkgeräten, über ein SIM oder eSIM.

Unter einem "Venue"-Gerät wird hier insbesondere ein elektronisches Gerät verstanden, welches der Betreiber eines Venues, das heißt eines Veranstaltungsorts für eine öffentliche oder private Veranstaltung oder einer Gaststätte, wie zum Beispiel Restaurant, Club oder dergleichen, verwendet, um die Nutzer, welche den Venue besuchen, zu erfassen. Ein Venue kann auch ein Fortbewegungsmittel oder ein Bereich in diesem sein, z.B. ein Flugzeug, ein Zug oder Zugabteil. Ein Venue-Gerät kann als monolithisches oder verteiltes Datenverarbeitungssystem ausgebildet sein, z.B. als Mobilfunkgerät oder als stationärer Terminal, wie zum Beispiel als PC. Bei einem Venue-Gerät kann es sich auch um eine automatisierte Zugangskontrollvorrichtung, beispielsweise mit einem Drehkreuz oder einer Zugangsschleuse, handeln. Das Venue-Gerät kann auch ein Datenverarbeitungssystem eines Drittanbieters sein, z.B. für Zwecke des Ticketings (automatisches Bezahlen, Besucherverwaltung, etc.) Das Venue-Gerät verfügt über eine Schnittstelle für die direkte Kommunikation mit der entsprechenden Schnittstelle des Nutzergeräts sowie über eine Netzwerkschnittstelle zur Kommunikation mit einem Server über ein Telekommunikationsnetzwerk, welches drahtgebunden oder drahtlos ausgebildet sein kann.

Unter einem "Server" wird hier ein Datenverarbeitungssystem verstanden, insbesondere ein Servercomputer, ein virtueller Server oder ein verteilter Server, der beispielsweise über Cloudcomputing realisiert ist. Der Server dient zum Tracing der Nutzer, wobei hier unter "Tracing" die Möglichkeit der Rückverfolgung von in der Vergangenheit liegenden Besuchen eines Nutzers an Venues verstanden wird sowie optional auch die Möglichkeit, diejenigen Nutzer zu ermitteln, welche sich gleichzeitig mit einem bestimmten Nutzer, der beispielsweise mit Covid-19 infiziert ist, an einem der Venues aufgehalten haben. Gemäß Ausführungsformen kann der Server auch Computer und/oder Funktionen von Drittanbietern integrieren und diese nutzen.

Unter einem "Nutzer" wird hier eine natürliche Person verstanden, die als Besucher einer oder mehrerer räumlicher Bereiche (Venues) agieren kann, oder ein mobiles, insbesondere autonom oder semiautonom fahrendes technisches System (z.B. Roboter, KfZ, LKW), oder ein innerhalb eines solchen mobilen technischen Systems transportiertes physisches Objekt (diverse Waren, industrielle Fertigungsprodukte, Ersatzteile, Bauteile, etc.).

Unter dem Begriff "Tracing" wird hier die Nachverfolgung von Kontakten eines bestimmten Nutzers über einen Zeitraum verstanden, wobei vorzugsweise keine Bewegungsprofile erstellt werden oder werden müssen.

Dieses Tracing soll für eine "Entität" stattfinden. Unter einer "Entität" wird hier eine vertrauenswürdige natürliche oder juristische Person verstanden, insbesondere eine Institution, die z.B. hoheitlichen Rechte für die Ermittlung der Aufenthaltsorte der Nutzer hat, wie zum Beispiel das Gesundheitsamt im Falle einer Pandemie.

Erfindungsgemäß wird gemäß Ausführungsformen so vorgegangen, dass sich die Nutzer zunächst mithilfe der jeweiligen Nutzergeräte registrieren. Hierzu werden zunächst Nutzerdaten erfasst, also beispielsweise durch Eingabe der Nutzerdaten in das betreffende Nutzergerät oder durch Einlesen der Nutzerdaten, beispielsweise aus einem elektronischen Personalausweis des Nutzers in das Nutzergerät. Die Nutzerdaten werden dann mithilfe des öffentlichen Entitäten-schlüssels, also beispielsweise mithilfe eines öffentlichen Schlüssels des Gesundheitsamts, von dem Nutzergerät verschlüsselt und das Resultat dieser Verschlüsselung wird mit einem User-Data-Key des betreffenden Nutzers verschlüsselt, wobei es sich bei dem User-Data-Key hier beispielsweise um einen Zufallswert hoher Entropie handelt, wie zum Beispiel einer Länge von 16 Bytes.

Die Verschlüsselung der Nutzerdaten kann gemäß einer bevorzugten Ausführungsform unmittelbar durch den für den Nutzer im Zuge der Registrierung erzeugten U-ser-Data-Key (nutzerspezifischer Verschlüsselungsschlüssel für die Nutzerdaten des Nutzers) erfolgen. Gemäß einer anderen Ausführungsform erfolgt die Ver-schlüsselung unmittelbar durch den öffentlichen Entitätenschlüssel. Gemäß einer weiteren alternativen Ausführungsform erfolgt die Verschlüsselung der Nutzerdaten durch eine zweifache sequenzielle Verschlüsselung zunächst mit dem User-Data-Key und dann mit dem öffentlichen Entitätenschlüssel oder umgekehrt. Gemäß einer weiteren Ausführungsform leitet das Nutzergerät aus dem öffentlichen Entitäten-schlüssel ein weiterer Schlüssel nach einem vordefinierten Verfahren ab, welcher dann zur Verschlüsselung der Nutzerdaten dient. Beispielsweise kann der weitere Schlüssel aus dem öffentlichen Entitätenschlüssel und dem User-Data-Key abgeleitet werden.

Bei dem Entitätenschlüssel kann es sich um einen Master Key handeln oder den Schlüssel einer der Entität zugeordneten abhängigen Entität. Beispielsweise kann es sich im Fall des Gesundheitsamts um den Masterschlüssel des Gesundheitsamts oder um einen Schlüssel des jeweils vor Ort verantwortlichen Gesundheitsamts handeln.

Der User-Data-Key kann beispielsweise für die Zwecke der Registrierung durch das Betriebssystem des Nutzergeräts zur Verfügung gestellt werden. Beispielsweise verfügt das Nutzergerät über eine Binary Symmetric Source zur Erzeugung von Zufallswerten hoher Entropie.

Die Hinterlegung der Nutzerdaten auf dem Server erfolgt also in einer Form, die es dem Server zunächst nicht ermöglicht, diese Nutzerdaten zu entschlüsseln, da dies ohne den privaten Entitätenschlüssel und/oder den zu dem User-Data-Key korrespondierenden User-Data-Entschlüsselungsschlüssel nicht möglich ist.

Gemäß Ausführungsformen handelt es sich bei dem User-Data-Key um einen symmetrischen Schlüssel, d.h., der zur Verschlüsselung der Nutzerdaten verwendete U-ser-Data Key und der zur Entschlüsselung verwendete User-Data-Entschlüsselungsschlüssel sind identisch. Gemäß einer alternativen Ausführungsform handelt es sich bei dem User-Data-Key und dem User-Data-Entschlüsselungsschlüssel um ein asymmetrisches kryptographisches Schlüsselpaar, wobei der User-Data-Key zur Verschlüsselung und der User-Data Entschlüsselungsschlüssel zur Entschlüsselung verwendetet wird. In dem Nutzergerät wird dieser User-Data-Key in einem nichtflüchtigen Speicher persistent gespeichert. Aufgrund der Registrierung auf dem Server erzeugt der Server eine User-ID, das heißt einen eindeutigen Identifikator für den betreffenden User, die der Server aufgrund der Registrierung an das Nutzergerät zurückgibt. Die User-ID wird in dem Nutzergerät in einem nichtflüchtigen Speicher persistent gespeichert. Um einen Nutzer beim Besuch einer Venue zu erfassen, wird z.B. wie folgt vorgegangen:

Das Nutzergerät erzeugt eine Trace-ID, die repräsentativ ist für eine Kombination aus der User-ID und einer aktuellen Zeitinformation. Bei der aktuellen Zeitinformation handelt es sich um die aktuelle Uhrzeit beim Check-in des Nutzers an dem Venue, wobei diese aktuelle Uhrzeit von dem Nutzergerät über das Netzwerk ermittelt werden. Vorzugsweise wird diese aktuelle Uhrzeit nach einem vordefinierten Rundungsverfahren gerundet, beispielsweise auf die volle Minute auf- oder abgerundet.

Außerdem erzeugt das Nutzergerät verschlüsselte Ausweis-Daten dadurch, dass z.B. die Nutzer-ID und der zur Entschlüsselung der Nutzerdaten erforderliche User-Data-Entschlüsselungsschlüssel mit dem öffentlichen Entitätenschlüssel verschlüsselt werden. Die verschlüsselten Ausweisdaten enthalten also nur eine Nutzer-ID anstelle der Nutzerdaten, sind also so klein, dass die verschlüsselten Ausweisdaten zusammen mit der Trace-ID und optionalen weiteren Daten schnell und effizient an das Venue-Gerät übermittelt werden können, z.B. dadurch, dass diese Daten in einem QR-Code kodiert und auf einem Display des Nutzergeräts angezeigt und von einer Kamera des Venue-Geräts erfasst werden. Die optionalen weiteren Daten umfassen z.B. dem Zeitstempel, der zur Ableitung der Trace-ID verwendet wurde, sowie ggf. einem Verifikationsdatenwert und/oder einer QR-Code Version.

Die besagten Informationen können auch bei einem automatischen Check-Out übermittelt werden, wobei hierbei vorzugsweise die gleiche Trace-ID wie beim Check-in verwendet wird, und die aktuell erfasste und an das Venue-Gerät übermittelte Zeit die Check-out Zeit repräsentiert. Das Venue-Gerät prüft gemäß Ausführungsformen der Erfindung zur Erfassung eines Nutzers beim Check-in, ob die Zeitinformation, auf der die Trace-ID beruht, hinreichend aktuell ist, und verwendet hierzu beispielsweise ebenfalls eine über ein Netzwerk bezogene Zeitinformation zur Synchronisierung mit der von dem Nutzergerät verwendeten Zeitbasis und/oder als Referenzwert zum Vergleich mit der vom Nutzergerät erhaltenen Zeitinformation. Die Zeitinformation gilt als hinreichend aktuell, wenn sie beispielsweise innerhalb eines vorgegebenen Toleranzbereichs liegt, also beispielsweise nicht älter ist als einige Sekunden oder Minuten. Gemäß einer Ausführungsform ist die Zeitinformation in der Trace-ID so kodiert, dass diese vom Venue-Gerät nicht aus der Trace-ID extrahiert werden kann. In diesem Fall kann das Nutzergerät zusätzlich zur der Trace-ID die Check-in Zeit, die als Basis für die Ableitung der Trace-ID verwendet wurde, an das Venue-Gerät übermitteln, sodass das Venue-Gerät diese zusätzliche Zeit mit einer Referenzzeit (z.B. der Systemzeit des Venue-Geräts oder einer von einem Zeit-Server über das Internet) verglichen kann. Wenn die Zeitinformation auf der die Trace-ID beruht hinreichend aktuell ist, so überträgt das Venue-Gerät gemäß Ausführungsformen die Trace-ID mit einer dem Venue-Gerät zugeordneten Venue-ID und vorzugsweise auch weiteren Daten wie insb. einem Chiffrat (also den mit dem öffentlichen Entitätenschlüssel verschlüsselten Ausweisdaten) und ggf. einem oder mehreren der weiteren Daten an den Server. Diese Informationen werden auf dem Server gespeichert, wobei vorzugsweise die Speicherung so erfolgt, dass die Trace-ID als Suchschlüssel dient. Beispielsweise kann ein durchsuchbarer Index in einer Datenbank über der Spalte "Trace-ID" erstellt werden, um ein effizientes Suchen in der Gesamtheit aller Check-in Datensätze der beim Server registrierten Nutzer gegenüber den beim Server registrierten Venues zu ermöglichen.

Vorzugsweise wird dies für eine möglichst große Anzahl von Nutzern, vorzugsweise für sämtliche Nutzer, durchgeführt, die eine Venue besuchen, um so möglichst lückenlos sämtliche Nutzer zu erfassen.

Nachträglich kann das Erfordernis bestehen, den oder die Nutzer, welche den Venue besucht haben, gegenüber der Entität offenzulegen. Dies ist beispielsweise dann der Fall, wenn einer der Nutzer positiv auf Covid-19 getestet wird. Dieser mit Covid-19 infizierte Nutzer meldet dies der Entität, hier dem Gesundheitsamt, und teilt der Entität seine User-ID und sein User-Secret mit. Anhand dieser Informationen werden dann sämtliche mögliche zurückliegende Trace-IDs, die zur Erfassung eines Nutzers durch ein Venue-Gerät für einen vorgegebenen vergangenen Zeitraum benutzt sein könnten, erzeugt. Die Erzeugung kann z.B. auf dem Server oder einem Entitäten-Gerät erfolgen, sofern die hierfür erforderlichen Daten vorhanden sind. Die Menge dieser möglichen Trace-IDs, auch als "Kandidaten-Trace-IDs" bezeichnet, ist typischerweise relativ begrenzt, da nicht sämtliche mögliche Zeiten innerhalb des relevanten Zeitraums in Frage kommen, sondern nur diejenigen, die aufgrund des vorgegebenen Rundungsverfahrens tatsächlich vorkommen können. Beispielsweise wird zum Zwecke eines Tracings eines Nutzers über einen Zeitraum von 2 Wochen bei Verwendung einer Ableit-Funktion, die auf Zeitwerten beruhen, die auf die Minute genau erfasst und abgerundet werden, 14 Tage x 24 Stunden x 60 Minuten = ca. 20.000 Kandidaten-Trace-IDs für eine bestimmte User-ID berechnet.

Diese Kandidaten-Trace-IDs werden dann als Suchkriterium verwendet, um entsprechend auf dem Server für den vergangenen Zeitraum gespeicherte Trace-IDs aufzufinden, sodass die Venue-IDs derjenigen Venues, von denen der Nutzer während des vergangenen Zeitraums erfasst wurde, ermittelt werden. Hierdurch erhält also das Gesundheitsamt die Venue-IDs der ermittelten Venus, die der Nutzer in dem vergangenen Zeitraum, beispielsweise zwei Wochen, in denen er möglicherweise bereits infektiös war, besucht hatte. Ein Abgleich von 20.000 Datenwerten über einen durchsuchbaren Datenbankindex kann auf Basis heutiger Datenbanktechnologie sehr rasch und in der Regel in Echtzeit durchgeführt werden.

Nach einer Ausführungsform der Erfindung ist das elektronische System so konfiguriert, dass von dem Nutzergerät eines durch ein Venue-Gerät erfassten Nutzers, also nach einem Check-in, eine Check-out Zeit an den Server übertragen wird, wenn der Nutzer den Venue verlässt. Diese Check-out-Zeit ist der zuvor für diesen Nutzer empfangenen Trace-ID zugeordnet, sodass serverseitig die Information vorliegt, in welchem Zeitraum sich der Nutzer an dem Venue aufgehalten hat. Alternativ erfolgt dies durch das Venue-Gerät.

Nach einer Ausführungsform der Erfindung erfolgt dieser Check-out durch manuelle Eingabe in das Nutzergerät oder automatisch durch sogenanntes Geofencing. In letzterem Fall wird die Check-out-Zeit mit der zugeordneten Trace-ID automatisch von dem Nutzergerät an den Server gesendet, sobald der Nutzer die Venue verlässt. Alternativ erfolgt dies durch das Venue-Gerät.

Vorzugsweise geht diese Information in die Ermittlung derjenigen Nutzer ein, die sich ebenfalls an dem Venue aufgehalten hatten, indem die Suche auf solche Nutzer beschränkt wird, die nicht nur den gleichen Venue besucht hatten, sondern dort auch in einem zumindest mit dem Aufenthaltszeitraum des erkannten Nutzers überlappenden Aufenthaltszeitraums gewesen sind. Beispielsweise können in den Check-in Datensätzen des Servers, die die Trace-IDs der Nutzer beinhalten, auch die Venue-IDs und Check-in und optional auch Check-Out Zeiten im Klartext angegeben werden, sodass die Check-in Datensätzen von Nutzern, die zur gleichen Zeit wie der Nutzer, auf den sich der Notfall bezieht, in einem bestimmten Venue waren, ermittelt werden können. Als Venue-ID kann beispielsweise der Name und/oder Adresse, ein Identifikator oder auch die geografischen Koordinaten des Venues dienen. Im Weiteren werden Ausführungsformen der Erfindung mit Bezugnahme auf die Zeichnungen näher erläutert.

Es zeigen:
- Figur 1: ein Flussdiagramm eines Verfahrens zum Tracing von Nutzern,
- Figur 2: ein Blockdiagramm eines verteilten elektronischen Systems zum Tracing von Nutzern,
- Figuren 3A-3C: verschiedene Beispiele für die Erzeugung und Speicherung von Nutzerdaten ("User-Data-Package"),
- Figuren 4A-3C: verschiedene Beispiele für den Inhalt eines Check-in QR-Codes,
- Figuren 5A-5C: verschiedene Beispiele für die Erzeugung und Speicherung von Check-in Daten, wie sie auf dem Server gespeichert sind,
- Figuren 6A, 6B: Beispiele für Venue-relevante Zusatzdaten,
- Figur 7: ein UML-Diagramm eines Beispiels für das Tracing-Verfahren.

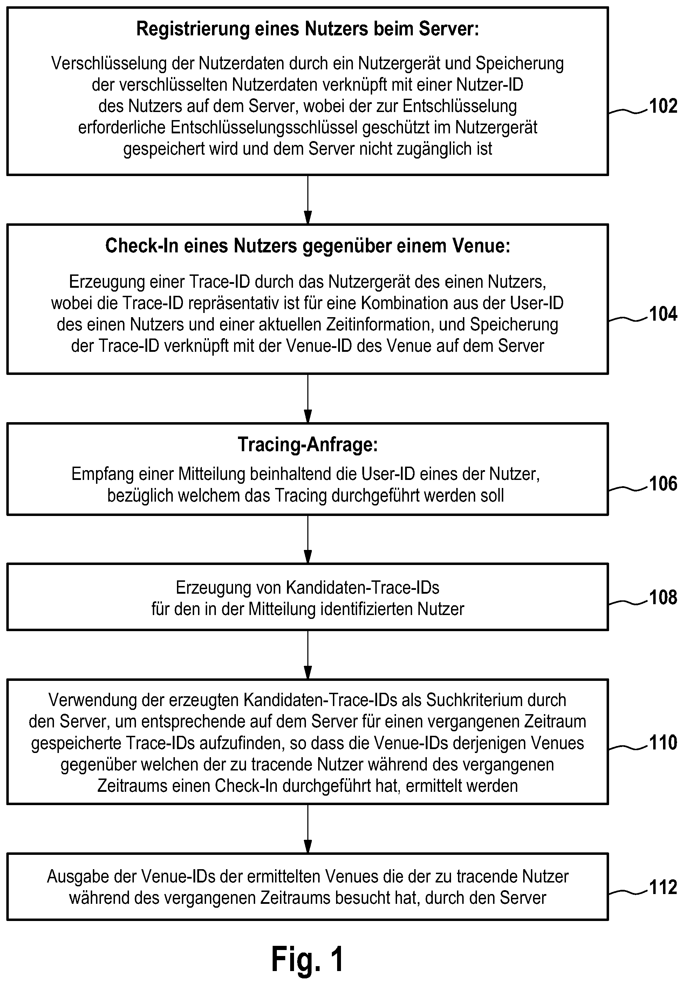

**Figur 1** zeigt ein Flussdiagramm eines Verfahrens zum Tracing von Nutzern. Nach Ausführungsform der Erfindung wird wie folgt vorgegangen:
   a) **Registrierungsschritt** 102: Für den Nutzer werden eine Reihe von nutzer-individuellen Daten erzeugt, die teilweise beim Server, gegenüber welchem die Registrierung erfolgt, hinterlegt werden. Insbesondere wird für den Nutzer eine User-ID und ein nutzerspezifischer Verschlüsselungsschlüssel (User-Data-Key) sowie optional weitere Datenwerte erzeugt. Der User-Data-Key kann z.B. ein symmetrischer Schlüssel sein. Der Nutzer spezifiziert seine beim Server verschlüsselt zu hinterlegenden persönlichen Daten (Nutzerdaten) wie z.B. Name und Anschrift, Kontaktadresse, verschlüsselt die Nutzerdaten mithilfe seines Nutzergeräts, d. h. einer darauf ausgeführten App, seine schutzbedürftigen Daten mit seinem User-Data-Key verschlüsselt. Optional können die Nutzerdaten zusätzlich auch durch einen (vorzugsweise täglich neu erstellten) öffentlichen Entitätenschlüssel (zum Beispiel einem Gesundheitsamtsschlüssel oder je nach Ausführungsform einem daraus abgeleiteten Schlüssel) verschlüsselt sein. Gemäß einer weiteren Ausführungsvariante sind die Nutzerdaten nur mit dem öffentlichen Entitätenschlüssel verschlüsselt. Die Ausführungsvariante mit der User-Data-Key basierten Ver-schlüsselung ist jedoch sicherer und bietet einen besseren Datenschutz. Die App auf dem Nutzergerät lädt diese verschlüsselten schutzbedürftigen Daten auf den Server. Der Server ordnet den von dem Nutzergerät des Nutzers empfangenen verschlüsselten schutzbedürftigen Daten eine User-ID zu und gibt diese User-ID an die App des Nutzers zurück. Diese User ID wird von der App in einem Speicher des Nutzergeräts gespeichert.
   b) **Check-in Schritt 104:** das Nutzergerät berechnet eine aktuelle Tracing-ID und übermittelt diese an ein Venue-Gerät zusammen mit anderen Check-in Daten, z.B. indem diese Daten in einem QR-Code kodiert und vom Venue-Gerät gescannt werden. Somit erhält das Venue-Gerät die Trace-ID von dem Nutzergerät des Nutzers und ggf. weitere Check-in Daten. Je nach Ausführungsform kann dies so erfolgen, dass die Trace-ID von dem Nutzergerät die User-ID im Klartext enthält wird oder in einer verschlüsselten bzw. verborgenen Form. In letzterem Fall kann der Server auf Basis der Trace-ID noch nicht einmal anonym einen Nutzer tracen; auch andere Erzeugungsverfahren für die Trace ID sind möglich, wenn man ein Tracing anhand der User-ID verhindern möchte. Das Venue-Gerät ergänzt zu der Trace-ID einen Zeitstempel, der die Check-in Zeit repräsentiert, und die Venue ID und schickt das (ggf. verschlüsselt) auf den Server. In anderen Ausführungsformen muss am Venue kein Venue-Gerät vorhanden sein. Das Nutzergerät liest die Venue-ID und ggf. weitere Venue-bezogene Daten ein (z.B. mittels eines QR-Codes auf einem Trägerobjekt oder über ein Nahfeldsignal), führt den Check-in mit einem den Venue repräsentierenden Modul der App durch und sendet die so erzeugten Check-in Daten mit der Venue-ID direkt an den Server.
   c) Check-out-Schritt: von Venue oder dem Nutzergerät wird ein Check-out timestamp an den Server geschickt und der User-ID (oder einem Chiffrat davon) zugeordnet.

### 3. Infektion/Notfallszenario:

a) Der Nutzer teilt dem Gesundheitsamt seine User ID und ggf. weitere Daten mit. Damit kann das Amt mit Hilfe der auf dem Server gespeicherten Daten feststellen, an welchen Venues der Nutzer z.B. in den letzten zwei Wochen gewesen ist. Hierzu kann ein Entitäts-Gerät des Amtes beispielsweise in Schritt 106 eine Mitteilung mit der User-ID dieses Nutzers an den Server senden;
b) Der Server erzeugt daraufhin in Schritt 108 auf Basis der User-ID und optional den weiteren Daten eine Vielzahl von Kandidaten-Trace-IDs;
c) Dann wird in Schritt 110 abgefragt, bei welchen Venues der zu tracende Nutzer in einem vergangenen Zeitraum einen Check-in vorgenommen hat. Hierfür wird eine Suche mit den Kandidaten-Trace-IDs in den Check-in-Datensätzen des Servers durchgeführt, um eine Liste der Venue-IDs der besuchen Venues zu erhalten;
d) In Schritt 112 werden die ermittelten Venue-IDs ausgegeben.
e) Dann wird in optionalen weiteren Schritten abgefragt, welche User-IDs gleichzeitig mit dem infizierten User in dem relevanten Zeitraum an den Venues waren.
f) Die verschlüsselten schutzbedürftigen Datensätze dieser User können dann mit dem privaten Entitätenschlüssel, z.B. Gesundheitsamtsschlüssel, entschlüsselt werden und vom Gesundheitsamt kontaktiert werden. Dies kann z.B. wie folgt erfolgen: es werden anhand der Venue-IDs, Check-in Zeiten und optional vorhandenen Check-Out-Zeiten, die alle jeweils im Klartext in einem Check-in Datensatz enthalten sind, alle Check-in Datensätze ermittelt, die zu Check-in Vorgängen gehören, die in zeitlicher Überlappung mit den Check-ins des zu tracenden Nutzers an den gleichen Venues erfolgt sind. Die so ermittelten Check-in daten der Kontaktpersonen (und möglichen Infizierten) enthalten beispielsweise verschlüsselte Ausweisdaten der jeweiligen Kontaktpersonen, die mit dem öffentlichen Entitätenschlüssel des Amts verschlüsselt sind, also vom Amt entschlüsselt werden können. Die entschlüsselten Ausweisdaten der Kontaktpersonen wiederum enthalten den Nutzerdaten-Entschlüsselungsschlüssel, mittels welchen das Amt die Nutzerdaten der Kontaktpersonen entschlüsseln kann, um die Kontaktpersonen darüber zu informieren, dass sie mit einem Infizierten für eine gewisse Zeit die gleiche Venue besucht haben.

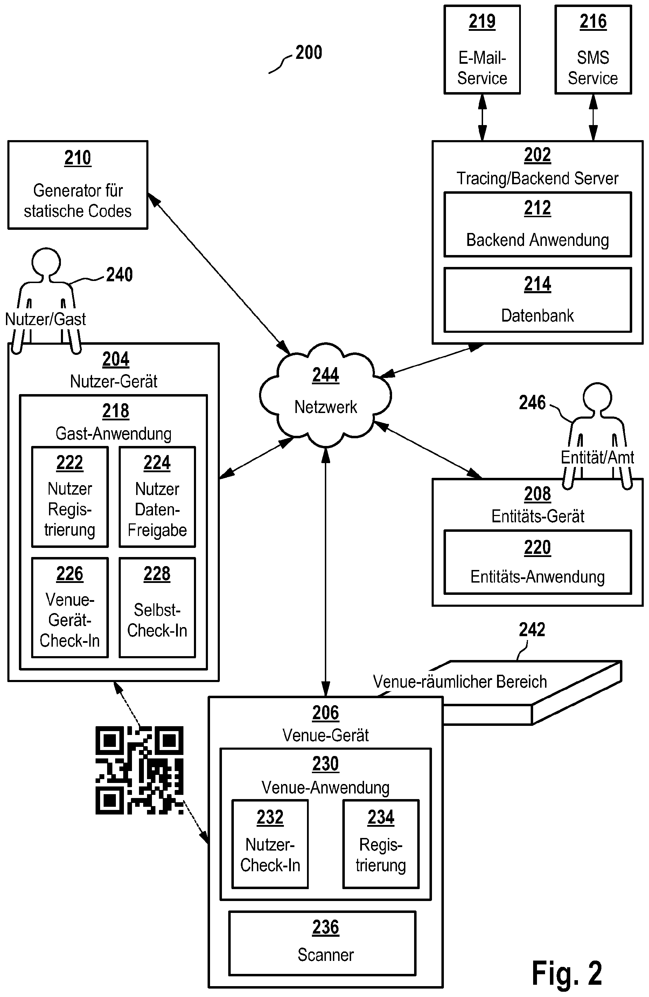

**Figur 2** zeigt ein Blockdiagramm eines verteilten elektronischen Systems 200 zum Tracing von Nutzern.

Das System 200 umfasst einen Server 202, welcher zum Beispiel von einem Betreiber eines Tracing-Dienstes bereitgestellt wird. Der Server kann eine Backend-Anwendung 212 umfassen, die verschiedene Funktionalitäten umfassen kann, zum Beispiel eine Funktionalität zur Registrierung von Nutzern 240, Venues 242, und Entitäten 246, und/oder zur Speicherung, Verwaltung und Durchsuchung von Nutzer- und Check-in Daten und/oder zur Erzeugung von Kandidaten-Trace-IDs. Zur Speicherung der Daten, insbesondere von Kontaktdaten der bei dem Server 202 registrierten Nutzer, Venues und Entitäten, zur Speicherung von Check-in-Datensätzen und/oder Tracing-Datensätzen kann der Backend Server eine Datenbank 214 beinhalten oder operativ an diese gekoppelt sein. Bei der Datenbank kann es sich zum Beispiel um eine relationale Datenbank oder eine andere Form von strukturierter Datenablage handeln.

In manchen Beispielen kann der Server einen E-Mail Service 218 und/oder einen SMS Service 216 umfassen, die dazu genutzt werden können, die bei dem System registrierten Nutzer im Bedarfsfall zu kontaktieren und beispielsweise zu warnen, dass sie sich gleichzeitig mit einer infizierten Person in einem bestimmten Venue aufgehalten haben. Der SMS-Service kann zur Validierung von Telefonnummern verwendet werden.

Das System umfasst gemäß Ausführungsformen der Erfindung ferner mehrere Nutzergeräte 204, die jeweils einem Nutzer 240 zugewiesen sind. Bei den Nutzergeräten handelt es sich um Datenverarbeitungsgeräte, insbesondere um portable Datenverarbeitungsgeräte, zum Beispiel Mobilfunkgeräte. Bei den Nutzern, denen die Nutzergeräte jeweils zugeordnet sind, handelt es sich um Personen, die sich temporär in verschiedenen räumlichen Bereichen (Venues) aufhalten können und somit als (zahlende oder nicht zahlende) Gäste des Venues fungieren können. Auf jedem der Nutzergerät kann ein Anwendungsprogramm 214, hier als Gast-Anwendung bezeichnet, instanziiert sein, welches mit dem Backend-Anwendungsprogramm 212 des Servers 202 interoperabel ist. Das Anwendungsprogramm 218 kann zum Beispiel als App ausgebildet sein und verschiedene Funktionen umfassen. Zu den Funktionen können zum Beispiel eine Funktion 222 zur Registrierung des Nutzers gegenüber dem Server 202, eine Funktion 226 zum Check-in des Nutzers 240 gegenüber einem Venue, eine Funktion 228 zum Selbst-Check-in gegenüber einem Venue, der anstatt über ein Venue-Gerät nur über ein Trägerobjekt mit einer automatisch erfassbaren Venue-ID ausgestattet ist, und/oder einer Funktionalität 224 zur Übermittlung eines Tracinggeheimnis-Transferobjekts mit nutzerbezogenen sensiblen Daten, um einer Entität 246 Zugriff auf die Nutzerdaten des Nutzers zu gewähren, gehören.

Gemäß Ausführungsformen der Erfindung umfasst das System ferner mehrere Entitäts-Geräte 208, die jeweils einer Entität 246 zugewiesen sind. Bei der Entität handelt es sich um eine vertrauenswürdige, im allgemeinen hoheitliche Instanz, zum Beispiel ein Amt oder eine Behörde, zum Beispiel ein Gesundheitsamt. Damit die Entität das Tracing-System nutzen kann, muss es sich gegenüber dem Server 202 registriert haben, wobei im Zuge der Registrierung der Entität ein asymmetrisches kryptographisches Entität-Schlüsselpaar erzeugt wird, das gemäß Ausführungsformen der Erfindung regelmäßig, zum Beispiel täglich, durch ein aktuell gültiges Schlüsselpaar ersetzt wird. Der öffentliche Teil dieses Schlüsselpaares wird an den Server 202 bereitgestellt, um diesem zu ermöglichen, den öffentlichen Verschlüsselungsschlüssel der Entität an die Nutzergeräte 204 und/oder die Venue-Geräte 206 zur Verfügung zu stellen, sodass diese mithilfe des öffentlichen Entitäten-schlüssels bestimmte Teile der Check-in Datensätze (insb. die Ausweis-Datensätze) und optional auch die Nutzerdaten verschlüsseln können, bevor die besagten Daten zum Server hochgeladen und dort zentral in der Datenbank 214 gespeichert werden. Es ist möglich, dass sich mehrere verschiedene Entitäten (zum Beispiel Gesundheitsbehörde) bei dem Server registriert haben und dass zu jeder Entität mehrere Entität-Geräte gehören, zum Beispiel Datenverarbeitungsgeräte (Computer) der Mitarbeiter der jeweiligen Behörde. Nach Ausführungsformen der Erfindung können mehrere Entitäten dadurch in das System integriert werden, dass sie das gleiche asymmetrische Entitäten-Schlüsselpaar zur Ver- und Entschlüsselung von Check-in-Daten und/oder Nutzerdaten verwenden, was zum Beispiel dadurch realisiert sein kann, dass die Entitäts-Anwendung eine Funktionalität zur Synchronisation sowohl des öffentlichen als auch des privaten Anteils dieses asymmetrischen kryptographischen Schlüsselpaares mit allen anderen Entitäts-Anwendungen der gleichen oder einer kooperierenden Entität (oder aller Entitäten, die funktional als eine Entität fungieren sollen) beinhaltet. Vorzugsweise werden zumindest die privaten Teile des Schlüsselpaars in verschlüsselter Form ausgetauscht und synchronisiert, zum Beispiel indem sie mit einem öffentlichen Transportschlüssel des jeweiligen Empfänger-Entität-Gerät verschlüsselt werden.

Gemäß einer Ausführungsform wird die Synchronisation der Entitäten-Schlüssel zwischen mehreren Mitarbeitern und Entitäten-Geräten einer Entität durch tägliche Schlüsselrotation realisiert: Die Schlüssel werden durch eine Key-ID (Ganzzahl) identifiziert, die mit jedem neuen Schlüssel inkrementiert wird. Die Rotation wird von jedem Mitarbeiter des Gesundheitsamtes ausgelöst, der sich nach Ablauf der Gültigkeit des letzten für z.B. einen Tag gültigen öffentlichen Entitäten-Schlüssels einloggt. Der tägliche öffentliche Entitäten-Schlüssel ist das alleinige Eigentum der Gesundheitsbehörden und wird verwendet, um die Check-in Daten des Nutzers zu sichern.

Das Rotationsverfahren beinhaltet, dass im Zuge der Registrierung und/oder im Zuge eines Check-ins eines Nutzers der jeweils aktuell gültige öffentliche Tagesschlüssel der Entität an das Nutzergerät übertragen wird, sodass das Nutzergerät den Schlüssel zur Verschlüsselung der Ausweisdaten bei dem Check-in und optional auch der Nutzerdaten bei der Nutzerregistrierung verwenden kann. Die Ablaufzeit eines täglichen Schlüsselpaares beträgt z.B. 24 Stunden. Wenn ein tageweise gültiges Entitätenschlüsselpaar abgelaufen ist, generiert der Mitarbeiter des Amts ein neues für den aktuellen Tag gültiges Entitätenschlüsselpaar, dessen öffentlicher Schlüssel von der Entitäten-Applikation zum Server hochgeladen wird und vom Server über das Netzwerk automatisch an die Nutzergeräte der Nutzer verteilt wird. Außerdem laden sich die teilnehmenden Entitäten-Geräte die jeweiligen (statischen) öffentlichen Schlüssel der anderen Entitäten-Geräte der Entität herunter. Das Entitäten-Gerät, das eben das tagesaktuelle Entitäten-Schlüsselpaar erzeugt hat, verschlüsselt mit den öffentlichen statischen Schlüsseln der jeweils anderen Entität-Geräte den aktuellen privaten tageweise gültigen EntitätenSchlüssel, und sendet dieses so gebildete Chiffrat an das betreffende Entitäten-Gerät, das das Chiffrat entschlüsselt und somit ebenfalls im Besitz des tageweise gültigen privaten Entitäten-Schlüssels ist. Es ist auch möglich, dass verschiedene Gesundheitsämter durch das besagte Schlüsselrotationsverfahren sich täglich (oder in einem anderen vordefinierten Zeitintervall) neue private Entitätenschlüssel zusenden.

Dieser Prozess geschieht vorzugsweise vollautomatisch im Hintergrund ohne aktives Zutun der Mitarbeiter des Amts und/oder der einzelnen Nutzer.

Gemäß Ausführungsformen der Erfindung umfasst das System ferner mehrere Venue-Geräte 206 mehrerer verschiedener Venues 242. Es ist möglich, dass an einem Venue mehrere Venue-Geräte 206 angebracht sind, zum Beispiel jeweils ein Gerät an jedem von mehreren Eingängen zu dem Venue. Auf jedem Venue-Gerät kann eine Venue Anwendung 230 instanziiert sein, die zum Beispiel Funktionalitäten 232, 234 für einen Check-in Vorgang eines Nutzers gegenüber dem Venue und/oder für die Registrierung des Venues gegenüber dem Server 202 beinhaltet. Beispielsweise kann das Venue-Gerät 206 eine Kamera beinhalten, die dazu ausgebildet ist, einen QR-Code, der auf einem Display des Nutzergeräts 204 angezeigt ist und in welchem Check-in Daten eines Gastes kodiert sind, zu erfassen. Der QR-Code wird von der Venue-Anwendung 230 dekodiert und zumindest einige der dekorierten Daten werden verknüpft mit einer Venue-ID des Venues 242 auf dem Server 204 gespeichert.

Optional kann das System ein oder mehrere weitere Datenverarbeitungsgeräte 210 für die Erzeugung statischer graphischer Codes beinhalten. Dies kann vorteilhaft sein, da hierdurch auch Personen das Tracing-System 200 nutzen können, die nicht sehr IT-affin sind und keinen eigenen Nutzeraccount anlegen können oder wollen. Beispielsweise kann ein Mitarbeiter einer Pflegeeinrichtung sich oder die Pflegeeinrichtung als Nutzer gegenüber dem Server 202 registrieren und das Datenverarbeitungsgerät 210 dazu veranlassen, eine bestimmte Menge, zum Beispiel 50 oder 100 Stück, eines QR-Codes zu erstellen. Diese vorab erstellten und noch nicht personalisiert QR-Codes können dazu verwendet werden, an die Gäste des Pflegeheimes verteilt zu werden, um diesen zu ermöglichen, ein Venue zu besuchen (zum Beispiel einen Konzertraum, ein Theater, etc.), ohne hierfür einen eigenen Account anlegen zu müssen. Die Personen, die die QR-Codes bestellt und zum Beispiel per Post oder über das Internet empfangen und ausgedruckt hat, verteilt die QR-Codes an die Gäste des Pflegeheimes und notiert im Zuge der Ausgabe eine Zuweisung des ausgegebenen QR-Codes an eine konkrete Person.

Beispielsweise können die bestellten QR-Codes mit einer Seriennummer versehen sein, und das Pflegepersonal erstellt handschriftlich oder elektronisch eine Liste, in welcher eine Zuweisung des ausgegebenen Codes an diejenige Person enthalten ist, die den Code erhalten hat. Die Liste kann zum Beispiel auf Papier erstellt werden, sie kann aber auch über eine Funktionalität eine Anwendung auf dem Generatorgerät 214, die zum Beispiel über das Internet bereitgestellt wird, erstellt und gespeichert werden.

Die einzelnen Komponenten des Systems sind über Netzwerk 244 miteinander verbunden. Bei dem Netzwerk kann es sich zum Beispiel um das Internet handeln, wobei Daten kabelgebunden und/oder drahtlos übermittelt werden kann. Beispielsweise kann die Datenübertragung zwischen Nutzergeräten 204 und Server 202 über eine Mobilfunkverbindung oder WLAN erfolgen. Die Übertragung von Check-in Daten von dem Nutzergerät eines Nutzers an ein Venue Gerät 206 kann beispielsweise über einen optischen Datenübertragungskanal und/oder über ein Nahfeldsignal wie zum Beispiel Bluetooth erfolgen.

Untenstehende Tabelle zeigt verschiedene Verfahrensschritte gemäß einer Ausführungsform der Erfindung, die von verschiedenen Systemkomponenten ausgeführt werden, sowie die jeweiligen Input und Output Daten.

| **Schritte** | **Ziel** | **Inputdaten** | **Outputdaten** |
|---|---|---|---|
| Venue Registrierung (bei dem Server) | Ermöglicht es privaten und gewerblichen Venue-Betreibern, Gäste automatisch zu registrieren und im Notfall rückwirkend zu tracen. | E-Mail, Passwort, Standort-informationen | Venuespezifisches asym. Schlüsselpaar venuePrivKey und VenuePubKey |
| Entitäten Registrierung (bei dem Server) | Ermöglicht es Angestellten eines Amtes, Besucher der Venues zu tracen | Zertifikat | Entitätsspezifisches asym. Schlüsselpaar: healthPrivKey health-PubKey |
| Nutzer Registrierung (bei dem Server) | Erlaubt es dem Nutzer, mittels des Systems automatisch einzuchecken bzw. auszuchecken | Nutzerdaten, insb. Adresse und Kontaktdaten | anonymisierter QR Code, userPrivKey userPubKey, mehrere Geheimnisse |
| Check-in/out eines Nutzers bei einer Venue | Übermittlung anonymer Kontaktinformation an den Venue-Betreiber Scanner check-in Selbst-Check-in mittels QR Code auf Trägerobjekt | anonymer QR Code | Hochgeladene Check-in Daten |
| Kontakt-Tracing (Notfall/Infektion) | Übermittlung von Nutzerkontaktdaten ans Gesundheitsamt Aufdeckung möglicher Kontaktpersonen durch das Gesundheitsamt, optional nur nach Freigabe durch Venue | TAN des infizierten Nutzers, privater Entschlüsselungsschlüssel des Gesundheitsamts healthPrivKey | Vom Infizierten besuchte Venues, Kontaktdaten möglicher Kontaktpersonen |

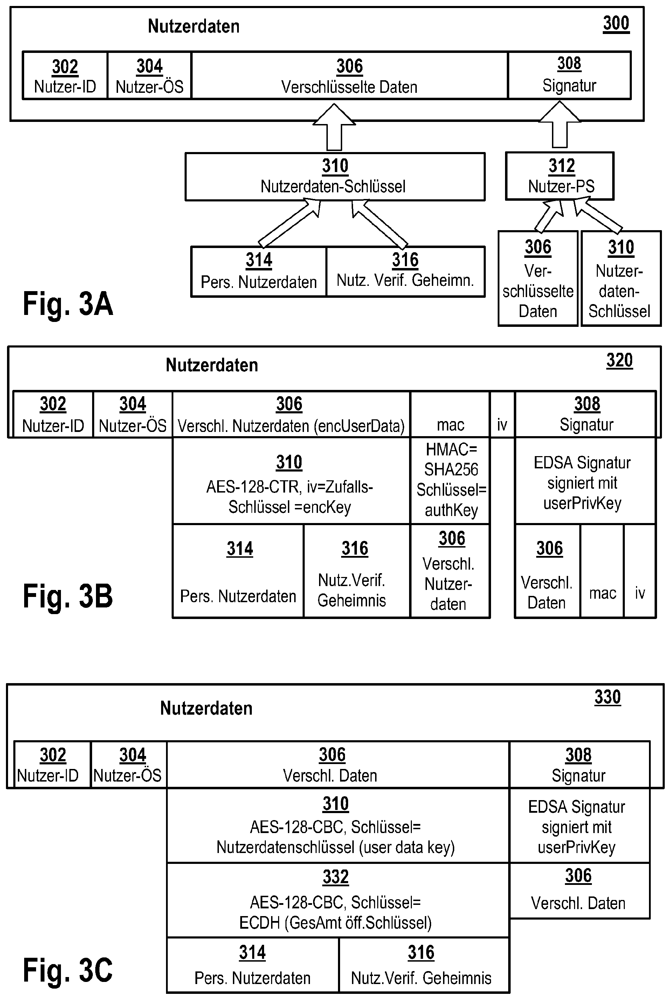

**Figuren 3A-3C** zeigen verschiedene Beispiele für die Erzeugung und Speicherung von Nutzerdaten ("User-Data-Package", "Nutzerdaten-Datensatz").

**Figur 3A** zeigt einen Nutzerdaten-Datensatz 300, wie er gemäß einer Ausführungsform auf dem Server 202 gespeichert ist. Der Datensatz 300 umfasst zumindest eine Nutzer-ID 302 im Klartext, mit welcher weitere nutzerbezogene Daten verknüpft auf dem Server gespeichert sind. Zu diesen weiteren Daten gehören zumindest verschlüsselte Nutzerdaten 306 einer Person. Optional können weitere personenbezogene Daten in dem Nutzerdaten-Datensatz 300 enthalten sein, zum Beispiel ein öffentlicher Schlüssel 304 eines asymmetrischen kryptographischen Schlüsselpaares, welches spezifisch für diesen Nutzer im Zuge dessen Registrierung ausgestellt wurde. Optional können die Nutzerdaten weitere Daten wie zum Beispiel eine Signatur 308 enthalten.

Im unteren Teil der Figur 3A ist dargestellt, wie verschiedene Teile der Nutzerdaten 300 erzeugt werden können. Beispielsweise können die verschlüsselten Daten 306 dadurch erzeugt werden, dass die persönliche Nutzerdaten 314 des Nutzers, also zum Beispiel der Name, die Adresse und/oder sonstige Kontaktinformationen des Nutzers, mit einem Nutzerdaten-Verschlüsselungsschlüssel 310 verschlüsselt werden. Beispielsweise kann es sich bei dem Schlüssel 310 um einen symmetrischen kryptographischen Schlüssel handeln, der im Zuge der Registrierung des Nutzers spezifisch für diesen Nutzer erzeugt wurde und welcher gleichzeitig auch eine Entschlüsselung der verschlüsselten Daten 306 ermöglicht. Vorzugsweise werden die persönlichen Nutzerdaten 314 zusammen mit einem nutzerspezifischen Verifikationsgeheimnis 316 von dem Nutzerdaten-Schlüssel 310 verschlüsselt und/oder das Geheimnis 316 wird als Parameter im Zuge der Verschlüsselung zur Erzeugung der verschlüsselten Nutzerdaten 306 verwendet.

In einer alternativen Ausführungsform wird im Zuge der Registrierung des Nutzers der UserDataKey als asymmetrisches kryptographisches Schlüsselpaar erzeugt. In diesem Fall muss nur der zur Entschlüsselung verwendete private Teil geschützt im Nutzergerät gespeichert und als Bestandteil der Ausweisdaten bzw. Check-in Daten beim Check-in in verschlüsselter Form an den Server übermittelt werden.

Die optionale Signatur 308 kann beispielsweise dadurch erzeugt werden, dass ein privater kryptographischer Schlüssel (Signaturschlüssel) 312, der spezifisch für den Nutzer bei dessen Registrierung erzeugt wurde, die verschlüsselten Nutzerdaten 306 und den zur Verschlüsselung der Nutzerdaten 314 verwendeten Nutzerdaten-schlüssel 310 signiert. Bei dem öffentlichen Schlüssel 304 kann es sich um einen zu dem Schlüssel 312 korrespondierenden Signaturprüfschlüssel handeln. Dies erlaubt es dem Amt, zu prüfen, ob der Nutzerdatensatz nach dessen Speicherung und Signierung eventuell nachträglich manipuliert wurde.

In **Figur 3B** ist ein Nutzerdaten-Datensatz 320 gemäß einer weiteren Ausführungsform dargestellt. Datenwerte bzw. Schlüssel des Datensatzes 320, die einem der Datenwerte bzw. Schlüssel des Datensatzes 300 inhaltlich und/oder funktional entsprechen, sind mit den gleichen Referenznummern gekennzeichnet. Die mit dem Rahmen umgrenzte Box kennzeichnet den Nutzerdatensatz, wie er auf dem Server gespeichert ist. Die darunter stehenden Boxen repräsentieren Inputdatenwerte bzw. Schlüssel, die zur Erzeugung entsprechender Elemente des Nutzerdatensatzes verwendet wurden. Bei dem mac handelt es sich um einen Message Authentication Code, der z.B. mittels eines HMAC-SHA256 Algorithmus auf Basis der verschlüsselten Nutzerdaten 306 erstellt wurde. Das Kürzel "iv" repräsentiert einen Zufallswert. Die Signatur umfasst in diesem Fall nicht nur die verschlüsselten Nutzerdaten 306, sondern auch den mac und den Zufallswert iv.

In **Figur 3C** ist ein Nutzerdaten-Datensatz 330 gemäß einer weiteren Ausführungsform dargestellt. Datenwerte bzw. Schlüssel des Datensatzes 330, die einem der Datenwerte bzw. Schlüssel des Datensatzes 300 inhaltlich und/oder funktional entsprechen, sind mit den gleichen Referenznummern gekennzeichnet. Die mit dem Rahmen umgrenzte Box kennzeichnet den Nutzerdatensatz, wie er auf dem Server gespeichert ist. In der hier gezeigten Ausführungsform werden die verschlüsselten Nutzerdaten 306 durch eine zweifache Verschlüsselung gebildet. In einem ersten Verschlüsselungsschritt werden die persönlichen Nutzerdaten 314 sowie vorzugsweise auch ein Nutzerverifikationsgeheimnis 316 mit einem öffentlichen Entitäten-Schlüssel 332 verschlüsselt. Das in diesem Schritt erzeugte Chiffrat wird in einem weiteren Verschlüsselungsschritt mit dem nutzerspezifisch erstellten User-Data-Key 310 erneut verschlüsselt, um die (doppelt) verschlüsselten Nutzerdaten 306 zu erhalten und auf dem Server zu speichern.

Gemäß einer Ausführungsform stellt der Benutzer der App (Anwendungsprogramm) 218 seines Nutzergeräts seine Kontaktinformationen 314 im Zuge eines Registrierungsprozesses zur Verfügung. Die Anwendung erzeugt ein EC secp256r1-Schlüsselpaar (user public key und user private key) 312, 304, ein Benutzer-Trace-Geheimnis 410, d.h. ein user trace secret (16 Bytes Entropie), ein Benutzer-Verifizierungs-Geheimnis 412, d.h. user verification secret (16 Bytes Entropie) und einen symmetrischen Benutzer-Datenschlüssel, d.h. user data key (16 Bytes Entropie) 310.

Der Nutzerdaten-Schlüssel 310 wird zur Verschlüsselung der persönlichen Nutzerdaten 314 verwendet, um die verschlüsselten Nutzerdaten 306 zu erhalten.

In manchen Ausführungsformen in welchen zusätzlich eine Verschlüsselung durch einen Entitäten-Schlüssel erfolgt, kann die Anwendung einen Verschlüsselungsschlüssel mit ECDH mit dem privaten Schlüssel des Benutzers und dem öffentlichen Hauptschlüssel (Entitätenschlüssel) des Gesundheitsamtes berechnen. Die App 218 berechnet ein HMAC-SHA256 mit der ECDH-Ausgabe als Daten und dem Datenschlüssel als Schlüssel. Die Anwendung verschlüsselt dann die persönlichen Informationen und das Benutzerverifizierungsgeheimnis mit diesem Verschlüsselungsschlüssel, zum Beispiel mit AES-128-CBC.

Die Anwendung 218 lädt dann diese verschlüsselten Daten auf den Server 202 und signiert sie mit dem privaten Schlüssel 312 des Benutzers zusammen mit dem öffentlichen Schlüssel des Benutzers. Der Server gibt eine Benutzerkennung, d.h. U-ser-ID, zurück.

Im Nutzergerät werden gespeichert:
- user id (uuid) 302,
- user public key (ec secp256r1) 304,
- user private key (ec secp256r1) 312,
- user data key (16 bytes random data) 310,
- user trace secret (16 bytes random data) 410,
- user verification secret (16 bytes random data) 412.

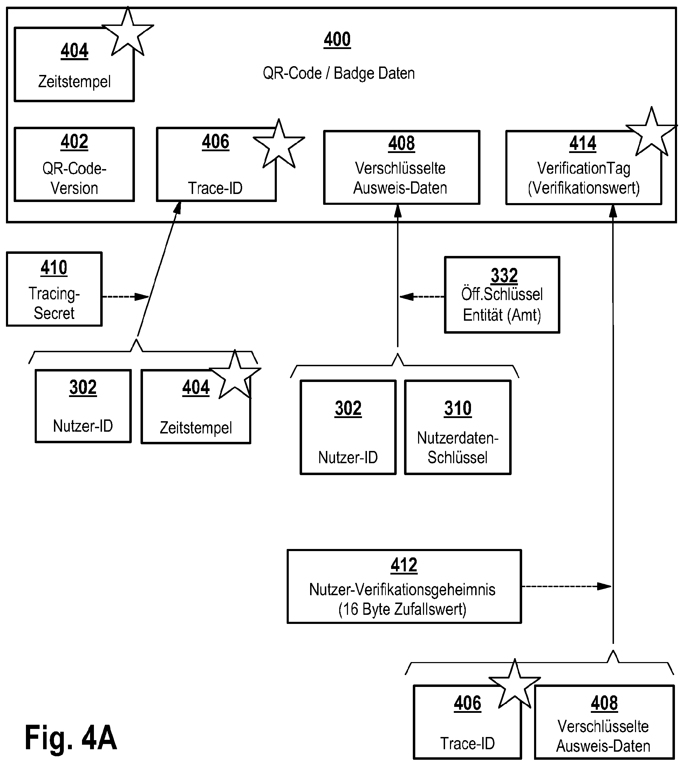

**Figuren 4A-3C** zeigen verschiedene Beispiele 400, 420, 430 für den Inhalt eines Check-in QR-Codes. Falls nicht ein graphischer Code zur Übermittlung der Check-in-Daten eines Nutzers an das Venue-Gerät verwendet wird, sondern zum Beispiel ein Nahfeld-Signal, sind die bezeichneten Daten nicht in Form eines graphischen Codes, sondern als Nahfeldsignal, zum Beispiel Bluetooth Signal, repräsentiert.

Beispielsweise kann das Nutzergerät eine eigene Uhr, insbesondere eine Echtzeituhr, beinhalten und/oder eine aktuelle Uhrzeit regelmäßig über das Netzwerk von einem Uhrzeit-Server empfangen.

Wenn der Nutzer Vorkehrungen zur Durchführung eines Check-ins gegenüber einem Venue durchführt, also zum Beispiel eine App startet oder ein entsprechendes GUI-Element der App bedient, zum Beispiel um einen graphischen Check-in Code zu erzeugen, erfasst das Nutzergerät einen aktuellen Zeitwert 404 und führt eine Ableitfunktion auf diesem Zeitwert 404 und der Nutzer-ID 302 aus, um eine Trace-ID 406 zu erhalten. Vorzugsweise geht diese Ableitung ein Tracing-Secret 410 ein, dass zum Beispiel ein Zufallswert sein kann, der dem Nutzer im Zuge der Registrierung zugewiesen und geschützt auf dem Nutzergerät gespeichert ist. Vorzugsweise stellt die Art der Ableitfunktion sicher, dass aus der Trace-ID die Nutzer-ID nicht im Klartext hervorgeht und vorzugsweise es auch nicht anhand der Trace-IDs ersichtlich ist, ob sie demselben oder unterschiedlichen Nutzern angehören. Der Umstand, dass eine aktuelle Zeitinformation 404 in die Trace-ID 406 eingeht, ist durch den Stern symbolisiert.

In analoger Weise können sich auch Venues, repräsentiert jeweils durch ein oder mehrere Venue-Geräte, bei dem Server registrieren. Zu den im Zuge der Registrierung hinterlegten Daten können u.a. gehören: Name des Standortes, Name und E-Mail der Kontaktperson, Adresse des Standortes, Koordinaten des Standortes und/oder Eincheck-Radius.

Der Datensatz 400 wie in **Figur 4A** gezeigt enthält außerdem verschlüsselte Ausweisdaten 408, die dadurch gebildet werden, dass mit einem öffentlichen Verschlüsselungsschlüssel 332 der Entität die Nutzer-ID 302 des Nutzers und der nutzerspezifische Entschlüsselungsschlüssel 310 (zum Beispiel der symmetrische User-Data-Key) verschlüsselt werden. Durch die Ausweisdaten 408, in welchen die User-ID verschlüsselt enthalten ist, ist also eine Zuweisung dieses Check-in Datensatzes an einen konkreten Nutzer möglich, allerdings nur für eine Partei, die die Ausweisdaten 408 entschlüsseln kann (also die Entität). Da die Ausweisdaten aber nur die Nutzer-ID und nicht die gesamten Nutzerdaten 314 enthalten, sind diese so klein, dass sie sich für die Kodierung innerhalb eines QR-Codes eignen. Wenn das Amt mit seinem privaten Entitätenschlüssel die verschlüsselten Ausweisdaten 408 entschlüsselt, erfährt es nicht nur die User-ID des Nutzers, zu welchen dieser Check-in Datensatz 400 gehört, sondern erhält auch den Entschlüsselungsschlüssel 310, der notwendig ist, um die Kontaktdaten des Nutzers in Erfahrung zu bringen.

Optional kann der QR-Code-Datensatz 400 auch einen Verifikationsdatenwert 414 enthalten, welcher einen zusätzlichen Schutz bietet, da er der Entität eine Prüfung erlaubt, ob der Check-in Datensatz manipuliert wurde.

Gemäß einer Ausführungsform wird der Verifikationsdatenwert 414 dadurch erzeugt, dass die Trace-ID 406 und die verschlüsselten Ausweisdaten 408 mit einem Nutzer-Verifikationsgeheimnis 412 verschlüsselt werden. Bei dem Nutzer-Verifikationsgeheimnis handelt es sich vorzugsweise um eine Zufallszahl, die im Zuge der Registrierung des Nutzers bei dem Server erzeugt wird und auf einem Speicher des Nutzergerätes des Nutzers geschützt gespeichert wird. Falls ein unberechtigter Nutzer also versuchen sollte, eine Trace-ID eines anderen Nutzers zu stehlen und einen manipulierten Check-in Datensatz mit der gestohlenen Trace-ID zu verwenden, so wäre es dem unberechtigten Nutzer nicht möglich, den zu dieser Trace-ID passenden Verifikationsdatenwert 414 zu erzeugen, solange sich der unberechtigte Nutzer nicht auch noch in den Besitz des Nutzergeräts des berechtigten Nutzers begibt, da nur dort das Nutzer-Verifikationsgeheimnis 412 gespeichert ist, das zur Ableitung des Verifikationsdatenwertes 414 erforderlich ist.

Das Nutzergerät ist dazu konfiguriert, regelmäßig zu prüfen, ob die aktuell erzeugten Check-in Daten noch aktuell sind. Falls die Trace-ID Ableitfunktion vorsieht, immer die aktuelle Zeit abgerundet auf die Minute als Zeitstempel 404 zur Ableitung der Trace-ID 406 zu verwenden, gilt ein QR-Code bzw. Check-in Datensatz 400 als veraltet, sobald die neue Minute begonnen hat. In diesem Fall verwirft das Nutzergerät automatisch den aktuellen Check-in Datensatz 400 bzw. den QR-Code 400 und erzeugt wie oben beschrieben einen neuen, aktuellen Check-in Datensatz auf Basis der aktuellen Zeitinformation 404, also auf Basis zum Beispiel der neu begonnenen abgerundeten Minute.

Falls dem Venue kein Venue-Gerät zugewiesen ist, sondern lediglich ein Trägerobjekt, auf oder an welchem die Venue-ID so angebracht ist, dass das Nutzergerät diese automatisch erfassen kann, kann die Erzeugung des Check-in Datensatzes 400 vom Nutzergerät ausgeführt werden, ohne dass dieser an ein Venue-Gerät übermittelt wird und ohne dass dieser Angaben zu einer QR-Code Version oder sonstige technische Parameter zur Datenübertragung an ein Venue-Gerät enthält. Vielmehr kann ein solcher Datensatz dann direkt mit der vom Nutzergerät empfangenen Venue-ID angereichert und als fertiger Check-in-Datensatz an den Server übermittelt werden.

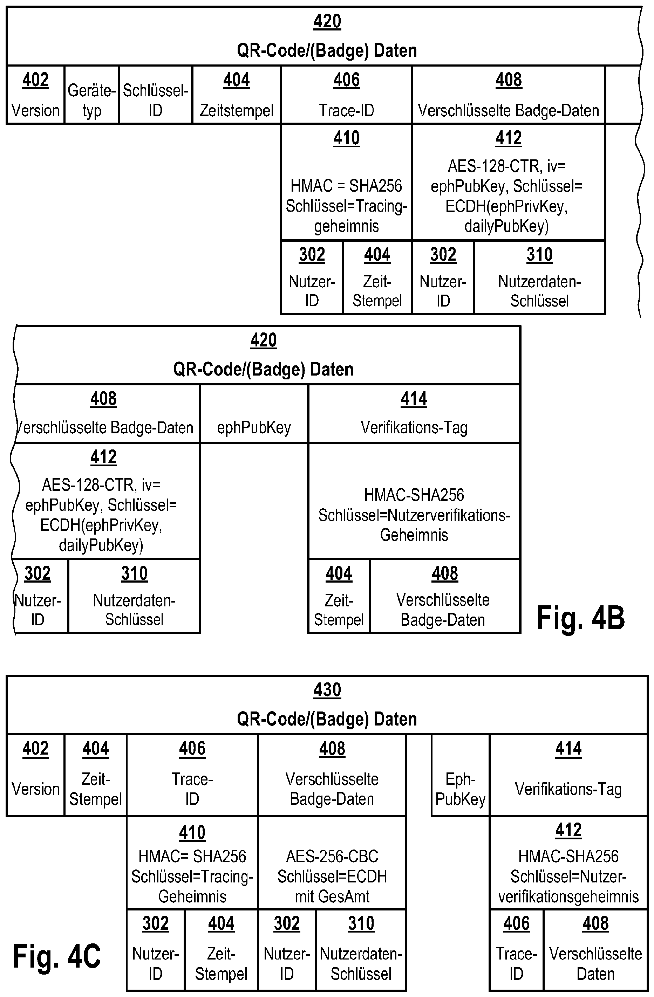

In **Figur 4B** ist ein Check-in-Datensatz 420 bzw. ein QR-Code, der diesen kodiert, gemäß einer weiteren Ausführungsform dargestellt. Datenwerte bzw. Schlüssel des Datensatzes 420, die einem der Datenwerte bzw. Schlüssel des Datensatzes 400 inhaltlich und/oder funktional entsprechen, sind mit den gleichen Referenznummern gekennzeichnet. Die ersten beiden Zeilen der Datenstruktur der Figur 4B zeigen den Teil des Check-in Datensatzes 420, wie er (bei den Check-in Varianten mit Venue-Gerät) an das Venue-Gerät übermittelt wird. Die darunter stehenden Boxen repräsentieren Inputdatenwerte bzw. Schlüssel, die zur Erzeugung entsprechender Elemente des Datensatzes 420 verwendet wurden. Die Datenstruktur 420 enthält noch weitere optionale Datenwerte wie zum Beispiel die Version 402 des zur Erzeugung des QR-Codes verwendeten Algorithmus ("QR-Code Version"), den Gerätetyp des Nutzergerätes, einen Identifikator des (täglich aktualisierten) asymmetrischen kryptographischen Entitäten-Schlüsselpaares, dessen öffentlicher Teil zur Verschlüsselung der Ausweis-Daten verwendet wurden, etc. Zusätzlich oder alternativ zu der im Klartext vorhandenen Check-in Zeit 404 kann auch eine Check-out Zeit enthalten sein.

In **Figur 4C** ist ein Check-in-Datensatz 430 bzw. ein QR-Code, der diesen kodiert, gemäß einer weiteren Ausführungsform dargestellt. Im Unterschied zu den Datenstrukturen 400 und 420 ist bei der Datenstruktur 430 der Nutzerverifikationsdatenwert 414 von der aktuellen Zeit indirekt (über die Trace-ID) abgeleitet.

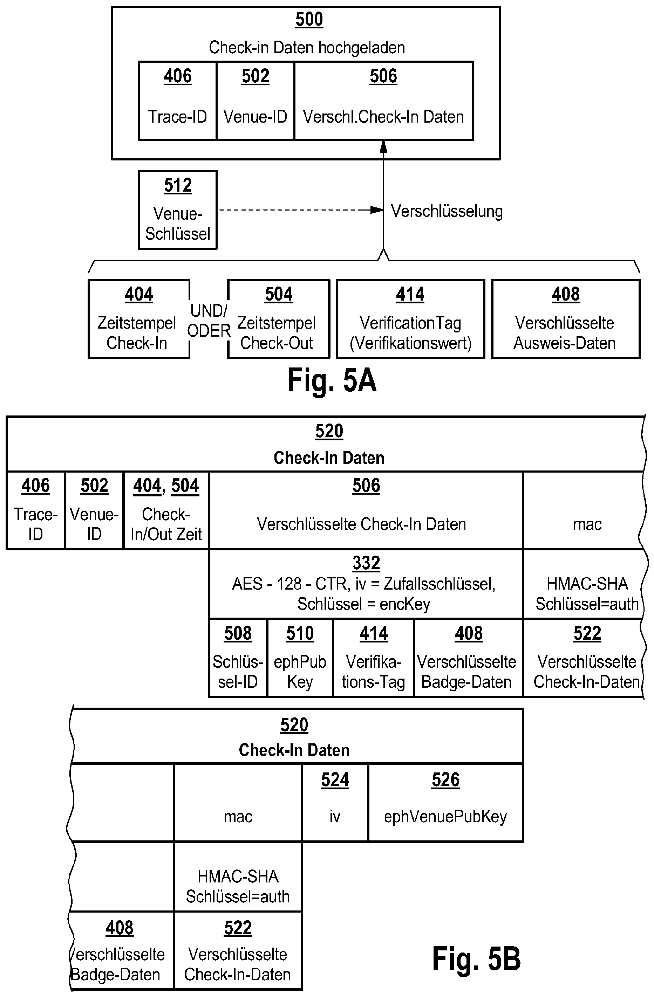

**Figuren 5A-5C** zeigen verschiedene Beispiele für die Erzeugung und Speicherung von Check-in Daten, wie sie auf dem Server gespeichert werden.

Im Zuge des Check-Prozesses empfängt das Venue-Gerät einen Check-in Datensatz 400, 420, 430 von dem Nutzergerät des Nutzers. Die in dem empfangenen Datensatz enthaltenen Daten können, wie sie sind, mit weiteren Daten, insbesondere der Venue-ID, verknüpft werden, um einen kompletten Check-in Datensatz zu erhalten, der auf dem Server gespeichert wird. Somit können die Check-in Datensätze des Servers aussehen wie die Datensätze 400, 420, 430, jeweils verknüpft mit weiteren Daten, insbesondere der Venue-ID des Venues des Venue-Gerätes.

Zumindest einige der Check-in Datensätze können faktisch Check-out Datensätze sein, und zusätzlich oder alternativ zu der Check-in Zeit 404 die Check-out Zeit 504 beinhalten.

In manchen Ausführungsformen führt das Venue-Gerät weitere Datenverarbeitungsschritte mit den vom Nutzergerät erhaltenen Check-in Daten 400, 420, 430 durch, um auf Basis der von dem Nutzergerät empfangenen Check-in Datensätze die fertigen, an den Server zu übermittelnden Check-in Datensätze zu erzeugen.

Beispielsweise kann jedem Venue im Zuge der Registrierung ein symmetrisches oder asymmetrisches Schlüsselpaar zugewiesen werden und das Venue-Gerät kann den privaten Teil des Schlüsselpaares (im asymmetrischen Fall) dazu verwenden, zumindest Teile jedes Check-in Datensatzes (abgesehen von der Trace-ID, die im Klartext vorliegenden muss), zu verschlüsseln. Manche Teile des Check-in Datensatzes liegen also in zweifach oder gar dreifach verschlüsselter Form vor: so sind zum Beispiel die Ausweisdaten 408 mit dem öffentlichen Schlüssel 332 der Entität erzeugt worden, sie können optional zusätzlich mit dem user-Data-Key 310 des Nutzers verschlüsselt sein, und optional außerdem mit dem öffentlichen Venue-Schlüssel. Dies kann den Vorteil haben, dass die Entität ein Tracing von Nutzern, die einen Venue besucht haben, nur mit Zustimmung des Betreibers des Venues vornehmen kann, da der Betreiber "seine" Check-in Datensätze zunächst entschlüsseln muss, damit ein Entitäten-Gerät diese weiterverarbeiten kann. Neben dem Ausweisdaten-Chiffrat 408 kann das Venue-Gerät beispielsweise auch den Zeitstempel 404, den Nutzerverifikationsdatenwert 414 und weitere Daten mit dem privaten Venue-Schlüssel 512 verschlüsseln, um die verschlüsselten Check-in Daten 506 zu erhalten, die auf den Server 202 hochgeladen werden.

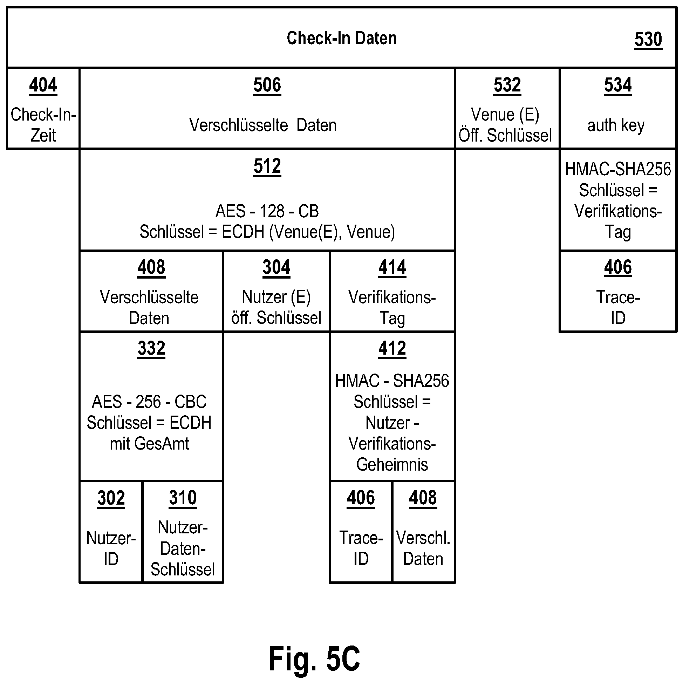

Die **Figuren 5B** **und** **5C** zeigen weitere alternative Varianten für Check-in Datensätze, wie sie auf dem Server gespeichert sind (erste beiden Zeilen) sowie darunterstehend die Datenwerte und Schlüssel, die zur Bildung der auf dem Server gespeicherten Werte verwendet werden.

Der Veranstaltungsort, d.h. das Venue-Gerät, prüft gemäß mancher Ausführungsformen zunächst nach Empfang von Check-in Daten, ob der Zeitstempel 404 neueren Datums ist und führt keine weitere Validierung durch.

Gemäß weiteren Beispielen können die verschlüsselten Check-in Daten 506 dadurch gebildet werden, dass die App 218 für jedes Einchecken ein Signal zur Übertragung der Trace-ID erzeugt, welches per direkter Kommunikation übertagen wird, beispielsweise per Anzeige eines QR-Codes, wobei auch ein ephemeres ECC-Schlüsselpaars secp256r1 und eines Verschlüsselungsschlüssels vorzugsweise für jeden neuen QR Code gebildet werden.

Die Trace-ID 406 kann z.B. ein HMAC-SHA256 sein, der aus der User-ID 302 und dem aktuellen Zeitstempel 404 (abgerundet auf die aktuelle Minute) als (Input)Daten und dem Benutzer-Trace-Geheimnis als Schlüssel abgeleitet und auf die ersten 16 Bytes gekürzt wird.

Der Verschlüsselungsschlüssel wird mit ECDH aus dem ephemeren privaten Schlüssel und dem öffentlichen Schlüssel des Gesundheitsamts berechnet. Die App wird einen QR-Code mit folgendem Inhalt anzeigen (der jede Minute mit dem aktuellen Zeitstempel aktualisiert wird):
- QR-Code-Version (1 Byte) 402,
- Zeitstempel (4 Bytes) 404,
- Trace-ID (16 Bytes) 406,
- verschlüsselte Daten (32 Bytes) 408, (Datenschlüssel und Benutzerkennung mit AES-128-CBC verschlüsselt und der Verschlüsselungsschlüssel als Schlüssel und die Trace-ID als IV) ephemerer öffentlicher Schlüssel (33 Bytes)
- Verifizierungs-Tag (8 Bytes) 414, (HMAC-SHA256 mit Trace-ID und verschlüsselten Daten als Daten und dem Verifizierungsgeheimnis des Benutzers als Schlüssel, abgeschnitten auf die ersten 8 Bytes).

Die Gesamtnutzlast beträgt 94 Bytes, die einfach verkettet und dann mit ASCII85 als QR-Code-Inhalt kodiert werden. Das ephemere Schlüsselpaar kann verworfen werden.

Der Veranstaltungsort verschlüsselt die vom Nutzergerät empfangenen verschlüsselten Daten, den ephemeren öffentlichen Schlüssel und den Verifizierungs-Tag z.B. mit AES-128-CBC mit dem ECDH eines ephemeren Schlüsselpaares und dem öffentlichen Schlüssel des Veranstaltungsortes. Der Veranstaltungsort erstellt einen Authentifizierungsschlüssel mit HMAC-SHA256 mit der Trace-ID 406 als Daten und dem Verifizierungsdatenwert ("Verifizierungstag") 414 als Schlüssel. Der Veranstaltungsort wird die Check-in-Zeit, die verschlüsselten Daten und den Authentifizierungsschlüssel auf den Server hochladen.

Wenn der Benutzer über die App (geofence oder manuell) auscheckt, sendet die App 218 gemäß Ausführungsformen der Erfindung einen Checkout-Zeitstempel 504 und einen HMAC-SHA256 mit dem Checkout-Zeitstempel als Daten und dem Authentifizierungsschlüssel (HMAC(data=trace id, key=verification tag) als Schlüssel).

Die Erzeugung der in den Figuren 4 und 5 gezeigten Datenstrukturen kann z.B. so erfolgen, dass die App 218 auf dem Nutzergerät eine Trace-ID für jeden neuen QR-Code generiert. Die Trace-ID ist ein HMAC-SHA256, abgeleitet von der User-ID und dem aktuellen Zeitstempel (abgerundet auf die aktuelle Minute) als Daten und dem Benutzer-Tracing-Geheimnis als Schlüssel, abgeschnitten auf die ersten 16 Bytes). Die Userld und der UserDataKey werden mit dem oben für den aktuellen DailyPublicKey beschriebenen asymmetrischen Verschlüsselungsschema verschlüsselt, wobei die IV in den Datensätzen 420 und 520 der Figuren 4B und 5B als die ersten 16 Bytes des ephemerenPublicKey definiert ist.

Die App 218 erzeugt gemäß eines weiteren Beispiels einen QR-Code mit folgendem Inhalt (der jede Minute mit dem aktuellen Zeitstempel aktualisiert wird) an (siehe Figur 4B und C): App Badge Data, QR-Code-Version (1 Byte), Gerätetyp (1 Byte), Schlüssel-ID (2 Byte) (gibt an, welcher DailyPublicKey verwendet wurde), Zeitstempel (4 Byte), Trace-ID (16 Bytes), verschlüsselte Daten (32 Bytes) (userDataKey und Benutzerkennung verschlüsselt nach dem oben beschriebenen asymmetrischen Schema für den öffentlichen Entitätenschlüssel mit den ersten 16 Bytes des ephemeren publicKey als IV), ephemerer öffentlicher Schlüssel (33 Bytes), sowie Verifizierungs-Tag (8 Bytes) (HMAC-SHA256 mit Zeitstempel und verschlüsselten Daten als Daten und dem Verifizierungsgeheimnis des Benutzers als Schlüssel, abgeschnitten auf die ersten 8 Bytes). Die Gesamtnutzlast beträgt 95 Bytes, die einfach verkettet und dann mit ASCII85 als QR-Code-Inhalt kodiert werden.

Das Venue-Gerät am Veranstaltungsort wird die empfangenen verschlüsselten Daten zunächst aus dem empfangenen QR-Code extrahieren. Danach prüft das Venue-Gerät vorzugsweise noch die Aktualität des im Code enthaltenen Check-in Zeitstempels 404 und verwirft den Code, falls der Zeitstempel älter als ein maximales Alter, z.B. 30 Sekunden, ist.

Dann kann das Venue-Gerät folgenden Datensatz z.B. mittels Verschlüsselung konkatenierter Werte (|| als Konkatenationsoperator) erstellen: AsymEnc_priv_Venue-Key(keyld || ePubKey || verificationTag || encData || venuePublicKey). Hierdurch wird der Wert encCheckInData erzeugt. Danach lädt das Nutzergerät die folgenden Daten zum Server hoch: Trace-ID, Venue-Gerät-ID, Zeitstempel, encCheckInData.

Der Server speichert z.B. zumindest die Trace-ID, die Venue-ID ("locationID" bzw. "scannerID"), Check-in Zeit, die verschlüsselten Daten encCheckINData, den mac Wert, iv und den ephPublicKey.

In einer alternativen Ausführungsform ohne Venue-Gerät stellt der Venue-Betreiber nur ein Träger-Objekt bereit, z.B. einen Tischaufsteller mit QR-Code, die die Venue-ID des Venues kodiert. Der Benutzer scannt mit seinem Smartphone den QR-Code, der eine URL enthält. Die URL enthält eine Venue-ID, wobei das Einlesen der URL die App 218 dazu veranlasst, die Informationen des Venues vom Server 202 abzurufen, sowie optionale Venue-Zusatzdaten. Die App 218 kann diese URL als universal URL (deeplink) registrieren und die Venue-ID und den venuePublicKey über diesen Deeplink vom Server beziehen. Gemäß einer anderen Ausführungsform sind diese Daten bereits im QR-Code kodiert. Die App 218 generiert dann alle Informationen des QR-Codes wie oben beschrieben und verarbeitet diese wie das oben beschriebene Venue-Gerät (QR-Code-Daten mit venuePublicKey 512 neu verschlüsseln und die Trace-ID, Venue-ID, Eincheckzeit, verschlüsselte Daten, mac, iv und den ephemeren öffentlichen Schlüssel auf den Server hochladen).

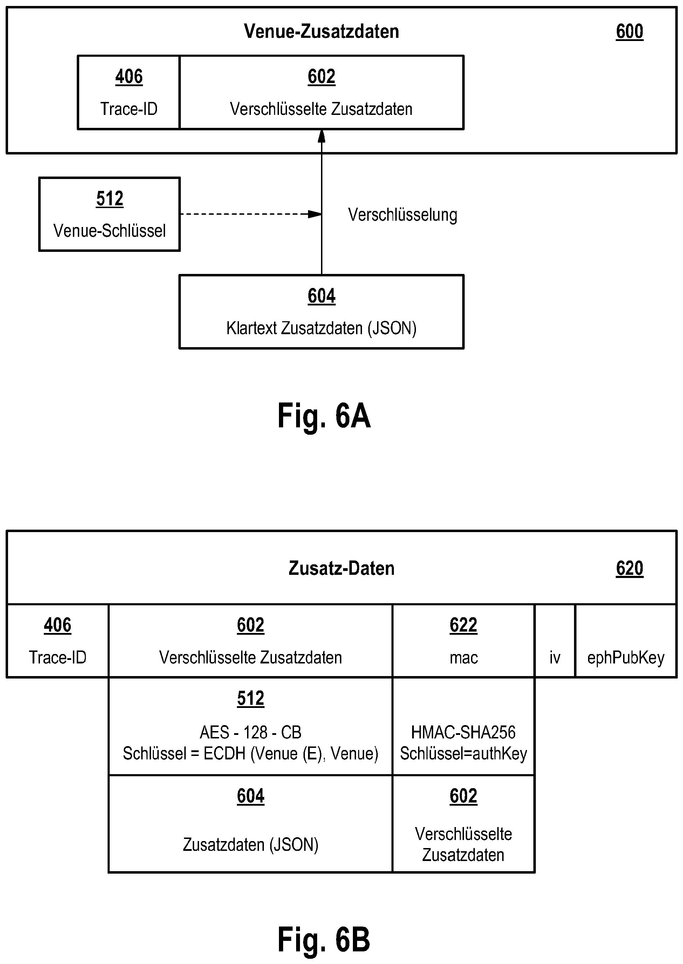

**Figuren 6A, 6B** zeigen Beispiele für Venue-Zusatzdaten 600, 620. Je nach Ausführungsform können die Venue-Zusatzdaten nur lokal auf den Venue-Geräten und/oder auf dem Server verknüpft mit den Venue-IDs gespeichert sein. Bei den Zusatzdaten handelt es sich um Daten, die sich auf den Nutzer und/oder dessen Umfeld beim Vorgang des Check-ins beziehen.

Beispielsweise können die Zusatzdaten weitere Angaben beinhalten, die der Betreiber des Venue benötigt, um dem Nutzer während seines Aufenthalts im Venue automatisch bestimmte Dienste bereitzustellen und/oder den Besuch in einer Venuebezogenen Verwaltungssoftware zu dokumentieren und/oder zu organisieren. Beispielsweise können die Zusatzdaten eine Tischnummer beinhalten und den Tisch bezeichnen, an welchem sich ein Gast niederlässt. Die Zusatzdaten können sich auf eine Serviceleistung oder ein Fahrgeschäft beziehen, das der Besucher über eine App seines Nutzergerätes gewählt hat und/oder auf Daten, die zum Beispiel zur Anbahnung eines automatischen Zahlprozesses dienen. Beispielsweise kann während oder nach einem erfolgreichen Check-in das Venue-Gerät eine Anfrage an das Nutzergerät senden, und dadurch das Nutzergerät veranlassen, dem Nutzer über eine grafische Benutzeroberfläche zur Eingabe weiterer Zusatzdaten aufzufordern. Der Nutzer gibt also die für den Venue relevanten Daten (Tischnummer, gebuchtes Fahrgeschäft, Zusatz- oder Serviceleistungen, Alter des Nutzers etc.) ein, das Nutzergerät erzeugt daraufhin ein Datenobjekt (zum Beispiel im JSON Format), dass die vom Nutzer eingegebenen Daten beinhaltet, und sendet dieses an das Venue-Gerät zurück. Beispielsweise können die Anwendungen 218 auf dem Nutzergerät und die Anwendung 230 auf dem Venue Gerät als interoperabel Anwendungen eines verteilten Software-Systems zur datenschutzkonformen und sicheren Nachverfolgung von Besuchern implementiert sein.

Vorzugsweise werden die Venue-Zusatzdaten 604 in verschlüsselter Form 602 lokal und/oder auf dem Server verknüpft mit einer Trace-ID 406 des Check-im Vorgangs, in dessen Kontext die Zusatzdaten erfasst wurden, gespeichert. Beispielsweise kann ein Venue-spezifischer Verschlüsselungsschlüssel 512 zur Verschlüsselung verwendet werden, sodass der Server nicht oder nur nach Zustimmung des Venue-Betreibers auf die Daten zugreifen kann.

Die Speicherung der Venue-Zusatzdaten kann vorteilhaft sein, da sich auf Basis zum Beispiel der Tischnummer oder des gewählten Fahrgeschäfts eines Vergnügungsparks der Aufenthaltsort des Besuchers noch genauer rekonstruieren lässt.

Zusätzlich oder alternativ dazu kann das Nutzergerät im Zuge eines Check-in Vorgangs auch Nutzer-Zusatzdaten erzeugen, welche mit dem UserDataKey (also dem nutzerspezifischen (öffentlichen asymmetrischen oder symmetrischen) Verschlüsselungsschlüssel dieses Nutzers und/oder mit dem Entitätenschlüssel verschlüsselt sind. Bei diesen Zusatzdaten handelt es sich um Daten, die ebenfalls im Kontext des Check-in Vorgangs stehen und welche das nachträgliche Tracing dieses Nutzers und/oder dessen Begleit- oder Kontaktpersonen erleichtern kann. Beispielsweise kann die App 218 dazu ausgebildet sein, den Nutzer beim Check-in (oder davor) aufzufordern, zu spezifizieren, mit welcher Begleitperson der Venue betreten wird und/oder welche sonstigen sich im Venue befindlichen Personen besucht werden sollen. Falls also ein Besucher eines Krankenhauses in Begleitung von Kindern erscheint und/oder einen bestimmten Patienten besucht, können diese Angaben zu den Kindern oder dem Patienten in Form der Nutzer-Zusatzdaten beim Check-in berücksichtigt und als Teil des Check-in Datensatzes auf dem Server gespeichert werden. Somit können auch Personen, die als Begleitpersonen, Besuchte oder in anderer Funktion Kontakt mit der zu tracenden Person hatten, leichter identifiziert werden auch dann, wenn diese Personen sich nicht gegenüber dem Server registriert haben.

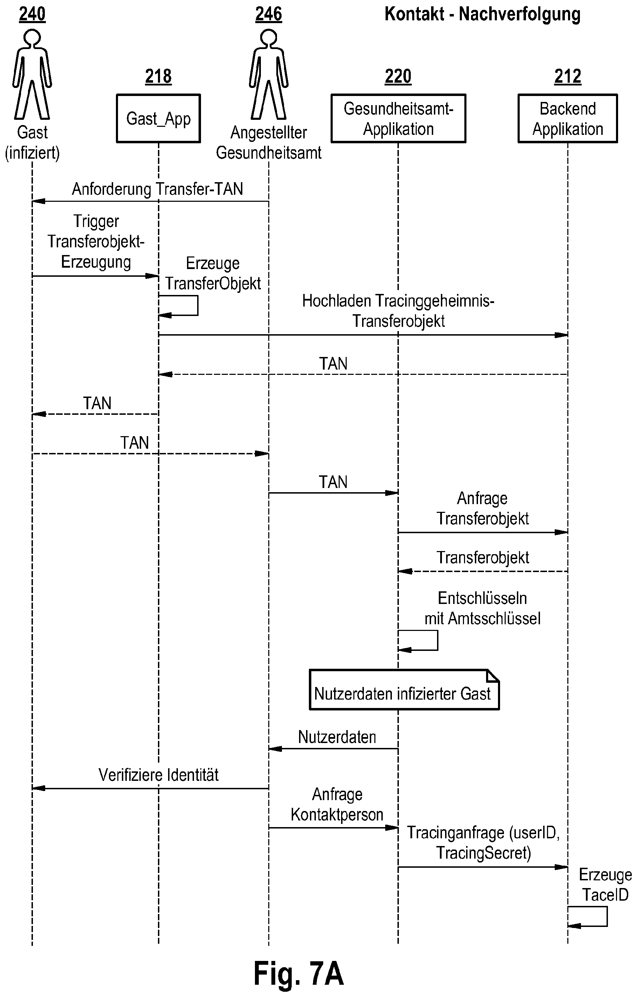

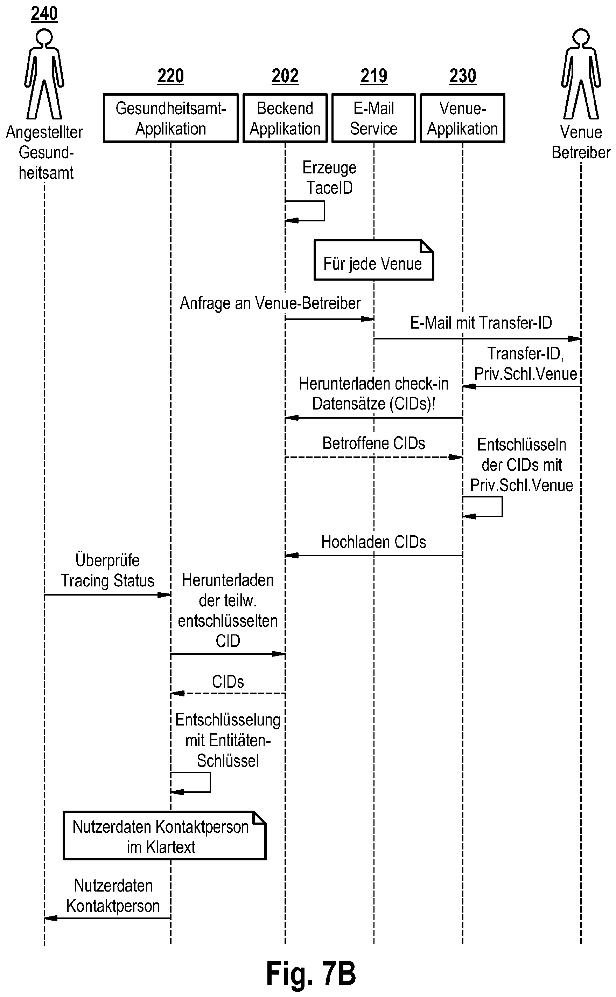

**Figur 7** zeigt ein UML-Diagramm eines Beispiels für das Tracing-Verfahren. Die von den gestrichelten Linien repräsentierten Personen bzw. Systemkomponenten entsprechen den in Figur 2 gezeigten Komponenten. Das Verfahren zum Tracing eines Nutzers integriert gemäß Ausführungsformen den gesamten Prozess vom ersten Kontakt eines infizierten Gastes mit dem Gesundheitsamt an bis zur Offenlegung der Kontaktdaten möglicher Kontaktpersonen gegenüber dem Amt. Um die Kontaktpersonen ausfindig zu machen, benötigt das Gesundheitsamt die TAN einer infizierten Person.

Die hier im Hinblick auf ein Gesundheitsamt und einen Pandemiefall beschriebenen Beispiele gelten analog auch für andere Typen von Entitäten und Notfälle.

Figur 7 illustriert den Fall, dass ein Nutzer (Gast), der in der Vergangenheit verschiedene Venues besucht hat, ein Testergebnis erhalten hat, wonach der Nutzer mit einer ansteckenden Krankheit (z.B. Covid 19) infiziert ist oder zumindest war. Dieses Testergebnis teilt der Nutzer, der Arzt oder das Labor der Gesundheitsbehörde mit. Bei einer Ansteckung werden die infizierten Gäste von der örtlichen Gesundheitsbehörde kontaktiert. Bei der Kontaktaufnahme wird der infizierte Gast gefragt, ob er das Tracing-System gemäß einer hier beschriebenen Ausführungsform der Erfindung benutzt, und er wird gebeten, seine persönlichen Daten und seine Kontakthistorie zur automatischen Kontaktverfolgung offenzulegen.

Der besagte infizierte Gast startet den Ablauf des Tracings, indem er ein Datenübertragungsobjekt (Tracinggeheimnis-Transferobjekt-) hochlädt, das verschiedene Geheimnisse enthält. Die App 218 erstellt das Tracinggeheimnis-Transferobjekt für den Austausch z.B. wie folgt:
- Serialisieren von userID, userDataKey und userTracingSecret in einem JSON Objekt;
- asymmetrische Verschlüsselung des serialisierten JSON Objekts mit dem aktuellen für diesen Tag gültigen Entitätenschlüssel dailyPublicKey durch das Nutzergerät
- die resultierenden verschlüsselten Daten, und optional auch iv, mac und ephPublicKey zum Server 202 hochladen und als Tracinggeheimnis-Transferobjekt dieses Nutzers auf dem Server speichern.
- Der Server wird eine TAN erzeugen, verknüpft mit dem besagten Tracinggeheimnis-Transferobjekt auf dem Server speichern und die TAN an das Nutzergerät zurückgeben. Beispielsweise kann der Server die TAN an das Nutzergerät des Nutzers zurückgeben, wo es auf dem Display des Geräts angezeigt wird, und der Nutzer kann die Tan telefonisch dem Gesundheitsamt mitteilen. Erst mit der TAN kann die Gesundheitsbehörde die Daten auf dem Server zur Kontaktverfolgung nutzen.

Nach Kontaktaufnahme mit dem infizierten Gast gibt der Mitarbeiter des Gesundheitsamtes die TAN in seiner Frontend-Applikation des Gesundheitsamtes ein. Der Mitarbeiter ist angemeldet z.B. unter Verwendung seines während des Registrierungsprozesses erzeugten Zertifikats, das die Identität des Benutzers verifiziert.

**Bezug des Entitäten-Schlüssels:** Damit der Prozess beginnen kann, benötigt der Mitarbeiter der Gesundheitsabteilung Zugang zu allen relevanten DailyKeypairs des Gesundheitsamts. Daher holt er sich das verschlüsselte, täglich neu erstellte asymmetrische Schlüsselpaar des Gesundheitsamts (HealthDepartmentEncryptionKeyPair), das er mit seinem persönlichen privaten Encryption-Key entschlüsseln kann. Mit dem lokal entschlüsselten healthDepartmentEncryptionKeyPair kann er die EncryptedDailyKeypairs abrufen und lesen.

In Ausführungsformen in welchen nur ein statisches, über eine lange Zeit gültiges asymmetrisches Schlüsselpaar für die Entität erstellt wird, kann der Bezug der Entitäten-Schlüssel auch einfacher sein und ein Lesen des privaten Entitäten-schlüssels umfassen.

### Abrufen der persönlichen Daten des infizierten Gastes:

Nachdem der angemeldete Mitarbeiter z.B. telefonisch oder per Mail oder Briefpost die TAN von dem Nutzer erfahren hat, sendet der Mitarbeiter des Amts eine Tracing-Anfrage an den Server, wobei die Anfrage die TAN beinhaltet. Der Server identifiziert das mit der TAN verknüpft gespeicherte Tracinggeheimnis-Transferobjekt und stellt es dem Mitarbeiter der Behörde zum Download bereit. Das Transferobjekt ist vorzugsweise mit dem aktuellen öffentlichen Schlüssel des Gesundheitsamts verschlüsselt und wird vom Mitarbeiter des Amts mit dem korrespondierenden privaten Schlüssel entschlüsselt.

Die dem Gesundheitsamt nun vorliegenden entschlüsselten Geheimnisse des Tracinggeheimnis-Transferobjekts enthalten die Userld und das UserTracingSecret des zu tracenden Nutzers, sodass das Amt (also ein Entitäten-Gerät, das dem Amt zugewiesen ist) die Kandidaten-Tracing-IDs dieses Gasts für z.B. die letzten 14 Tage (20.160 Kandidaten Trace-IDs) berechnen kann.

### Identifikation besuchter Venues und möglicher Kontaktpersonen

Das Gesundheitsamt sendet eine Anfrage an den Server, die die berechneten Kandidaten-Tracing-IDs beinhaltet. Der Server durchsucht seine Check-in Datensätze in seiner Datenbank und ermittelt alle Venue-IDs aller Check-in Datensätze, deren Trace-IDs identisch sind zu einer der in der Anfrage übermittelten Kandidaten-Trace-IDs. Die Kontaktdaten dieser ermittelten Venues werden vom Server ans Amt zurückgegeben.

Das Gesundheitsamt kann dann die Veranstaltungsorte (Venues bzw. die Venue-Geräte) auffordern, Zugang zu allen Check-in Datensätzen zu gewähren, die sich zur gleichen Zeit wie die infizierten Benutzer am Veranstaltungsort befanden. Die Veranstaltungsorte werden z.B. per Mail kontaktiert, um diese über das laufende Tracingverfahren zu informieren und um Freigabe der auf dieses Venue bezogenen Check-in Datensätze zu bitten.

Der Venue wird im Falle seiner Zustimmung alle Check-in Datensätze (oder alle Check-in Datensätze bis zu einem maximalen Alter, falls die Check-in Time im Klartext in den Check-in Datensätzen enthalten sein sollte) herunterladen, die von diesem Venue stammen, diese entschlüsseln und z.B. anhand der Zeitstempel 404 überprüfen, welche Gäste Kontaktpersonen sein könnten. Dann lädt das Venue-Gerät die Check-in Daten der möglichen Kontaktpersonen (deren sensible Daten wie die Ausweisdaten immer noch mit dem DailyPublicKey der Entität verschlüsselt sind) zum Server hoch, der diese Daten an das Gesundheitsamt weiterleitet. In einer alternativen Ausführungsform kann das Venue-Gerät die mit dem privaten Venue-Schlüssel entschlüsselten Daten auch direkt an das Entität-Gerät senden.

Dieser Umweg über den Venue ist nur in Ausführungsformen erforderlich, in welchen die Check-in Datensätze zusätzlich noch mit dem öffentlichen Venue-Schlüssel verschlüsselt sind und der Venue die Check-in Datensätze mit dem korrespondierenden Entschlüsselungsschlüssel wieder entschlüsseln muss damit die Entität sie verarbeiten kann. Falls die Check-in Daten nicht noch zusätzlich mit dem Venue-Schlüssel verschlüsselt sind, ist es auch möglich, dass die mit dem Entitätenschlüssel verschlüsselten Check-in Datensätze der möglichen Kontaktpersonen direkt vom Server an das Entitäten-Gerät übermittelt werden.

Nachdem das Entitäten-Gerät alle Check-in Datensätze der möglichen Kontaktpersonen im fraglichen Zeitraum erhalten hat und diese nicht (mehr) mit dem Venue-Schlüssel verschlüsselt sind, kann das Entitäten-Gerät diese Check-in Datensätze mit den entsprechenden DailyPrivateKeys der Entität entschlüsseln.

Nachdem der Entitäten-Rechner sowohl User-IDs als auch die UserDataKeys (also die zur Entschlüsselung der Nutzerdaten nötigen Entschlüsselungsschlüssel) möglicher Kontaktpersonen durch die Entschlüsselung erhalten hat, kann das Entitäten-Gerät vom Server die verschlüsselten Nutzerdatensätze dieser möglichen Kontaktpersonen anfordern und mit den jeweiligen nutzerspezifischen UserDataKeys entschlüsseln und Kontakt zu diesen Nutzern aufnehmen. Das Amt kann aber kein Tracing für diese Kontaktpersonen vornehmen, solange diese nicht deren Tracinggeheimnis-Transferobjekt dem Amt zur Verfügung stellen.

Die Gesundheitsbehörde kann nun zudem auch die Integrität des Check-ins mit dem Verifizierungsgeheimnis verifizieren.

Es ist für Ausführungsformen des elektronischen Systems somit unmöglich, die Benutzer ohne deren Zustimmung zu verfolgen, da die Check-in Datensätze verschlüsselt sind und die als Suchschlüssel verwendete Trace-IDs dieser Check-in Datensätze ohne Kenntnis weiterer Daten, z.B. des Tracing-Secrets, welches nutzerindividuell erzeugt wird und geschützt in den jeweiligen Nutzergeräten gespeichert ist, nicht korreliert werden können. Das TracingSecret wird aber nur im Infektionsfall und nur mit Zustimmung des Nutzers ans Amt übertragen. Benutzer, die nur Kontakt mit einem infizierten Benutzer hatten, werden ihr TracingSecret nicht preisgeben und daher nicht zurückverfolgt werden können.

Die persönlichen Daten des Benutzers sind zu jeder Zeit verschlüsselt und können nur von Personen gelesen werden, die Kenntnis vom UserDataKey haben. Der userDataKey kann nur vom Benutzer selbst oder in verschlüsselter Form von einem Venue-Gerät bezogen werden, das einen Check-in QR-Code des Nutzers gescannt hat. Alle Entschlüsselungsvorgänge werden vorzugsweise lokal durch die jeweiligen Systemkomponenten durchgeführt. Nach Ausführungsformen sind alle Daten, die auf dem Server und/oder den Systemkomponenten (Nutzergerät, Venue-Gerät, Entitäts-Gerät und/oder Server) gespeichert sind, in irgendeiner Weise vom Eigentümer der Daten signiert/gemac'ed (wurden also als Input einer MAC Funktion zur Berechnung eines mac Werts), so dass kein Mitarbeiter die Daten ändern kann.

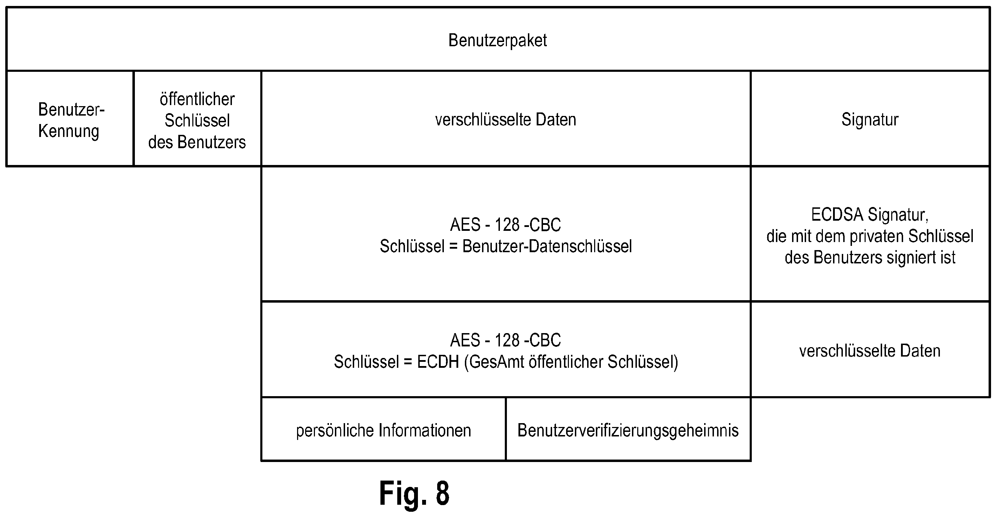

### Offenbarungsteil "System 2", Figuren 8-12

Der obige Offenbarungsteil betrifft ein Verfahren und System, das hier als "System 1" bezeichnet werden soll.

In einem weiteren Aspekt umfasst diese Offenbarung ein weiteres System ("System 2") zum Tracing von Nutzern, insbesondere zur Bekämpfung einer Pandemie, wie zum Beispiel Covid-19, sowie ein entsprechendes digitales Speichermedium, auf dem ein Computerprogramm bzw. eine App gespeichert ist. Das "System 2" ist z.B. in den Figuren 8 bis 12 illustriert. Ausführungsformen, Beispiele und Erläuterungen des Offenbarungsteils bezüglich des "System 1" (illustriert z.B. anhand der Figuren 1-7) und des Offenbarungsteils bezüglich des "Systems 2" (illustriert z.B. anhand der Figuren 8-12) können frei miteinander kombiniert werden, sofern sie sich nicht gegenseitig ausschließen. Bezüglich der Bedeutung der Begriffe "Mobilfunkgerät", "Server", "Venue" und "Entität" wird auf die obenstehenden Definitionen verwiesen.

Zur Begrenzung der Verbreitung von Covid-19 wird bislang so vorgegangen, dass bei Besuch einer öffentlichen Veranstaltung oder einer Gaststätte die betreffende Person ihre persönlichen Daten angeben muss. Die damit einhergehenden Dokumentationspflichten sind für die entsprechenden Veranstalter bzw. Betreiber von Restaurants aufwendig, zumal auch die datenschutzrechtlichen Bestimmungen eingehalten werden müssen.

Demgegenüber wird gemäß des hier beschriebenen elektronischen Systems 2 ein System bereitgestellt, welches ein weitgehend automatisiertes Tracing unter Wahrung von datenschutzrechtlichen Vorschriften gewährleistet.

Beim Einsatz des Systems wird so vorgegangen, dass sich die Nutzer zunächst mithilfe der jeweiligen Mobilfunkgeräte registrieren. Hierzu werden zunächst Nutzerdaten erfasst, also beispielsweise durch Eingabe der Nutzerdaten in das betreffende Mobilfunkgerät oder durch Einlesen der Nutzerdaten, beispielsweise aus einem elektronischen Personalausweis des Nutzers in das Mobilfunkgerät. Die Nutzerdaten werden dann mithilfe des öffentlichen Entitätenschlüssels, also beispielsweise mithilfe eines öffentlichen Schlüssels des Gesundheitsamts, von dem Mobilfunkgerät verschlüsselt und das Resultat dieser Verschlüsselung wird mit einem User-Data-Key des betreffenden Nutzers verschlüsselt, wobei es sich bei dem User-Data-Key hier beispielsweise um einen Zufallswert hoher Entropie handelt, wie zum Beispiel einer Länge von 16 Bytes. Das Mobilfunkgerät kann z.B. als Nutzergerät verwendet werden.

Das Mobilfunkgerät ist einem Nutzer eindeutig zugeordnet. Das Tracing soll für eine "Entität" stattfinden.

Bei dem Entitätenschlüssel kann es sich um einen Master key handeln oder den Schlüssel einer der Entität zugeordneten abhängigen Entität. Beispielsweise kann es sich im Fall des Gesundheitsamts um den Masterschlüssel des Gesundheitsamts oder um einen Schlüssel des jeweils vor Ort verantwortlichen Gesundheitsamts handeln.

Der User-Data-Key kann beispielsweise für die Zwecke der Registrierung durch das Betriebssystem des Mobilfunkgeräts zur Verfügung gestellt werden. Beispielsweise verfügt das Mobilfunkgerät über eine Binary Symmetric Source zur Erzeugung von Zufallswerten hoher Entropie.

Die Hinterlegung der Nutzerdaten auf dem Server erfolgt also in einer Form, die es dem Server zunächst nicht ermöglicht, diese Nutzerdaten zu entschlüsseln, da die Verschlüsselung zweistufig ist und insbesondere ohne den privaten Entitätenschlüssel nicht möglich ist.

In dem Mobilfunkgerät wird dieser User-Data-Key in einem nichtflüchtigen Speicher persistent gespeichert. Ferner wird auch ein User-Secret für den Nutzer durch dessen Mobilfunkgerät generiert, wobei es sich auch wiederum um einen Zufallswert hoher Entropie von zum Beispiel 16 Bytes Länge handeln kann. Aufgrund der Registrierung auf dem Server erzeugt der Server eine User-ID, das heißt einen eindeutigen Identifikator für den betreffenden User, die der Server aufgrund der Registrierung an das Mobilfunkgerät zurückgibt. Der User-Data-Key, das User-Secret und die User-ID werden in dem Mobilfunkgerät in einem nichtflüchtigen Speicher persistent gespeichert.

Um einen Nutzer beim Besuch einer venue zu erfassen, wird nun wie folgt vorgegangen:
Das Mobilfunkgerät erzeugt eine Trace-ID, die repräsentativ ist für eine Kombination aus der User-ID, dem User-Secret und einer aktuellen Zeitinformation. Bei der aktuellen Zeitinformation handelt es sich um die aktuelle Uhrzeit beim Check-in des Nutzers an dem Venue, wobei diese aktuelle Uhrzeit von dem Mobilfunkgerät über das Netzwerk ermittelt werden. Vorzugsweise wird diese aktuelle Uhrzeit nach einem vordefinierten Rundungsverfahren gerundet, beispielsweise auf die volle Minute auf- oder abgerundet.

Außerdem erzeugt das Mobilfunkgerät ein Chiffrat aus dem User-Data-Key und der User-ID, wobei der öffentliche Entitätenschlüssel zur Erzeugung des Chiffrats verwendet wird. Die Trace-ID sowie das Chiffrat werden dann von dem Mobilfunkgerät per direkter Kommunikation, also nicht über ein Netzwerk, an das Venue-Gerät übertragen.

Das Venue-Gerät prüft zur Erfassung eines Nutzers, ob die Zeitinformation der Trace-ID hinreichend aktuell ist und verwendet hierzu beispielsweise ebenfalls eine über ein Netzwerk bezogene Zeitinformation zur Synchronisierung mit der von dem Mobilfunkgerät verwendeten Zeitbasis. Die Zeitinformation gilt als aktuell, wenn sie beispielsweise innerhalb eines vorgegebenen Toleranzbereichs liegt, also beispielsweise nicht älter ist als einige Sekunden oder Minuten.

Wenn die Zeitinformation der Trace-ID hinreichend aktuell ist, so überträgt das Venue-Gerät die Trace-ID mit einer dem Venue-Gerät zugeordneten Venue-ID und dem Chiffrat an den Server. Diese Informationen werden auf dem Server gespeichert.

Vorzugsweise wird dies für eine möglichst große Anzahl von Nutzern, vorzugsweise für sämtliche Nutzer, durchgeführt, die eine venue besuchen, um so möglichst lückenlos sämtliche Nutzer zu erfassen.

Nachträglich kann das Erfordernis bestehen, den oder die Nutzer, welche den venue besucht haben, gegenüber der Entität offenzulegen. Dies ist beispielsweise dann der Fall, wenn einer der Nutzer positiv auf Covid-19 getestet wird. Dieser mit Covid-19 infizierte Nutzer meldet dies der Entität, hier dem Gesundheitsamt, und teilt der Entität seine User-ID und sein User-Secret mit.

Anhand dieser Informationen werden dann sämtliche mögliche zurückliegende Trace-ID, die zur Erfassung eines Nutzers durch ein Venue-Gerät für einen vorgegebenen vergangenen Zeitraum benutzt sein könnten, erzeugt. Die Menge dieser möglichen Trace-IDs ist relativ begrenzt, da nicht sämtliche mögliche Zeiten innerhalb des relevanten Zeitraums in Frage kommen, sondern nur diejenigen, die aufgrund des vorgegebenen Rundungsverfahrens tatsächlich vorkommen können.

Diese Trace-IDs werden dann als Suchkriterium verwendet, um entsprechend auf dem Server für den vergangenen Zeitraum gespeicherte Trace-IDs aufzufinden, sodass die Venue-IDs derjenigen Venues, von denen der Nutzer während des vergangenen Zeitraums erfasst wurde, ermittelt werden. Hierdurch erhält also das Gesundheitsamt die Venue-IDs der ermittelten Venus, die der Nutzer in dem vergangenen Zeitraum, beispielsweise zwei Wochen, in denen er möglicherweise bereits infektiös war, besucht hatte.

Nach einer Ausführungsform des "Systems 2" ist das elektronische System so konfiguriert, dass von dem Mobilfunkgerät eines durch ein Venue-Gerät erfassten Nutzers, also nach einem Check-in, eine Check-out Zeit an den Server übertragen wird, wenn der Nutzer den venue verlässt. Diese Check-out-Zeit ist der zuvor für diesen Nutzer empfangenen Trace-ID zugeordnet, sodass serverseitig die Information vorliegt, im welchen Zeitraum sich der Nutzer an dem venue aufgehalten hat.

Vorzugsweise geht diese Information in die Ermittlung derjenigen Nutzer ein, die sich ebenfalls an dem venue aufgehalten hatten, indem die Suche auf solche Nutzer beschränkt wird, die nicht nur den gleichen venue besucht hatten, sondern dort auch in einem zumindest mit dem Aufenthaltszeitraum des erkannten Nutzers überlappenden Aufenthaltszeitraums gewesen sind. Nach einer Ausführungsform des "Systems 2" erfolgt dieser Check-out durch manuelle Eingabe in das Mobilfunkgerät oder automatisch durch sogenanntes Geofencing. In letzterem Fall wird die Check-out-Zeit mit der zugeordneten Trace-ID automatisch von dem Mobilfunkgerät an den Server gesendet, sobald der Nutzer die venue verlässt.

Als Venue-ID kann beispielsweise der Name und/oder Adresse, ein Identifikator oder auch die geografischen Koordinaten des Venues dienen.

In einem weiteren Aspekt betrifft die Offenbarung zu dem "System 2" ein digitales Speichermedium mit von einem Prozessor eines Mobilfunkgeräts ausführbaren Programminstruktionen.

Gemäß Ausführungsformen des Systems kann dieses für ein Verfahren zum Tracing von Nutzern für eine Entität mit den folgenden Schritten verwendet werden:
- Speicherung von Nutzerdaten der Nutzer anlässlich einer Registrierung auf einem Server, wobei die Nutzerdaten durch den öffentlichen Entitätenschlüssel verschlüsselt sind und das Resultat dieser Verschlüsselung mit einem jeweiligen User-Data-Key des betreffenden Nutzers verschlüsselt ist,
- Zurverfügungstellung von tragbaren Mobilfunkgeräten, die jeweils einem Nutzer zugeordnet sind, wobei in jedem der Mobilfunkgeräte ein User-Data-Key, ein User-Secret und eine User-ID gespeichert sind, wobei das Mobilfunkgerät über eine Netzwerkschnittstelle Zugriff auf eine aktuelle Zeitinformation hat und das Mobilfunkgerät konfiguriert ist zur Erzeugung einer Trace-ID, die repräsentativ ist für eine Kombination aus User-ID, User-Secret und der aktuellen Zeitinformation sowie zur Erzeugung eines Chiffrats aus dem User-Data-Key und der User-ID, wobei der öffentliche Entitätenschlüssel zur Erzeugung des Chiffrats verwendet wird, und wobei das Mobilfunkgerät eine Kommunikationsschnittstelle zur direkten Kommunikation mit einem Venue-Gerät aufweist, über welche die Trace-ID und das Chiffrat an das Venue-Gerät übertragen werden,
- Erfassung eines der Nutzer durch ein Venue-Gerät mit Hilfe des Mobilfunkgeräts des Nutzers, wobei das Venue-Gerät eine Schnittstelle für die direkte Kommunikation mit der entsprechenden Schnittstelle des Mobilfunkgeräts aufweist, und mit einer Netzwerkschnittstelle zur Kommunikation mit dem Server, wobei das Venue-Gerät dazu konfiguriert ist, zur Erfassung des Nutzers die Trace-ID und das Chiffrat von dem Mobilfunkgerät zu empfangen und zu prüfen, ob die Zeitinformation einer empfangenen Trace-ID hinreichend aktuell ist, und wenn dies der Fall ist, die Trace-ID mit einer dem Venue-Gerät zugeordneten Venue-ID und dem Chiffrat an den Server zu übertragen,
- Speicherung einer empfangenen Trace-ID und dem Chiffrat mit der Zeitinformation als Zugriffsschlüssel durch den Server,
- Mitteilung der User-ID und des User-Secrets von einem der Nutzer an die Entität,
- Erzeugung von möglichen zurückliegenden Trace-IDs für den betreffenden Nutzer, die innerhalb eines vorgegebenen vergangenen Zeitraums liegen und Verwendung der erzeugten Trace-IDs als Suchkriterium, um entsprechende auf dem Server für den vergangenen Zeitraum gespeicherte Trace-IDs aufzufinden, so dass die Venue-IDs derjenigen Venues von denen der Nutzer während des vergangenen Zeitraums erfasst wurde, ermittelt werden,
- Ausgabe der Venue-IDs der ermittelten Venues zur Identifizierung derjenigen Venues, die der Nutzer während des vergangenen Zeitraums besucht hat.

Nach Ausführungsformen des im Kontext von "System 2" beschriebenen Verfahrens wird von dem Mobilfunkgerät des einen der Nutzer eine Check-out Zeit an den Server übertragen, wenn der Nutzer den venue verlässt, wobei die Check-out Zeit der zuvor für diesen Nutzer empfangenen Trace-ID zugeordnet wird, so dass für den Nutzer der Aufenthaltszeitraum an dem venue gespeichert wird.

Nach Ausführungsformen dieses Verfahrens werden diejenigen Nutzer ermittelt, die ebenfalls einen oder mehrere der ermittelten Venues während des vergangenen Zeitraums und während einer Aufenthaltsdauer des Nutzers besucht haben, wozu diejenigen der trace-IDs, die von den ermittelten Venues während des vergangenen Zeitraums zu dem Server übertragen wurden, ermittelt werden.

Im Weiteren werden Ausführungsformen bezüglich des "Systems 2" mit Bezugnahme auf die Zeichnungen 8-12 näher erläutert, wobei diese Zeichnungen jeweils zeigen:
- Figur 8: ein User-Data-Package mit den Nutzerdaten, welche zweifach verschlüsselt sind,

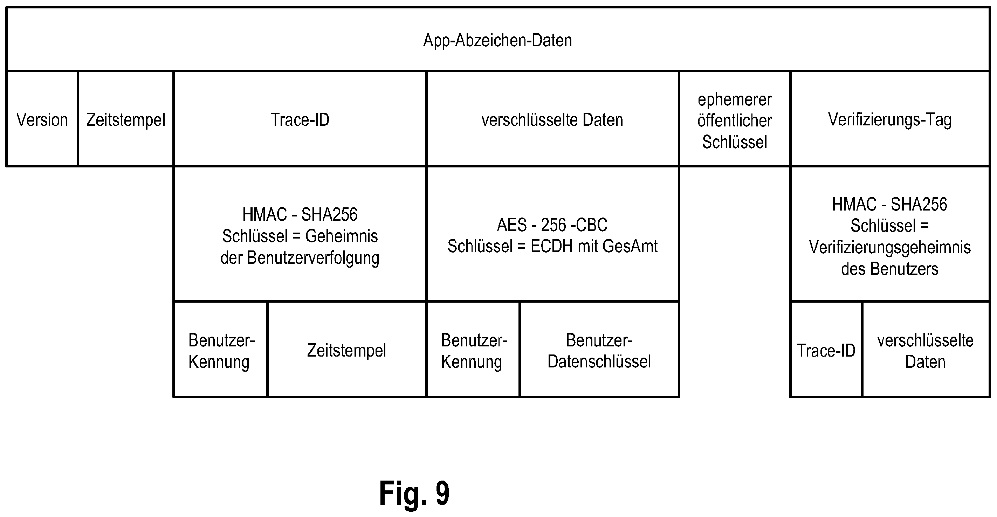

- Figur 9: eine Ausführungsform der Trace-ID,

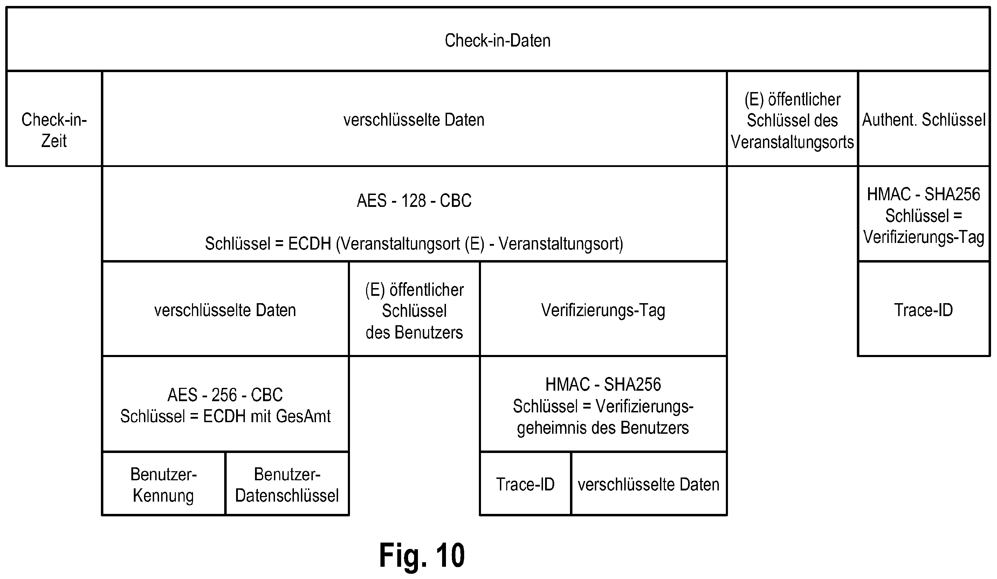

- Figur 10: eine Ausführungsform für den Check-in,

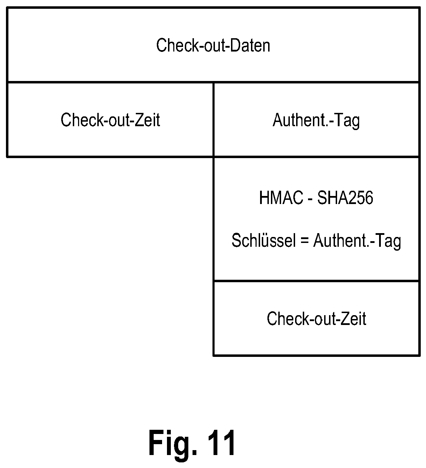

- Figur 11: eine Ausführungsform für den Check-out,

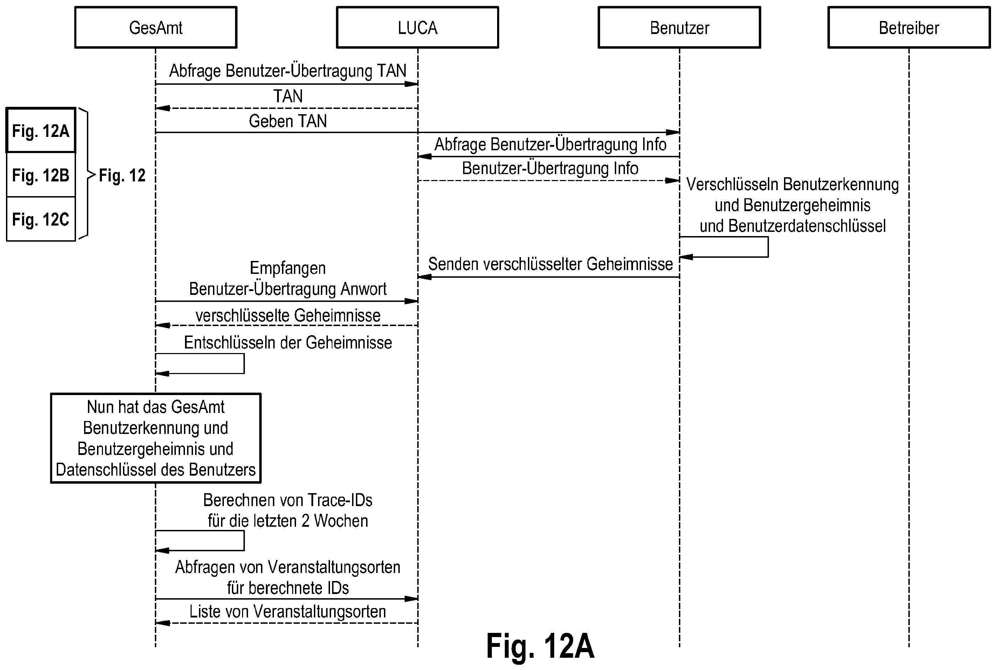

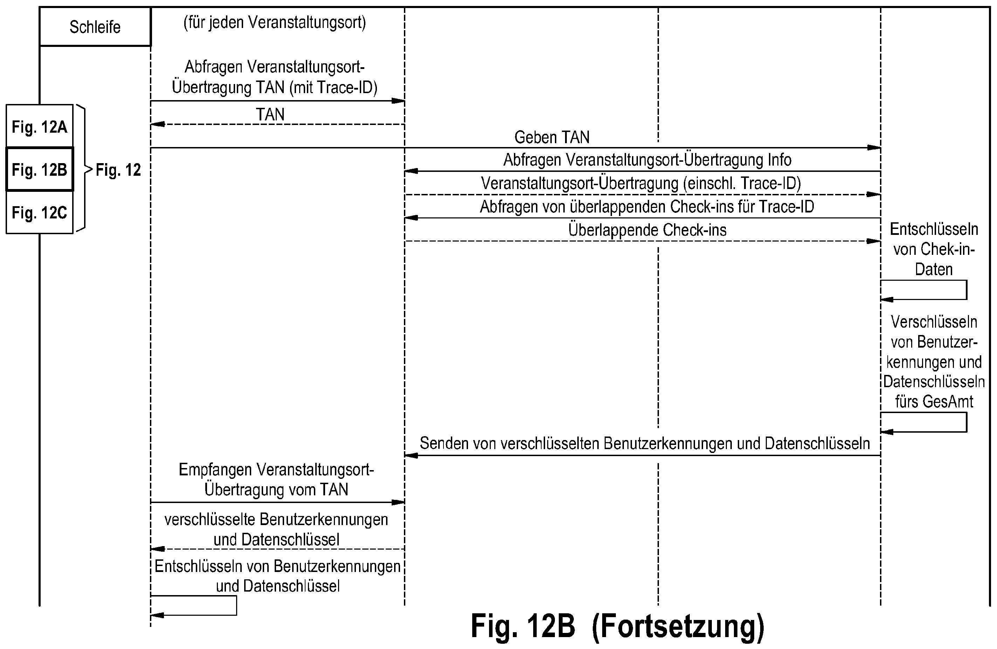

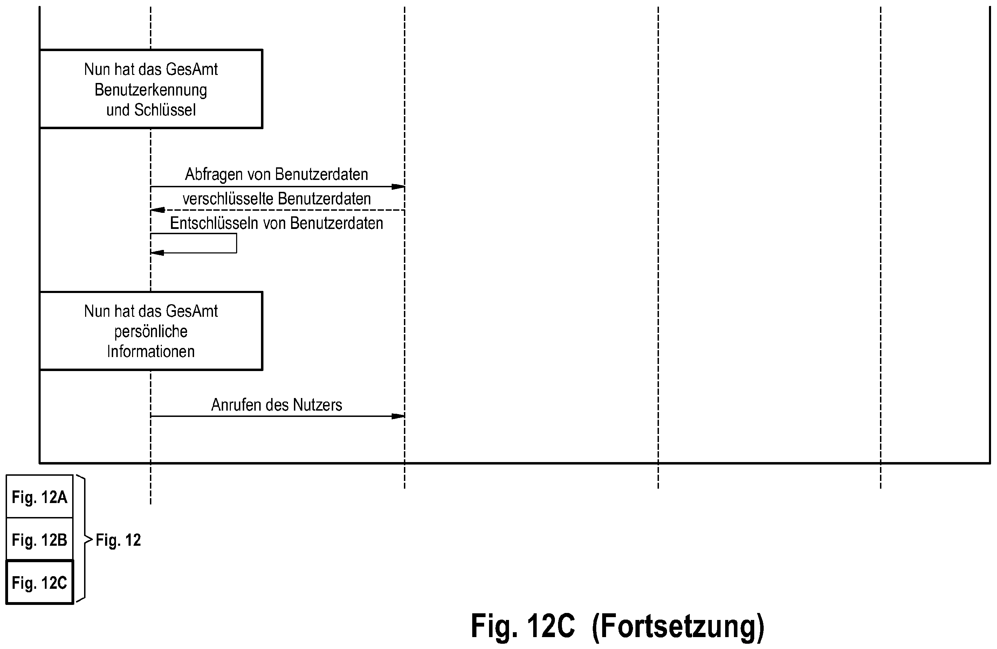

- Figur 12: ein UML-Diagramm.

Mit Bezug auf die Fig. 8 wird die Registrierung im Kontext des "Systems 2" beschrieben.

Der Benutzer stellt der App (Anwendungsprogramm) seines Mobilfunkgeräts seine Kontaktinformationen zur Verfügung. Die Anwendung erzeugt ein EC secp256r1-Schlüsselpaar (user public key und user private key), ein Benutzer-Trace-Geheimnis, d.h. ein user trace secret (16 Bytes Entropie), ein Benutzer-Verifizierungs-Geheimnis, d.h. user verification secret ein (16 Bytes Entropie) und einen Benutzer-Datenschlüssel, d.h. user data key (16 Bytes Entropie).

Dann berechnet die Anwendung einen Verschlüsselungsschlüssel mit ECDH mit dem privaten Schlüssel des Benutzers und dem öffentlichen Hauptschlüssel (Entitätenschlüssel) des Gesundheitsamtes. Die App verschlüsselt dann die persönlichen Informationen und das Benutzerverifizierungsgeheimnis mit diesem Verschlüsselungsschlüssel mit AES-128-CBC und verschlüsselt die Ausgabe mit AES-128-CBC mit dem Benutzerdatenschlüssel als Schlüssel. Die Anwendung lädt dann diese verschlüsselten Daten hoch und signiert sie mit dem privaten Schlüssel des Benutzers zusammen mit dem öffentlichen Schlüssel des Benutzers. Der Server gibt eine Benutzerkennung, d.h. User-ID, zurück.

Im Mobilfunkgerät werden gespeichert:
- user id (uuid)
- user public key (ec secp256r1)
- user private key (ec secp256r1)
- user data key (16 bytes random data)
- user trace secret (16 bytes random data) user verification secret (16 bytes random data)

### Einchecken (vgl. Fig. 9)

Die App generiert für jedes Einchecken ein Signal mit trace-ID und Chiffrat, welches per direkter Kommunikation übertagen wird, beispielsweise per Anzeige eines QR-Codes, zur Übertragung einer Trace-ID, eines ephemeres ECC-Schlüsselpaar secp256r1 und eines Verschlüsselungsschlüssels sowie des Chiffrats.

Die Trace-ID ist ein HMAC-SHA256, der aus der User-ID und dem aktuellen Zeitstempel (abgerundet auf die aktuelle Minute) als Daten und dem Benutzer-Trace-Geheimnis als Schlüssel abgeleitet und auf die ersten 16 Bytes gekürzt wird).

Der Verschlüsselungsschlüssel wird mit ECDH aus dem ephemeren privaten Schlüssel und dem öffentlichen Schlüssel des Gesundheitsministeriums berechnet. Die App wird einen QR-Code mit folgendem Inhalt anzeigen (der jede Minute mit dem aktuellen Zeitstempel aktualisiert wird):
- QR-Code-Version (1 Byte)
- Zeitstempel (4 Bytes)
- Trace-ID (16 Bytes)
- verschlüsselte Daten (32 Bytes) (Datenschlüssel und Benutzerkennung mit AES-128-CBC verschlüsselt und der Verschlüsselungsschlüssel als Schlüssel und die Trace-ID als IV) ephemerer öffentlicher Schlüssel (33 Bytes)
- Verifizierungs-Tag (8 Bytes) (HMAC-SHA256 mit traceid und verschlüsselten Daten als Daten und dem Verifizierungsgeheimnis des Benutzers als Schlüssel, abgeschnitten auf die ersten 8 Bytes)

Die Gesamtnutzlast beträgt 94 Bytes, die einfach verkettet und dann mit ASCII85 als QR-Code-Inhalt kodiert werden. Das ephemere Schlüsselpaar kann verworfen werden.

Der Veranstaltungsort, d.h. das Venue-Gerät, prüft, ob der Zeitstempel neueren Datums ist und führt keine weitere Validierung durch.

Der Veranstaltungsort verschlüsselt die empfangenen verschlüsselten Daten, den ephemeren öffentlichen Schlüssel und den Verifizierungs-Tag mit AES-128-CBC mit dem ECDH eines ephemeren Schlüsselpaares und dem öffentlichen Schlüssel des Veranstaltungsortes.

Der Veranstaltungsort erstellt einen Authentifizierungsschlüssel mit HMAC-SHA256 mit der trace-ID als Daten und dem Verifizierungstag als Schlüssel.

Der Veranstaltungsort wird die Check-in-Zeit, die verschlüsselten Daten und den Authentifizierungsschlüssel auf den Server hochladen, vgl. Fig. 10.

### Check-out (vgl. Fig. 11)

Wenn der Benutzer über die App (geofence oder manuell) auscheckt, sendet die App einen Checkout-Zeitstempel und einen HMAC-SHA256 mit dem Checkout-Zeitstempel als Daten und dem Authentifizierungsschlüssel (HMAC(data=trace id, key=verification tag) als Schlüssel).

### Infektion (vgl. Fig. 12)

Eine Gesundheitsbehörde fragt den Nutzer nach der Benutzerkennung (User-Id) und dem Geheimnis der Benutzerverfolgung (user trace secret). Das Gesundheitsministerium kann dann alle möglichen Trace-IDs der letzten zwei Wochen (20.160 Trace-IDs) berechnen und den Server fragen, welche Venues Daten mit diesen Trace-IDs hochgeladen haben. Das Gesundheitsamt erhält eine Liste der Venues zurück, die der Benutzer besucht hat.

Das Gesundheitsamt kann dann die Veranstaltungsorte auffordern, Zugang zu allen Benutzerdaten zu gewähren, die sich zur gleichen Zeit wie die infizierten Benutzer am Veranstaltungsort befanden. Dazu wird die entsprechende Trace-ID auf sichere Weise an den Veranstaltungsort übertragen. Der Veranstaltungsort kann von dem Server die hochgeladenen Daten anfragen, die sich mit der Check-in-Zeit des Traces überschneiden.

Der Veranstaltungsort wird die verschlüsselten Check-in-Daten herunterladen und entschlüsseln, die die für das Gesundheitsministerium verschlüsselten Daten enthalten, welche die User-ID und den user data key sowie den Verifizierungs-Tag enthalten.

Die Gesundheitsbehörden erhalten diese Liste der verschlüsselten Daten für jeden Veranstaltungsort und entschlüsseln sie und können dann das Benutzerdatenpaket (user data package) herunterladen und seinen Inhalt mit dem user data key und seinem privaten Schlüssel entschlüsseln.

Die Gesundheitsbehörde kann nun auch die Integrität des Check-ins mit dem Verifizierungsgeheimnis verifizieren.

Ausführungsformen des "Systems 2" können z.B. gemäß der folgenden Klauseln beschrieben werden:
Klausel 1:
1. Elektronisches System mit Mobilfunkgeräten, die jeweils einem Nutzer zugeordnet sind, Venue-Geräten und zumindest einem Server zum Tracing von Nutzern für eine Entität, der ein asymmetrisches kryptographisches Schlüsselpaar mit einem öffentlichen und einem privaten Entitätenschlüssel zugeordnet ist, wobei das elektronische System zur Durchführung der folgenden Schritte konfiguriert ist:
   - Speicherung von Nutzerdaten der Nutzer anlässlich einer Registrierung auf einem Server, wobei die Nutzerdaten durch den öffentlichen Entitätenschlüssel verschlüsselt sind und das Resultat dieser Verschlüsselung mit einem jeweiligen User-Data-Key des betreffenden Nutzers verschlüsselt ist,
   - Speicherung in jedem der Mobilfunkgeräte eines User-Data-Key, eines User-Secret und einer User-ID, wobei das Mobilfunkgerät über eine Netzwerkschnittstelle Zugriff auf eine aktuelle Zeitinformation hat und das Mobilfunkgerät konfiguriert ist zur Erzeugung einer Trace-ID, die repräsentativ ist für eine Kombination aus User-ID, User-Secret und der aktuellen Zeitinformation sowie zur Erzeugung eines Chiffrats aus dem User-Data-Key und der User-ID, wobei der öffentliche Entitätenschlüssel zur Erzeugung des Chiffrats verwendet wird, und wobei das Mobilfunkgerät eine Kommunikationsschnittstelle zur direkten Kommunikation mit einem Venue-Gerät aufweist, über welche die Trace-ID und das Chiffrat an das Venue-Gerät übertragen werden,
   - Erfassung eines der Nutzer durch ein Venue-Gerät mit Hilfe des Mobilfunkgeräts des Nutzers, wobei das Venue-Gerät eine Schnittstelle für die direkte Kommunikation mit der entsprechenden Schnittstelle des Mobilfunkgeräts aufweist, und mit einer Netzwerkschnittstelle zur Kommunikation mit dem Server, wobei das Venue-Gerät dazu konfiguriert ist, zur Erfassung des Nutzers die Trace-ID und das Chiffrat von dem Mobilfunkgerät zu empfangen und zu prüfen, ob die Zeitinformation einer empfangenen Trace-ID hinreichend aktuell ist, und wenn dies der Fall ist, die Trace-ID mit einer dem Venue-Gerät zugeordneten Venue-ID und dem Chiffrat an den Server zu übertragen,

   - Speicherung einer empfangenen Trace-ID und dem Chiffrat mit der Zeitinformation als Zugriffsschlüssel durch den Server,
   - Empfang einer Mitteilung der User-ID und des User-Secrets von einem der Nutzer an die Entität,
   - Erzeugung von möglichen zurückliegenden Trace-IDs für den betreffenden Nutzer, die innerhalb eines vorgegebenen vergangenen Zeitraums liegen und Verwendung der erzeugten Trace-IDs als Suchkriterium, um entsprechende auf dem Server für den vergangenen Zeitraum gespeicherte Trace-IDs aufzufinden, so dass die Venue-IDs derjenigen Venues von denen der Nutzer während des vergangenen Zeitraums erfasst wurde, ermittelt werden,
   - Ausgabe der Venue-IDs der ermittelten Venues zur Identifizierung derjenigen Venues, die der Nutzer während des vergangenen Zeitraums besucht hat.
2. Elektronisches System nach Klausel 1, wobei das elektronische System so konfiguriert ist, dass von dem Mobilfunkgerät des einen der Nutzer eine Check-out Zeit an den Server übertragen wird, wenn der Nutzer den venue verlässt, wobei die Check-out Zeit der zuvor für diesen Nutzer empfangenen Trace-ID zugeordnet wird, so dass für den Nutzer der Aufenthaltszeitraum an dem venue gespeichert wird.
3. Elektronisches System nach Klausel 2, wobei das elektronische System so konfiguriert ist, dass diejenigen Nutzer ermittelt werden, die ebenfalls einen oder mehrere der ermittelten Venues während des vergangenen Zeitraums und während einer Aufenthaltsdauer des Nutzers besucht haben, wozu diejenigen der trace-IDs, die von den ermittelten Venues während des vergangenen Zeitraums zu dem Server übertragen wurden, ermittelt werden.
4. Digitales Speichermedium mit von einem Prozessor eines Mobilfunkgeräts ausführbaren Programminstruktionen zur Durchführung derjenigen Schritte zu deren Durchführung des elektronische System nach Klausel 1, 2 oder 3 konfiguriert ist, welche das Mobilfunkgerät betreffen.
5. Computerimplementiertes Verfahren umfassend die von dem Mobilfunkgerät und/oder dem Server gemäß "System 2" auszuführenden Schritte.

### Offenbarungsteil "System 3", Figuren 8-12

In einem weiteren Aspekt umfasst diese Offenbarung ein weiteres System ("System 3") zum Tracing von Nutzern. Das "System 3" ist z.B. ebenfalls in den Figuren 8 bis 12 illustriert. Beispiele und Erläuterungen des Offenbarungsteils bezüglich des "System 1" (illustriert z.B. anhand der Figuren 1-7) und des Offenbarungsteils bezüglich des "Systems 2" (illustriert z.B. anhand der Figuren 8-12) und/oder des Offenbarungsteils bezüglich des "Systems 3" (ebenfalls illustriert z.B. anhand der Figuren 8-12) können frei miteinander kombiniert werden, sofern sie sich nicht gegenseitig ausschließen. Bezüglich der Bedeutung der Begriffe "Mobilfunkgerät", "Server", "Venue" und "Entität" wird auf die obenstehenden Definitionen verwiesen.

Das "System 3" betrifft ein elektronisches System zum Tracing von Nutzern, insbesondere zur Bekämpfung einer Pandemie, wie zum Beispiel Covid-19, sowie ein entsprechendes digitales Speichermedium, auf dem ein Computerprogramm bzw. eine App gespeichert ist.

Zur Begrenzung der Verbreitung von Covid-19 wird bislang so vorgegangen, dass bei Besuch einer öffentlichen Veranstaltung oder einer Gaststätte die betreffende Person ihre persönlichen Daten angeben muss. Die damit einhergehenden Dokumentationspflichten sind für die entsprechenden Veranstalter bzw. Betreiber von Restaurants aufwendig, zumal auch die datenschutzrechtlichen Bestimmungen eingehalten werden müssen.

Demgegenüber wird gemäß der technischen Lehre von "System 3" ein elektronisches System geschaffen, welches ein weitgehend automatisiertes Tracing unter Wahrung von datenschutzrechtlichen Vorschriften gewährleistet.

Das Mobilfunkgerät ist einem Nutzer eindeutig zugeordnet. Das Tracing soll für eine "Entität" stattfinden.

Gemäß Ausführungsformen des "Systems 3" wird so vorgegangen, dass sich die Nutzer zunächst mithilfe der jeweiligen Mobilfunkgeräte registrieren. Hierzu werden zunächst Nutzerdaten erfasst, also beispielsweise durch Eingabe der Nutzerdaten in das betreffende Mobilfunkgerät oder durch Einlesen der Nutzerdaten, beispielsweise aus einem elektronischen Personalausweis des Nutzers in das Mobilfunkgerät. Die Nutzerdaten werden dann mithilfe des öffentlichen Entitätenschlüssels, also beispielsweise mithilfe eines öffentlichen Schlüssels des Gesundheitsamts, von dem Mobilfunkgerät verschlüsselt und das Resultat dieser Verschlüsselung wird mit einem User-Data-Key des betreffenden Nutzers verschlüsselt, wobei es sich bei dem User-Data-Key hier beispielsweise um einen Zufallswert hoher Entropie handelt, wie zum Beispiel einer Länge von 16 Bytes.

Die Verschlüsselung der Nutzerdaten kann unmittelbar durch den öffentlichen Entitätenschlüssel erfolgen. Alternativ wird aus dem öffentlichen Entitätenschlüssel ein weiterer Schlüssel nach einem vordefinierten Verfahren abgeleitet, welcher dann zur Verschlüsselung der Nutzerdaten dient. Beispielsweise kann der weitere Schlüssel aus dem öffentlichen Entitätenschlüssel und dem User-Data-Key abgeleitet werden.

Bei dem Entitätenschlüssel kann es sich um einen Master key handeln oder den Schlüssel einer der Entität zugeordneten abhängigen Entität. Beispielsweise kann es sich im Fall des Gesundheitsamts um den Masterschlüssel des Gesundheitsamts oder um einen Schlüssel des jeweils vor Ort verantwortlichen Gesundheitsamts handeln.

Der User-Data-Key kann beispielsweise für die Zwecke der Registrierung durch das Betriebssystem des Mobilfunkgeräts zur Verfügung gestellt werden. Beispielsweise verfügt das Mobilfunkgerät über eine Binary Symmetric Source zur Erzeugung von Zufallswerten hoher Entropie.

Die Hinterlegung der Nutzerdaten auf dem Server erfolgt also in einer Form, die es dem Server zunächst nicht ermöglicht, diese Nutzerdaten zu entschlüsseln, da dies ohne den privaten Entitätenschlüssel nicht möglich ist.

In dem Mobilfunkgerät wird dieser User-Data-Key in einem nichtflüchtigen Speicher persistent gespeichert. Aufgrund der Registrierung auf dem Server erzeugt der Server eine User-ID, das heißt einen eindeutigen Identifikator für den betreffenden U-ser, die der Server aufgrund der Registrierung an das Mobilfunkgerät zurückgibt. Die User-ID wird in dem Mobilfunkgerät in einem nichtflüchtigen Speicher persistent gespeichert.

Um einen Nutzer beim Besuch einer venue zu erfassen, wird nun wie folgt vorgegangen:
Das Mobilfunkgerät erzeugt eine Trace-ID, die repräsentativ ist für eine Kombination aus der User-ID und einer aktuellen Zeitinformation. Bei der aktuellen Zeitinformation handelt es sich um die aktuelle Uhrzeit beim Check-in des Nutzers an dem Venue, wobei diese aktuelle Uhrzeit von dem Mobilfunkgerät über das Netzwerk ermittelt werden. Vorzugsweise wird diese aktuelle Uhrzeit nach einem vordefinierten Rundungsverfahren gerundet, beispielsweise auf die volle Minute auf- oder abgerundet.

Das Venue-Gerät prüft zur Erfassung eines Nutzers, ob die Zeitinformation der Trace-ID hinreichend aktuell ist und verwendet hierzu beispielsweise ebenfalls eine über ein Netzwerk bezogene Zeitinformation zur Synchronisierung mit der von dem Mobilfunkgerät verwendeten Zeitbasis. Die Zeitinformation gilt als aktuell, wenn sie beispielsweise innerhalb eines vorgegebenen Toleranzbereichs liegt, also beispielsweise nicht älter ist als einige Sekunden oder Minuten.

Wenn die Zeitinformation der Trace-ID hinreichend aktuell ist, so überträgt das Venue-Gerät die Trace-ID mit einer dem Venue-Gerät zugeordneten Venue-ID und dem Chiffrat an den Server. Diese Informationen werden auf dem Server gespeichert.

Vorzugsweise wird dies für eine möglichst große Anzahl von Nutzern, vorzugsweise für sämtliche Nutzer, durchgeführt, die eine venue besuchen, um so möglichst lückenlos sämtliche Nutzer zu erfassen.

Nachträglich kann das Erfordernis bestehen, den oder die Nutzer, welche den venue besucht haben, gegenüber der Entität offenzulegen. Dies ist beispielsweise dann der Fall, wenn einer der Nutzer positiv auf Covid-19 getestet wird. Dieser mit Covid-19 infizierte Nutzer meldet dies der Entität, hier dem Gesundheitsamt, und teilt der Entität seine User-ID und sein User-Secret mit.

Anhand dieser Informationen werden dann sämtliche mögliche zurückliegende Trace-ID, die zur Erfassung eines Nutzers durch ein Venue-Gerät für einen vorgegebenen vergangenen Zeitraum benutzt sein könnten, erzeugt. Die Menge dieser möglichen Trace-IDs ist relativ begrenzt, da nicht sämtliche mögliche Zeiten innerhalb des relevanten Zeitraums in Frage kommen, sondern nur diejenigen, die aufgrund des vorgegebenen Rundungsverfahrens tatsächlich vorkommen können.

Diese Trace-IDs werden dann als Suchkriterium verwendet, um entsprechend auf dem Server für den vergangenen Zeitraum gespeicherte Trace-IDs aufzufinden, sodass die Venue-IDs derjenigen Venues, von denen der Nutzer während des vergangenen Zeitraums erfasst wurde, ermittelt werden. Hierdurch erhält also das Gesundheitsamt die Venue-IDs der ermittelten Venus, die der Nutzer in dem vergangenen Zeitraum, beispielsweise zwei Wochen, in denen er möglicherweise bereits infektiös war, besucht hatte.

Nach einer Ausführungsform des "System 3" ist das elektronische System so konfiguriert, dass von dem Mobilfunkgerät eines durch ein Venue-Gerät erfassten Nutzers, also nach einem Check-in, eine Check-out Zeit an den Server übertragen wird, wenn der Nutzer den venue verlässt. Diese Check-out-Zeit ist der zuvor für diesen Nutzer empfangenen Trace-ID zugeordnet, sodass serverseitig die Information vorliegt, im welchen Zeitraum sich der Nutzer an dem venue aufgehalten hat. Alternativ erfolgt dies durch das venue-Gerät.

Vorzugsweise geht diese Information in die Ermittlung derjeniger Nutzer ein, die sich ebenfalls an dem venue aufgehalten hatten, indem die Suche auf solche Nutzer beschränkt wird, die nicht nur den gleichen venue besucht hatten, sondern dort auch in einem zumindest mit dem Aufenthaltszeitraum des erkannten Nutzers überlappenden Aufenthaltszeitraums gewesen sind. Nach einer Ausführungsform des "System 3" erfolgt dieser Check-out durch manuelle Eingabe in das Mobilfunkgerät oder automatisch durch sogenanntes Geofencing. In letzterem Fall wird die Check-out-Zeit mit der zugeordneten Trace-ID automatisch von dem Mobilfunkgerät an den Server gesendet, sobald der Nutzer die venue verlässt. Alternativ erfolgt dies durch das venue-Gerät.

Als Venue-ID kann beispielsweise der Name und/oder Adresse, ein Identifikator oder auch die geografischen Koordinaten des Venues dienen.

Nach Ausführungsform des Konzepts von "System 3" wird wie folgt vorgegangen:
a) Registrierung: Der Nutzer verschlüsselt mithilfe seines Mobilfunkgeräts, d. h. einer darauf ausgeführten App, seine schutzbedürftigen Daten mit dem öffentlichen Entitätenschlüssel (zum Beispiel einm Gesundheitsamtschlüssel oder je nach Ausführungsform einem daraus abgeleiteten Schlüssel) und lädt diese verschlüsselten schutzbedürftigen Daten auf den Server. Der Server ordnet den von dem Mobilfunkgerät des Nutzers empfangenen verschlüsselten schutzbedürftigen Daten eine User-ID zu und gibt diese User-ID an die App des Nutzers zurück. Diese User ID wird von der App in einem Speicher des Mobilfunkgeräts gespeichert.
b) Check-In: das venue-Gerät erhält die User-ID von dem Mobilfunkgerät des Nutzers. Je nach Ausführungsform kann dies so erfolgen, dass die User-ID von dem Mobilfunkgerät im Klartext gesendet wird oder in einer verschlüsselter Form. In letzterem Fall kann für jeden check-in ein key generiert werden, so dass sich die User -ID jedes Mal zu ändern scheint; auch andere Verschlüsselungsverfahren für die User ID sind möglich, wenn man ein tracing anhand der User-ID verhindern möchte. Das venue-Gerät ergänzt zu der User-ID die time stamp und die venue ID und schickt das (ggf. verschlüsselt) auf den Server.
c) Check-out: von venue oder app wird die check-out timestamp auf den Server geschickt und zwar der User-Id (oder einem Chiffrat davon) zugeordnet.

### 3. Infektion:

a) Der Nutzer teilt dem Gesundheitsamt seine User ID mit. Damit lässt sich mit Hilfe der auf dem Server gespeicherten Daten feststellen, an welchen venues der Nutzer z.B. in den letzten zwei Wochen gewesen ist.
b) Dann wird abgefragt, welche User-IDs gleichzeitig mit dem infizierten User in dem relevanten Zeitraum an den Venues waren.
c) Die verschlüsselten schutzbedürftigen Datensätze dieser User können dann mit dem privaten Gesundheitsamtschlüssel entschlüsselt werden und vom Gesundheitsamt kontaktiert werden.

In einem weiteren Aspekt betrifft die technische Lehre zu "System 3" ein digitales Speichermedium mit von einem Prozessor eines Mobilfunkgeräts ausführbaren Programminstruktionen.

Beispielsweise kann ein Verfahren zum Tracing von Nutzern für eine Entität mit den folgenden Schritten durchgeführt werden:
- Speicherung von Nutzerdaten der Nutzer anlässlich einer Registrierung auf einem Server, wobei die Nutzerdaten durch den öffentlichen Entitätenschlüssel verschlüsselt sind und das Resultat dieser Verschlüsselung optional mit einem jeweiligen User-Data-Key des betreffenden Nutzers verschlüsselt ist,
- Zurverfügungstellung von tragbaren Mobilfunkgeräten, die jeweils einem Nutzer zugeordnet sind, wobei in jedem der Mobilfunkgeräte eine User-ID gespeichert ist, wobei das Mobilfunkgerät über eine Netzwerkschnittstelle Zugriff auf eine aktuelle Zeitinformation hat und das Mobilfunkgerät konfiguriert ist zur Erzeugung einer Trace-ID, die repräsentativ ist für eine Kombination aus User-ID und der aktuellen, und wobei das Mobilfunkgerät eine Kommunikationsschnittstelle zur direkten Kommunikation mit einem Venue-Gerät aufweist, über welche die Trace-ID und das Chiffrat an das Venue-Gerät übertragen werden,

- Erfassung eines der Nutzer durch ein Venue-Gerät mit Hilfe des Mobilfunkgeräts des Nutzers, wobei das Venue-Gerät eine Schnittstelle für die direkte Kommunikation mit der entsprechenden Schnittstelle des Mobilfunkgeräts aufweist, und mit einer Netzwerkschnittstelle zur Kommunikation mit dem Server, wobei das Venue-Gerät dazu konfiguriert ist, zur Erfassung des Nutzers die Trace-ID von dem Mobilfunkgerät zu empfangen und zu prüfen, ob die Zeitinformation einer empfangenen Trace-ID hinreichend aktuell ist, und wenn dies der Fall ist, die Trace-ID mit einer dem Venue-Gerät zugeordneten Venue-ID und dem Chiffrat an den Server zu übertragen,
- Speicherung einer empfangenen Trace-ID und dem Chiffrat mit der Zeitinformation als Zugriffsschlüssel durch den Server,
- Mitteilung der User-ID und des User-Secrets von einem der Nutzer an die Entität,
- Erzeugung von möglichen zurückliegenden Trace-IDs für den betreffenden Nutzer, die innerhalb eines vorgegebenen vergangenen Zeitraums liegen und Verwendung der erzeugten Trace-IDs als Suchkriterium, um entsprechende auf dem Server für den vergangenen Zeitraum gespeicherte Trace-IDs aufzufinden, so dass die Venue-IDs derjenigen Venues von denen der Nutzer während des vergangenen Zeitraums erfasst wurde, ermittelt werden,
- Ausgabe der Venue-IDs der ermittelten Venues zur Identifizierung derjenigen Venues, die der Nutzer während des vergangenen Zeitraums besucht hat.

Nach einer weiteren Ausführungsform des Verfahrens wird von dem Mobilfunkgerät des einen der Nutzer oder dem venue-Gerät eine Check-out Zeit an den Server übertragen, wenn der Nutzer den venue verlässt, wobei die Check-out Zeit der zuvor für diesen Nutzer empfangenen Trace-ID zugeordnet wird, so dass für den Nutzer der Aufenthaltszeitraum an dem venue gespeichert wird.

Nach einer weiteren Ausführungsform des Verfahrens werden diejenigen Nutzer ermittelt, die ebenfalls einen oder mehrere der ermittelten Venues während des vergangenen Zeitraums und während einer Aufenthaltsdauer des Nutzers besucht haben, wozu diejenigen der trace-IDs, die von den ermittelten Venues während des vergangenen Zeitraums zu dem Server übertragen wurden, ermittelt werden.

Im Weiteren werden Ausführungsformen des "Systems 3" mit Bezugnahme auf die gleichen Zeichnungen 8-12 näher erläutert, die bereits zur Erläuterung des "Systems 2" dienten.

Mit Bezug auf die Fig. 8 wird die Registrierung beschrieben.

Der Benutzer stellt der App (Anwendungsprogramm) seines Mobilfunkgeräts seine Kontaktinformationen zur Verfügung. Die Anwendung erzeugt ein EC secp256r1-Schlüsselpaar (user public key und user private key), ein Benutzer-Trace-Geheimnis, d.h. ein user trace secret (16 Bytes Entropie), ein Benutzer-Verifizierungs-Geheimnis, d.h. user verification secret ein (16 Bytes Entropie) und einen Benutzer-Datenschlüssel, d.h. user data key (16 Bytes Entropie).

Dann berechnet die Anwendung einen Verschlüsselungsschlüssel mit ECDH mit dem privaten Schlüssel des Benutzers und dem öffentlichen Hauptschlüssel (Entitätenschlüssel) des Gesundheitsamtes. Die App berechnet ein HMAC-SHA256 mit der ECDH-Ausgabe als Daten und dem Datenschlüssel als Schlüssel. Die Anwendung verschlüsselt dann die persönlichen Informationen und das Benutzerverifizierungsgeheimnis mit diesem Verschlüsselungsschlüssel mit AES-128-CBC. Die Anwendung lädt dann diese verschlüsselten Daten hoch und signiert sie mit dem privaten Schlüssel des Benutzers zusammen mit dem öffentlichen Schlüssel des Benutzers. Der Server gibt eine Benutzerkennung, d.h. User-ID, zurück.

Im Mobilfunkgerät werden gespeichert:
- user id (uuid)
- user public key (ec secp256r1)
- user private key (ec secp256r1)
- user data key (16 bytes random data)
- user trace secret (16 bytes random data)
- user verification secret (16 bytes random data)

### Einchecken (vgl. Fig. 9)

Die App generiert für jedes Einchecken ein Signal zur Übertragung der trace-ID, welches per direkter Kommunikation übertagen wird, beispielsweise per Anzeige eines QR-Codes, zur Übertragung einer Trace-ID, eines ephemeres ECC-Schlüsselpaar secp256r1 und eines Verschlüsselungsschlüssels vorzugsweise für jeden neuen QR Code. S.

Die Trace-ID ist ein HMAC-SHA256, der aus der User-ID und dem aktuellen Zeitstempel (abgerundet auf die aktuelle Minute) als Daten und dem Benutzer-Trace-Geheimnis als Schlüssel abgeleitet und auf die ersten 16 Bytes gekürzt wird).

Der Verschlüsselungsschlüssel wird mit ECDH aus dem ephemeren privaten Schlüssel und dem öffentlichen Schlüssel des Gesundheitsministeriums berechnet. Die App wird einen QR-Code mit folgendem Inhalt anzeigen (der jede Minute mit dem aktuellen Zeitstempel aktualisiert wird):
- QR-Code-Version (1 Byte)
- Zeitstempel (4 Bytes)
- Trace-ID (16 Bytes)
- verschlüsselte Daten (32 Bytes) (Datenschlüssel und Benutzerkennung mit AES-128-CBC verschlüsselt und der Verschlüsselungsschlüssel als Schlüssel und die Trace-ID als IV) ephemerer öffentlicher Schlüssel (33 Bytes)
- Verifizierungs-Tag (8 Bytes) (HMAC-SHA256 mit traceid und verschlüsselten Daten als Daten und dem Verifizierungsgeheimnis des Benutzers als Schlüssel, abgeschnitten auf die ersten 8 Bytes)

Die Gesamtnutzlast beträgt 94 Bytes, die einfach verkettet und dann mit ASCII85 als QR-Code-Inhalt kodiert werden. Das ephemere Schlüsselpaar kann verworfen werden.

Der Veranstaltungsort, d.h. das Venue-Gerät, prüft, ob der Zeitstempel neueren Datums ist und führt keine weitere Validierung durch.

Der Veranstaltungsort verschlüsselt die empfangenen verschlüsselten Daten, den ephemeren öffentlichen Schlüssel und den Verifizierungs-Tag mit AES-128-CBC mit dem ECDH eines ephemeren Schlüsselpaares und dem öffentlichen Schlüssel des Veranstaltungsortes.

Der Veranstaltungsort erstellt einen Authentifizierungsschlüssel mit HMAC-SHA256 mit der trace-ID als Daten und dem Verifizierungstag als Schlüssel.

Der Veranstaltungsort wird die Check-in-Zeit, die verschlüsselten Daten und den Authentifizierungsschlüssel auf den Server hochladen, vgl. Fig. 10.

### Check-out (vgl. Fig. 11)

Wenn der Benutzer über die App (geofence oder manuell) auscheckt, sendet die App einen Checkout-Zeitstempel und einen HMAC-SHA256 mit dem Checkout-Zeitstempel als Daten und dem Authentifizierungsschlüssel (HMAC(data=trace id, key=verification tag) als Schlüssel).

### Infektion (vgl. Fig. 12)

Eine Gesundheitsbehörde fragt den Nutzer nach der Benutzerkennung (User-Id) und dem Geheimnis der Benutzerverfolgung (user trace secret). Das Gesundheitsministerium kann dann alle möglichen Trace-IDs der letzten zwei Wochen (20.160 Trace-IDs) berechnen und den Server fragen, welche Venues Daten mit diesen Trace-IDs hochgeladen haben. Das Gesundheitsamt erhält eine Liste der Venues zurück, die der Benutzer besucht hat.

Das Gesundheitsamt kann dann die Veranstaltungsorte auffordern, Zugang zu allen Benutzerdaten zu gewähren, die sich zur gleichen Zeit wie die infizierten Benutzer am Veranstaltungsort befanden. Dazu wird die entsprechende Trace-ID auf sichere Weise an den Veranstaltungsort übertragen. Der Veranstaltungsort kann von dem Server die hochgeladenen Daten anfragen, die sich mit der Check-in-Zeit des Traces überschneiden.

Der Veranstaltungsort wird die verschlüsselten Check-in-Daten herunterladen und entschlüsseln, die die für das Gesundheitsministerium verschlüsselten Daten enthalten, welche die User-ID und den user data key sowie den Verifizierungs-Tag enthalten.

Die Gesundheitsbehörden erhalten diese Liste der verschlüsselten Daten für jeden Veranstaltungsort und entschlüsseln sie und können dann das Benutzerdatenpaket (user data package) herunterladen und seinen Inhalt mit dem user data key und seinem privaten Schlüssel entschlüsseln.

Die Gesundheitsbehörde kann nun auch die Integrität des Check-ins mit dem Verifizierungsgeheimnis verifizieren.

## Patentansprüche

1. Verfahren zum Tracing von Nutzern, die sich temporär in verschiedenen räumlichen Bereichen, im weiteren Venues genannt, aufhalten, für eine Entität (246), insbesondere für Notfallszenarien wie in einer Pandemie, wobei das Verfahren umfasst:
- anlässlich einer Registrierung (102) eines jeden der Nutzer: Speicherung von Nutzerdaten (306) des Nutzers auf einem Server (202), wobei die Nutzerdaten (314) von einem dem Nutzer zugeordneten Nutzergerät mit einem für den Nutzer individuellen User-Data-Key (310) verschlüsselt werden, wobei insbesondere eine User-ID (302) des Nutzers verknüpft mit den verschlüsselten Nutzerdaten (306) auf dem Server gespeichert wird; und Speicherung der User-ID und eines Nutzer-individuellen, zur Entschlüsselung der verschlüsselten Nutzerdaten erforderlichen, User-Data-Entschlüsselungsschlüssels (310) in dem Nutzergerät, wobei vorzugsweise der User-Data-Key und/oder der User-Data-Entschlüsselungsschlüssel (310) geschützt gespeichert werden und dem Server nicht zugänglich sind;
- Im Zuge eines Check-ins (104) eines der Nutzer gegenüber einem der Venues, Erzeugung einer Trace-ID durch das Nutzergerät des einen Nutzers, wobei die Trace-ID repräsentativ ist für eine Kombination aus der User-ID des einen Nutzers und einer aktuellen Zeitinformation, und Speicherung der Trace-ID verknüpft mit der Venue-ID des einen Venue auf dem Server (202), wobei die Trace-ID als Zugriffsschlüssel gespeichert wird,
- Empfang (106) einer Mitteilung beinhaltend die User-ID eines der Nutzer, bezüglich welchem das Tracing durchgeführt werden soll;
- Erzeugung (108) von Kandidaten-Trace-IDs für den in der Mitteilung identifizierten Nutzer, wobei die Kandidaten-Trace-IDs mögliche zurückliegende Trace-IDs sind, die innerhalb eines vorgegebenen vergangenen Zeitraums liegen,
- Verwendung (110) der erzeugten Kandidaten-Trace-IDs als Suchkriterium durch den Server (202), um entsprechende auf dem Server für den vergangenen Zeitraum gespeicherte Trace-IDs aufzufinden, so dass die Venue-IDs (502) derjenigen Venues gegenüber welchen der zu tracende Nutzer während des vergangenen Zeitraums einen Check-in durchgeführt hat, ermittelt werden, und
- Ausgabe (112) der Venue-IDs der ermittelten Venues zur Identifizierung derjenigen Venues, die der zu tracende Nutzer während des vergangenen Zeitraums besucht hat, durch den Server (202).

2. Elektronisches System (200) mit Nutzergeräten (204), die jeweils einem Nutzer (240) zugeordnet sind, und mit zumindest einem Server (202) zum Tracing von Nutzern, die sich temporär in verschiedenen räumlichen Bereichen, im folgenden Venues genannt, aufhalten, für eine Entität (246), wobei das elektronische System zur Durchführung der folgenden Schritte konfiguriert ist:
- anlässlich einer Registrierung (102): Speicherung von Nutzerdaten (300) der Nutzer auf dem Server (202), wobei die Nutzerdaten mit einem für den jeweiligen Nutzer individuellen User-Data-Key (310) verschlüsselt sind, wobei insbesondere eine User-ID (302) des betreffenden Nutzers verknüpft mit den verschlüsselten Nutzerdaten (306) auf dem Server gespeichert werden; und Speicherung der User-IDs und eines Nutzer-individuellen, zur Entschlüsselung der verschlüsselten Nutzerdaten erforderlichen, User-Data-Entschlüsselungsschlüssels (310) jeweils in demjenigen der Nutzergeräte, welches dem Nutzer zugeordnet ist, wobei vorzugsweise der User-Data-Key und/oder der User-Data-Entschlüsselungsschlüssel (310) geschützt in dem Nutzergerät des jeweiligen Nutzer gespeichert wird und dem Server nicht zugänglich ist;
- Im Zuge eines Check-ins (104) eines der Nutzer gegenüber einem der Venues, Erzeugung einer Trace-ID durch das Nutzergerät des einen Nutzers, wobei die Trace-ID repräsentativ ist für eine Kombination aus User-ID des einen Nutzers und einer aktuellen Zeitinformation, und Speicherung der Trace-ID verknüpft mit der Venue-ID des einen Venue auf dem Server (202), wobei die Trace-ID als Zugriffsschlüssel gespeichert wird,
- Empfang (106) einer Mitteilung beinhaltend die User-ID eines der Nutzer, bezüglich welchem das Tracing durchgeführt werden soll;
- Erzeugung (108) von Kandidaten-Trace-IDs für den in der Mitteilung identifizierten Nutzer, wobei die Kandidaten-Trace-IDs mögliche zurückliegende Trace-IDs sind, die innerhalb eines vorgegebenen vergangenen Zeitraums liegen,
- Verwendung (110) der erzeugten Kandidaten-Trace-IDs als Suchkriterium durch den Server (202), um entsprechende auf dem Server für den vergangenen Zeitraum gespeicherte Trace-IDs aufzufinden, so dass die Venue-IDs derjenigen Venues gegenüber welchen der zu tracende Nutzer während des vergangenen Zeitraums einen Check-in durchgeführt hat, ermittelt werden, und
- Ausgabe (112) der Venue-IDs der ermittelten Venues zur Identifizierung derjenigen Venues, die der zu tracende Nutzer während des vergangenen Zeitraums besucht hat, durch den Server (202).

3. Verfahren oder elektronisches System nach einem der vorigen Ansprüche,
- wobei die im Zuge des Check-ins erzeugte Trace-ID mittels einer Ableitfunktion als Funktion der Kombination der zu repräsentierenden User-ID und der zu repräsentierenden aktuellen Zeitinformation beim Check-in berechnet wird; und
- wobei jeder der Kandidaten-Trace-IDs jeweils mittels der gleichen Ableitfunktion als Funktion der Kombination der in der Mitteilung enthaltenen User -ID und einer der Kandidaten-Zeitinformationen für einen möglichen in der Vergangenheit liegenden Check-in-Zeitpunkt, berechnet wird,
- wobei vorzugsweise die Ableitfunktion umfasst eine Rundung der aktuellen bzw. Kandidaten-Zeitinformation auf Zeitpunkte innerhalb vordefinierter Zeitintervalle, wobei die Zeitintervalle insbesondere Minuten oder Stunden oder Tage sind.

4. Verfahren oder elektronisches System nach Anspruch 3,
- wobei das Nutzergerät dazu konfiguriert ist, im Zuge der Registrierung gegenüber dem Server einen Zufallswert (userTracingSecret, 410) zu erzeugen und zugriffsgeschützt ausschließlich im Nutzergerät zu speichern;
- wobei die Ableitfunktion die Trace-ID als Funktion des Zufallswerts (410) berechnet;
- wobei das Nutzergerät dazu konfiguriert ist, von dem Benutzer, dem es zugewiesen ist, eine Bestätigung zur Übermittlung des Zufallswerts einzuholen, und erst in Antwort auf die Bestätigung den Zufallswert an den Server oder an ein Entität-Gerät der Entität zu übermitteln um dem Server oder der Entität die Ableitung der Kandidaten-Trace-IDs zu ermöglichen.

5. Verfahren oder elektronisches System nach einem der vorigen Ansprüche ,
- wobei das Nutzergerät dazu konfiguriert ist, im Zuge der Registrierung gegenüber dem Server einen Zufallswert (412) zu erzeugen, im Folgenden als userVerificationSecret bezeichnet, und zugriffsgeschützt ausschließlich im Nutzergerät zu speichern;
- wobei das Nutzergerät dazu konfiguriert ist, im Zuge des Check-ins gegenüber dem Venue das userVerificationSecret zu verwenden, um mit diesem einen Verifikationsdatenwert (414) als Funktion der Trace-ID (406) und den verschlüsselten Ausweis-Daten (408) zu berechnen und verknüpft mit der Trace-ID auf dem Server zu speichern.

6. Verfahren oder elektronisches System nach einem der vorigen Ansprüche,
- wobei der Entität ein asymmetrisches kryptographisches Schlüsselpaar mit einem öffentlichen und einem privaten Entitätenschlüssel zugeordnet ist,
- wobei das Nutzergerät des Nutzers dazu konfiguriert ist, im Zuge der Registrierung die bereits mit dem Nutzerdaten-Schlüssel (310) verschlüsselten Nutzerdaten zusätzlich mit dem öffentlichen Entitätenschlüssel (332) zu verschlüsseln, und/oder
- wobei das Nutzergerät des Nutzers im Zuge des Check-ins dazu konfiguriert ist, den für die Entschlüsselung dieser verschlüsselten Nutzerdaten (306) erforderlichen Nutzerdaten-Entschlüsselungsschlüssel (310) durch den öffentlichen Entitätenschlüssel zusammen mit der User-ID des Nutzers zu verschlüsseln, um verschlüsselte Ausweis-Daten (408) zu erzeugen, und dazu konfiguriert ist, die verschlüsselten Ausweis-Daten (408, 506) im Zuge des Check-ins verknüpft mit der Venue-ID (502) des einen Venue auf dem Server (202) zu speichern.

7. Verfahren oder elektronisches System nach Anspruch 6, ferner umfassend zumindest das Entitäts-Gerät (208), das der Entität zugeordnet ist,
- wobei das Entitäts-Gerät dazu konfiguriert ist, nacheinander mehrere asymmetrische kryptographische Schlüsselpaare zu erzeugen, die der Entität zugewiesen sind, wobei bei Erzeugung eines neuen Entitäten-Schlüsselpaares das bisher gültige invalidiert wird, wobei jedem der Entitäten-Schlüsselpaare eine Schlüsselversionsnummer zugewiesen ist,
- wobei das zumindest eine Entitäts-Gerät dazu konfiguriert ist, den öffentlichen Schlüssel des erzeugten und aktuell gültigen Schlüsselpaares an die Nutzergeräte zu übertragen;
- wobei die Nutzergeräte dazu konfiguriert sind, immer nur den jüngsten übertragenen Entitätenschlüssel (332) zur Erzeugung der mehrfach verschlüsselten Nutzerdaten und/oder zur Erzeugung der verschlüsselten Ausweis-Daten (408) zu verwenden,
wobei insbesondere:
- das zumindest das Entitäts-Gerät (208) eine Vielzahl von Entitätsgeräten umfasst,
- wobei dasjenige der Entitäts-Geräte, welches das aktuelle asymmetrische Entitäten-Schlüsselpaar erzeugt, dazu konfiguriert ist, den privaten Schlüssel des aktuell erzeugten asymmetrischen Entitäten-Schlüsselpaares in verschlüsselter Form an alle anderen der Entitäts-Geräte über ein Netzwerk zu senden,
- wobei jedes der Entitäts-Geräte dazu konfiguriert ist, eine Mitteilung eines Notfalls mit Bezug zu einem der Nutzer zu erzeugen und an den Server (202) zu senden und/oder dazu konfiguriert ist, mittels eines der bisher empfangenen privaten Entitätenschlüssel die verschlüsselten Nutzerdaten und/oder die verschlüsselten Ausweis-Daten (408) zu entschlüsseln.

8. Verfahren oder elektronisches System nach einem der vorigen Ansprüche, ferner umfassend mehrere Venue-Geräte (206), wobei jedem der Venues ein oder mehrere der Venue-Geräte zugewiesen sind,
wobei insbesondere:
der Nutzer den Check-in gegenüber einem der Venue-Geräte (206) durchführt, wobei das eine Venue-Gerät eine Kommunikationsschnittstelle zur direkten Kommunikation mit dem Nutzergerät eines eincheckenden Nutzers beinhaltet, wobei das Nutzergerät und das eine Venue-Gerät so konfiguriert sind, dass im Zuge des Check-ins folgendes erfolgt:
- Erzeugung des Check-in Datenpakets (400, 420, 430) durch das Nutzergerät, wobei das Check-in Datenpaket die Trace-ID (406) und vorzugweise auch die verschlüsselten Ausweisdaten (408) kodiert;
- Übertragung des Check-in Datenpakets vom Nutzergerät über die direkte Kommunikationsschnittstelle an das Venue-Gerät;
- Dekodieren des empfangenen Check-in Datenpakets durch das Venue-Gerät, um die Trace-ID und vorzugsweise auch die Ausweisdaten (408) des eincheckenden Nutzers zu erhalten und diese verknüpft mit der Venue-ID des Venue, dem das Venue-Gerät zugewiesen ist, auf dem Server (202) zu speichern;
- wobei es sich vorzugsweise bei der direkten Kommunikationsverbindung handelt um eine Nahfeld-Kommunikationsverbindung oder um eine optische Datenübertragung, wobei die optische Datenübertragung eine Erfassung eines graphischen Codes, der das Check-in Datenpaket kodiert und auf einer Anzeige des Nutzergeräts angezeigt wird, durch einen optischen Sensor des Venue-Geräts umfasst.

9. Verfahren oder elektronisches System nach einem der vorigen Ansprüche, ferner umfassend zumindest ein Trägerobjekt mit einem graphischen Venue-Code eines der Venues, wobei der Venue-Code eine Venue-ID kodiert, unter welcher dieser Venue bei dem Server (202) registriert ist, wobei der Nutzer den Check-in gegenüber diesem Venue vornimmt, wobei das Nutzergerät so konfiguriert ist, dass im Zuge des Check-ins folgendes erfolgt:
- Erfassung des graphischen Venue-Codes mit einer Kamera des Nutzergerätes;
- Dekodierung des Venue-Codes durch das Nutzergerät, um die Venue-ID des Venues zu erhalten;
- wobei die Übertragung der Trace-ID und vorzugsweise auch der Ausweisdaten (408) des eincheckenden Nutzers an den Server durch das Nutzergerät erfolgt, wobei hierbei auch die dekodierte Venue-ID verknüpft mit der Trace-ID auf dem Server (202) gespeichert wird;
- wobei es sich bei dem Venue-Code vorzugsweise um einen QR-Code oder Barcode handelt, der auf einem Aufdruck oder einer Digitalanzeige sichtbar ist, und/oder wobei es sich bei dem Trägerobjekt vorzugsweise handelt um einen Tischaufsteller oder ein Türschild zu einem Fahrzeugwagen, Fahrzeugabteil, Gebäude oder Zimmer.

10. Verfahren oder elektronisches System nach einem der vorigen Ansprüche, wobei das elektronische System so konfiguriert ist, dass von dem Nutzergerät des einen der Nutzer oder von dem Venue-Gerät eine Check-out Zeit an den Server übertragen wird, wenn der Nutzer den Venue verlässt, wobei die Check-out Zeit der zuvor für diesen Nutzer empfangenen Trace-ID zugeordnet wird, so dass für den Nutzer der Aufenthaltszeitraum an dem Venue gespeichert wird.

11. Verfahren oder elektronisches System nach einem der vorigen Ansprüche, wobei das System konfiguriert ist zur:
- Erzeugung eines Tracinggeheimnis-Transferobjekts durch das Nutzergerät, wobei das Tracinggeheimnis-Transferobjekt zumindest die User-ID und den Nutzerdaten-Entschlüsselungsschlüssel des Nutzers, dem das Nutzergerät zugewiesen ist, enthält;
- Verschlüsselung des Tracinggeheimnis-Transferobjekts mit einem öffentlichen Schlüssel (332) der Entität durch das Nutzergerät, wobei es sich bei dem öffentlichen Schlüssel insbesondere um den jüngsten aus einer Reihe nacheinander erzeugter öffentlicher Entitätenschlüssel handelt;
- Übertragung des verschlüsselten Tracinggeheimnis-Transferobjekts über ein Netzwerk von dem Nutzergerät an den Server;
- In Antwort auf den Erhalt des verschlüsselten Tracinggeheimnis-Transferobjekt, Erzeugen einer TAN und Speicherung der TAN verknüpft mit dem verschlüsselten Tracinggeheimnis-Transferobjekt durch den Server, und Ausgabe der TAN an den Nutzer;
- Empfang der TAN von dem Nutzer durch ein der Entität zugewiesenes Entitäts-Gerät (208);
- Verwenden der TAN durch das Entitäts-Gerät, um in Interoperation mit dem Server die verschlüsselten Nutzerdaten des Nutzers zu empfangen und zu entschlüsseln.

12. Verfahren oder elektronisches System nach Anspruch 11, wobei das Verwenden der TAN umfasst:
- Senden einer ersten Nutzerdatenanfrage beinhaltend die TAN an den Server (202) durch das Entitätsgerät (208);
- Empfang einer ersten Nutzerdatenanfrage beinhaltend die TAN durch den Server (202) von dem Entitätsgerät;
- In Antwort auf den Empfang der Nutzerdatenanfrage, senden des verschlüsselten Tracinggeheimnis-Transferobjekts, das mit der TAN verknüpft gespeichert ist, von dem Server an das Entitäts-Gerät;
- Entschlüsseln des Tracinggeheimnis-Transferobjekts mit dem privaten Entitätenschlüssel, der zu dem öffentlichen Schlüssel (332) der Entität korrespondiert, um die User-ID und den zur Entschlüsselung der verschlüsselten Nutzerdaten des Nutzers erforderlichen User-Data-Entschlüsselungsschlüssel (310) zu erhalten, durch das Entitäten-Gerät; und
- Verwenden der entschlüsselten User-ID, um eine zweite Nutzerdatenanfrage beinhaltend die User-ID zu erzeugen und vom Entitäten-Gerät an den Server zu senden; und
- Empfang der verschlüsselten Nutzerdaten des Nutzers vom Server durch das Entitäten-Gerät in Antwort auf die zweite Anfrage; und
- Entschlüsselung der verschlüsselten Nutzerdaten mittels des in dem Tracinggeheimnis-Transferobjekt enthaltenen Nutzerdaten-Entschlüsselungsschlüssels durch das Entitäten-Gerät.

13. Verfahren oder elektronisches System nach einem der vorigen Ansprüche, wobei die Trace-IDs die User-ID nicht im Klartext enthalten und wobei die Trace-IDs so erzeugt werden, dass allein auf Basis einer Analyse der auf dem Server gespeicherten Trace-IDs nicht festgestellt werden kann, ob zwei Trace-IDs die gleiche oder unterschiedliche User-IDs repräsentieren.

14. Verfahren oder elektronisches System nach einem der vorigen Ansprüche,
- wobei das Nutzergerät dazu konfiguriert ist, im Zuge des Check-Ins die eigene aktuelle Position zu erfassen;
- wobei das System dazu konfiguriert ist, im Zuge des Check-ins zu prüfen, ob das Nutzergerät innerhalb einer maximalen Distanz von der Position des Venues entfernt ist, und den Check-in nur dann durchzuführen, wenn das Nutzergerät innerhalb der maximalen Distanz von der Position des Venues entfernt liegt.

15. Verfahren oder elektronisches System nach Anspruch 14, wobei die Erfassung der aktuellen Position des Nutzergeräts ausgewählt ist aus seiner Gruppe umfassend:
- Erfassung von GPS-Daten des Nutzergeräts mittels eines GPS-Sensors des Nutzergeräts, wobei die von dem Nutzergerät ermittelten GPS Daten mit Positionsdaten des Venues verglichen werden um festzustellen, ob das Nutzergerät beim Check-in innerhalb der maximalen Distanz von dem Venue entfernt ist;
- Erfassung eines WLAN-Identifikators eines lokal vorhandenen WLAN-Netzes, wobei der von dem Nutzergerät ermittelte WLAN-Identifikator bei der Prüfung als Nachweis fungiert, dass das Nutzergerät beim Check-in innerhalb der maximalen Distanz von dem Venue entfernt ist;
- Erfassung eines Nahfeldsignals von einem Nahfeldsignalsender des Venues, wobei das Nahfeldsignal einen Identifikator des Venues codiert und wobei der von dem Nutzergerät über das Nahfeldsignal ermittelte Identifikator des Venues bei der Prüfung als Nachweis fungiert, dass das Nutzergerät beim Check-in innerhalb der maximalen Distanz von dem Venue entfernt ist.

16. Computerprogramm, Computerprogrammprodukt oder Digitales Speichermedium mit von einem Prozessor eines Datenverarbeitungsgeräts ausführbaren Programminstruktionen zur Durchführung derjenigen Schritte, zu deren Durchführung eine oder mehrere Komponenten des elektronischen Systems nach einem der vorigen Ansprüche 8 -15 konfiguriert ist, wobei diese Systemkomponenten das Nutzergerät oder der Server (202) oder das Venue-Gerät oder das Entitäten-Gerät sind.

## Claims

1. A method for tracing users who are temporarily located in different spatial areas, referred to hereinafter as venues, for an entity (246), in particular for emergency scenarios such as a pandemic, wherein the method comprises:
- upon registration (102) of each of the users: storing user data (306) of the user on a server (202), wherein the user data (314) is encrypted by a user device assigned to the user with a user data key (310) that is unique to the user, wherein, in particular, a user ID (302) of the user is stored on the server in association with the encrypted user data (306); and storing the user ID and a user-specific user data decryption key (310) required for decrypting the encrypted user data in the user device, wherein the user data key and/or the user data decryption key (310) are preferably stored in a protected manner and are not accessible to the server;
- during a check-in (104) of one of the users at one of the venues, generating a trace ID by the user device of the one user, wherein the trace ID is representative of a combination of the user ID of the one user and current time information, and storing the trace ID linked to the venue ID of the one venue on the server (202), wherein the trace ID is stored as an access key,
- receiving (106) a message containing the user ID of one of the users with regard to whom the tracing is to be performed;
- generating (108) candidate trace IDs for the user identified in the message, wherein the candidate trace IDs are possible past trace IDs that lie within a predetermined past time period,
- use (110) of the generated candidate trace IDs as search criteria by the server (202) to find corresponding trace IDs stored on the server for the past period, so that the venue IDs (502) of those venues at which the user to be traced checked in during the past period are determined, and
- outputting (112) the venue IDs of the determined venues by the server (202) for identifying those venues which the user to be traced has visited during the past period.

2. An electronic system (200) with user devices (204), each of which is assigned to a user (240), and with at least one server (202) for tracing users who are temporarily present in different spatial areas, hereinafter referred to as venues, for an entity (246), wherein the electronic system is configured to perform the following steps:
- upon registration (102): storing user data (300) of the users on the server (202), wherein the user data is encrypted with a user data key (310) that is individual for the respective user, wherein, in particular, a user ID (302) of the relevant user is stored on the server in association with the encrypted user data (306); and storing the user IDs and a user-specific user data decryption key (310) required for decrypting the encrypted user data in each case in the user device assigned to the user, wherein the user data key and/or the user data decryption key (310) is preferably stored in a protected manner in the user device of the respective user and is not accessible to the server; during a check-in (104) of one of the users at one of the venues, generating a trace ID by the user device of the one user, wherein the trace ID is representative of a combination of the user ID of the one user and current time information, and storing the trace ID linked to the venue ID of the one venue on the server (202), wherein the trace ID is stored as an access key,
- receiving (106) a message containing the user ID of one of the users for whom the tracing is to be performed;
- generating (108) candidate trace IDs for the user identified in the message, wherein the candidate trace IDs are possible past trace IDs that lie within a predetermined past time period;
- using (110) the generated candidate trace IDs as search criteria by the server (202) to find corresponding trace IDs stored on the server for the past period, so that the venue IDs of those venues at which the user to be traced checked in during the past period are determined, and
- outputting (112) the venue IDs of the determined venues for identifying those venues that the user to be tracked has visited during the past period by the server (202).

3. The method or electronic system according to any one of the preceding claims,
- wherein the trace ID generated during check-in is calculated by means of a derivation function as a function of the combination of the user ID to be represented and the current time information to be represented at check-in; and
- wherein each of the candidate trace IDs is calculated by means of the same derivation function as a function of the combination of the user ID contained in the message and one of the candidate time information for a possible check-in time in the past,
- wherein the derivation function preferably comprises rounding the current or candidate time information to times within predefined time intervals, wherein the time intervals are in particular minutes or hours or days.

4. The method or electronic system according to claim 3,
- wherein the user device is configured to generate a random value (userTracingSecret, 410) during registration with the server and to store it in an access-protected manner exclusively in the user device;
- wherein the derivation function calculates the trace ID as a function of the random value (410);
- wherein the user device is configured to obtain confirmation from the user to whom it is assigned for the transmission of the random value, and only in response to the confirmation to transmit the random value to the server or to an entity device of the entity in order to enable the server or the entity to derive the candidate trace IDs.

5. The method or electronic system according to any one of the preceding claims,
- wherein the user device is configured to generate a random value (412), hereinafter referred to as userVerificationSecret, during registration with the server and to store it in an access-protected manner exclusively in the user device;
- wherein the user device is configured to use the userVerificationSecret during check-in with the venue to calculate a verification data value (414) as a function of the trace ID (406) and the encrypted ID data (408) and to store it on the server linked to the trace ID.

6. The method or electronic system according to any one of the preceding claims,
- wherein the entity is assigned an asymmetric cryptographic key pair with a public entity key and a private entity key,
- wherein the user device of the user is configured to additionally encrypt the user data already encrypted with the user data key (310) with the public entity key (332) during registration, and/or
- wherein the user's user device is configured, during check-in, to encrypt the user data decryption key (310) required for decrypting this encrypted user data (306) using the public entity key together with the user ID of the user in order to generate encrypted ID data (408), and is configured to store the encrypted ID data (408, 506) in the course of the check-in linked to the venue ID (502) of the one venue on the server (202).

7. The method or electronic system according to claim **6,** further comprising at least the entity device (208) assigned to the entity,
- wherein the entity device is configured to generate, one after the other, a plurality of asymmetric cryptographic key pairs assigned to the entity, wherein, upon generation of a new entity key pair, the previously valid one is invalidated, wherein each of the entity key pairs is assigned a key version number,
- wherein the at least one entity device is configured to transmit the public key of the generated and currently valid key pair to the user devices;
- wherein the user devices are configured to always use only the most recently transmitted entity key (332) to generate the multiply encrypted user data and/or to generate the encrypted ID data (408), wherein in particular:
- the entity device (208) comprises a plurality of entity devices,
- wherein the entity device which generates the current asymmetric entity key pair is configured to send the private key of the currently generated asymmetric entity key pair in encrypted form to all other entity devices via a network,
- wherein each of the entity devices is configured to generate an emergency message relating to one of the users and to send it to the server (202) and/or configured to decrypt the encrypted user data and/or the encrypted ID data (408) using one of the previously received private entity keys.

8. The method or electronic system according to any one of the preceding claims, further comprising a plurality of venue devices (206), wherein one or more of the venue devices are assigned to each of the venues,
wherein, in particular:
the user performs the check-in with one of the venue devices (206), wherein the one venue device includes a communication interface for direct communication with the user device of a checking-in user, wherein the user device and the one venue device are configured such that, during the check-in, the following occurs:
- generation of the check-in data packet (400, 420, 430) by the user device, wherein the check-in data packet encodes the trace ID (406) and preferably also the encrypted ID data (408);
- transmission of the check-in data packet from the user device to the venue device via the direct communication interface;
- decoding of the received check-in data packet by the venue device to obtain the trace ID and preferably also the ID data (408) of the checking-in user and storing these on the server (202) linked to the venue ID of the venue to which the venue device is assigned;
- wherein the direct communication link is preferably a near-field communication link or optical data transmission, wherein the optical data transmission comprises detection by an optical sensor of the venue device of a graphic code encoding the check-in data packet and displayed on a display of the user device.

9. The method or electronic system according to any one of the preceding claims, further comprising at least one carrier object with a graphical venue code of one of the venues, wherein the venue code encodes a venue ID under which this venue is registered with the server (202), wherein the user checks in to this venue, wherein the user device is configured such that the following occurs during the check-in:
- capturing of the graphical venue code with a camera of the user device;
- decoding of the venue code by the user device to obtain the venue ID of the venue;
- wherein the trace ID and preferably also the identification data (408) of the checking-in user are transmitted to the server by the user device, wherein the decoded venue ID linked to the trace ID is also stored on the server (202);
- wherein the venue code is preferably a QR code or barcode that is visible on a label or digital display, and/or wherein the carrier object is preferably a table display or a door sign for a vehicle, vehicle compartment, building or room.

10. The method or electronic system according to any one of the preceding claims, wherein the electronic system is configured such that a check-out time is transmitted to the server from the user device of one of the users or from the venue device when the user leaves the venue, wherein the check-out time is assigned to the trace ID previously received for this user, so that the period of time spent by the user at the venue is stored.

11. The method or electronic system according to any one of the preceding claims, wherein the system is configured to:
- generate a tracing secret transfer object by the user device, wherein the tracing secret transfer object contains at least the user ID and the user data decryption key of the user to whom the user device is assigned;
- encrypt the tracing secret transfer object with a public key (332) of the entity by the user device, wherein the public key is in particular the most recent of a series of consecutively generated public entity keys;
- transmit the encrypted tracing secret transfer object via a network from the user device to the server;
- in response to receiving the encrypted tracing secret transfer object, generating a TAN and storing the TAN linked to the encrypted tracing secret transfer object by the server, and issuing the TAN to the user;
- receive the TAN from the user via an entity device (208) assigned to the entity;
- use the TAN by the entity device to receive and decrypt the user's encrypted user data in interoperation with the server.

12. The method or electronic system according to claim 11, wherein using the TAN comprises:
- sending a first user data request containing the TAN to the server (202) by the entity device (208);
- receiving a first user data request containing the TAN by the server (202) from the entity device;
- in response to receiving the user data request, sending the encrypted tracing secret transfer object stored in association with the TAN from the server to the entity device;
- decrypting the tracing secret transfer object with the private entity key corresponding to the public key (332) of the entity to obtain the user ID and the user data decryption key (310) required for decrypting the encrypted user data of the user, by the entity device; and
- using the decrypted user ID to generate a second user data request containing the user ID and sending it from the entity device to the server; and
- receiving the encrypted user data of the user from the server by the entity device in response to the second request; and
- decrypting the encrypted user data by means of the user data decryption key contained in the tracing secret transfer object by the entity device.

13. The method or electronic system according to any one of the preceding claims, wherein the trace IDs do not contain the user ID in plain text, and wherein the trace IDs are generated in such a way that it cannot be determined on the basis of an analysis of the trace IDs stored on the server alone whether two trace IDs represent the same or different user IDs.

14. The method or electronic system according to any one of the preceding claims,
- wherein the user device is configured to detect its own current position during the check-in process;
- wherein the system is configured to check during the check-in whether the user device is within a maximum distance from the position of the venue and to perform the check-in only if the user device is within the maximum distance from the position of the venue.

15. The method or electronic system according to claim 14, wherein the detection of the current position of the user device is selected from its group comprising:
- capturing GPS data from the user device using a GPS sensor on the user device, whereby the GPS data determined by the user device is compared with position data from the venue to determine whether the user device is within the maximum distance from the venue at the time of check-in;
- capturing a WLAN identifier of a locally available WLAN network, wherein the WLAN identifier determined by the user device serves as proof during the check when determining whether the user device is within the maximum distance from the venue during check-in;
- detecting a near-field signal from a near-field signal transmitter of the venue, wherein the near-field signal encodes an identifier of the venue, and wherein the identifier of the venue determined by the user device via the near-field signal serves as proof during the check when the user device is within the maximum distance from the venue during check-in.

16. A computer program, computer program product or digital storage medium with program instructions executable by a processor of a data processing device for performing those steps for which one or more components of the electronic system according to any one of preceding claims 8-15 is configured, wherein these system components are the user device or the server (202) or the venue device or the entity device.

## Revendications

1. Procédé de suivi d'utilisateurs qui sont temporairement situés dans des zones d'espace différentes, appelées « salles » dans la suite, pour une entité (246), en particulier dans des scénarios d'urgence tels qu'une pandémie, dans lequel le procédé comprend les étapes consistant à :
- lors de l'enregistrement (102) de chacun des utilisateurs : stocker des données utilisateur (306) de l'utilisateur sur un serveur (202), dans lequel les données utilisateur (314) sont chiffrées par un dispositif utilisateur attribué à l'utilisateur avec une clé de données utilisateur (310) qui est unique pour l'utilisateur, dans lequel, en particulier, un ID utilisateur (302) de l'utilisateur est stocké sur le serveur en association avec les données utilisateur chiffrées (306) ; et stocker l'ID utilisateur et une clé de déchiffrement de données utilisateur spécifique de l'utilisateur (310) exigée pour déchiffrer les données utilisateur chiffrées dans le dispositif utilisateur, dans lequel la clé de données utilisateur et/ou la clé de déchiffrement de données utilisateur (310) sont de préférence stockées de manière protégée et ne sont pas accessibles au serveur ;
- pendant une vérification d'entrée (104) de l'un des utilisateurs au niveau de l'une des salles, faire générer un ID de suivi par le dispositif utilisateur de l'utilisateur concerné, dans lequel l'ID de suivi est représentatif d'une combinaison de l'ID utilisateur de l'utilisateur concerné et des informations de date et d'heure actuelles, et stocker l'ID de suivi lié à l'ID de salle de la salle concernée sur le serveur (202), dans lequel l'ID de suivi est stocké en tant que clé d'accès,
- recevoir (106) un message contenant l'ID utilisateur de l'un des utilisateurs concernant la personne pour laquelle le suivi doit être réalisé ;
- générer (108) des ID de suivi candidats pour l'utilisateur identifié dans le message, dans lequel les ID de suivi candidats sont d'éventuels ID de suivi passés qui se trouvent au sein d'une période de temps passée prédéterminée,
- faire utiliser (110) les ID de suivi candidats générés en tant que critères de recherche par le serveur (202) pour trouver les ID de suivi correspondants stockés sur le serveur pour la période passée, de telle sorte que les ID de salle (502) pour les salles au niveau desquelles l'utilisateur à suivre a été vérifié en entrée pendant la période passée sont déterminés, et
- faire renvoyer (112) les ID de salle des salles déterminées par le serveur (202) pour identifier les salles que l'utilisateur à suivre a visitées pendant la période passée.

2. Système électronique (200) avec des dispositifs utilisateurs (204), chacun étant attribué à un utilisateur (240), et avec au moins un serveur (202) pour suivre les utilisateurs qui sont temporairement présents dans différentes zones d'espace, appelées « salles » dans la suite, pour une entité (246), dans lequel le système électronique est configuré pour réaliser les étapes suivantes consistant à :
- lors de l'enregistrement (102) : stocker les données utilisateur (300) des utilisateurs sur le serveur (202), dans lequel les données utilisateur sont chiffrées avec une clé de données utilisateur (310) qui est individuelle pour l'utilisateur respectif, dans lequel, en particulier, un ID utilisateur (302) de l'utilisateur correspondant est stocké sur le serveur en association avec les données utilisateurs chiffrées (306) ; et stocker les ID utilisateur et une clé de déchiffrement de données utilisateur spécifique de l'utilisateur (310) exigée pour déchiffrer les données utilisateur chiffrées dans chaque cas dans le dispositif utilisateur attribué à l'utilisateur, dans lequel la clé de données utilisateur et/ou la clé de déchiffrement de données utilisateur (310) sont de préférence stockées de manière protégée dans le dispositif utilisateur de l'utilisateur respectif et ne sont pas accessibles au serveur ; pendant une vérification d'entrée (104) de l'un des utilisateurs au niveau de l'une des salles, faire générer un ID de suivi par le dispositif utilisateur de l'utilisateur concerné, dans lequel l'ID de suivi est représentatif d'une combinaison de l'ID utilisateur de l'utilisateur concerné et des informations de date et d'heure actuelles, et stocker l'ID de suivi lié à l'ID de salle de la salle concernée sur le serveur (202), dans lequel l'ID de suivi est stocké en tant que clé d'accès,
- recevoir (106) un message contenant l'ID utilisateur de l'un des utilisateurs pour la personne pour laquelle le suivi doit être réalisé ;
- générer (108) des ID de suivi candidats pour l'utilisateur identifié dans le message, dans lequel les ID de suivi candidats sont d'éventuels ID de suivi passés qui se trouvent au sein d'une période de temps passée prédéterminée ;
- faire utiliser (110) les ID de suivi candidats générés en tant que critères de recherche par le serveur (202) pour trouver les ID de suivi correspondants stockés sur le serveur pour la période passée, de telle sorte que les ID de salle pour les salles au niveau desquelles l'utilisateur à suivre a été vérifié en entrée pendant la période passée sont déterminés, et
- faire renvoyer (112) les ID de salle des salles déterminées pour identifier les salles que l'utilisateur à suivre a visitées pendant la période passée par le serveur (202).

3. Procédé ou système électronique selon l'une quelconque des revendications précédentes,
- dans lequel l'ID de suivi généré pendant la vérification d'entrée est calculé au moyen d'une fonction de dérivation en tant que fonction de la combinaison de l'ID utilisateur à représenter et des informations de date et d'heure actuelles à représenter au niveau de la vérification d'entrée ; et
- dans lequel chacun des ID de suivi candidats est calculé au moyen de la même fonction de dérivation en tant que fonction de la combinaison de l'ID utilisateur contenu dans le message et de l'une des informations de date et d'heure candidates pour une date et une heure de vérification d'entrée éventuelles dans le passé,
- dans lequel la fonction de dérivation comprend de préférence une étape consistant à arrondir les informations de date et d'heure actuelles ou candidates à des dates et des heures au sein d'intervalles de temps prédéfinis, dans lequel les intervalles de temps sont en particulier des minutes, des heures ou des jours.

4. Procédé ou système électronique selon la revendication 3,
- dans lequel le dispositif utilisateur est configuré pour générer une valeur aléatoire (userTracingSecret, 410) pendant l'enregistrement auprès du serveur et pour la stocker d'une manière protégée en accès exclusivement dans le dispositif utilisateur ;
- dans lequel la fonction de dérivation calcule les ID de suivi en tant que fonction de la valeur aléatoire (410) ;
- dans lequel le dispositif utilisateur est configuré pour obtenir une confirmation auprès de l'utilisateur auquel il est attribué pour la transmission de la valeur aléatoire, et uniquement en réponse à la confirmation visant à transmettre la valeur aléatoire au serveur ou à un dispositif d'entité de l'entité afin de permettre au serveur ou à l'entité de dériver les ID de suivi candidats.

5. Procédé ou système électronique selon l'une quelconque des revendications précédentes,
- dans lequel le dispositif utilisateur est configuré pour générer une valeur aléatoire (412), appelée « userVerificationSecret » dans la suite, pendant l'enregistrement auprès du serveur et pour la stocker d'une manière protégée en accès exclusivement dans le dispositif utilisateur ;
- dans lequel le dispositif utilisateur est configuré pour utiliser l'userVerificationSecret pendant la vérification d'entrée auprès de la salle afin de calculer une valeur de données de vérification (414) en tant que fonction de l'ID de suivi (406) et des données d'ID chiffrées (408) et pour la stocker sur le serveur lié à l'ID de suivi.

6. Procédé ou système électronique selon l'une quelconque des revendications précédentes,
- dans lequel l'entité se voit attribuer une paire de clés cryptographiques asymétriques avec une clé d'entité publique et une clé d'entité privée,
- dans lequel le dispositif utilisateur de l'utilisateur est configuré pour chiffrer de plus les données utilisateur déjà chiffrées avec la clé de données utilisateur (310) avec la clé d'entité publique (332) pendant l'enregistrement, et/ou
- dans lequel le dispositif utilisateur de l'utilisateur est configuré, pendant la vérification d'entrée, pour chiffrer la clé de déchiffrement de données utilisateur (310) exigée pour déchiffrer lesdites données utilisateur chiffrées (306) en utilisant la clé d'entité publique conjointement avec l'ID utilisateur de l'utilisateur afin de générer des données d'ID chiffrées (408), et est configuré pour stocker les données d'ID chiffrées (408, 506) au cours de la vérification d'entrée liée à l'ID de salle (502) de la salle concernée sur le serveur (202).

7. Procédé ou système électronique selon la revendication 6, comprenant en outre au moins le dispositif d'entité (208) attribué à l'entité,
- dans lequel le dispositif d'entité est configuré pour générer, l'une après l'autre, une pluralité de paires de clés cryptographiques asymétriques attribuées à l'entité, dans lequel, lors de la génération d'une nouvelle paire de clés d'entité, la paire précédemment valide est invalidée, dans lequel chacune des paires de clés d'entité se voit attribuer un numéro de version de clé,
- dans lequel l'au moins un dispositif d'entité est configuré pour transmettre la clé publique de la paire de clés générée et actuellement valide aux dispositifs utilisateurs ;
- dans lequel les dispositifs utilisateur sont configurés pour toujours utiliser uniquement la clé d'entité la plus récemment transmise (332) afin de générer les données utilisateur à chiffrage multiple et/ou pour générer les données d'ID chiffrées (408), dans lequel en particulier :
- le dispositif d'entité (208) comprend une pluralité de dispositifs d'entité,
- dans lequel le dispositif d'entité qui génère la paire de clés d'entité asymétriques actuelle est configuré pour envoyer la clé privée de la paire de clés d'entité asymétriques actuellement générée sous forme chiffrée à tous les autres dispositifs d'entité par l'intermédiaire d'un réseau,
- dans lequel chacun des dispositifs d'entité est configuré pour générer un message d'urgence lié à l'un des utilisateurs et pour l'envoyer au serveur (202) et/ou configuré pour déchiffrer les données utilisateur chiffrées et/ou les données d'ID chiffrées (408) en utilisant l'une des clés d'entité privées précédemment reçues.

8. Procédé ou système électronique selon l'une quelconque des revendications précédentes, comprenant en outre une pluralité de dispositifs de salle (206), dans lequel un ou plusieurs des dispositifs de salle sont attribués à chacune des salles,
dans lequel, en particulier :
l'utilisateur réalise la vérification d'entrée avec l'un des dispositifs de salle (206), dans lequel le dispositif de salle concerné inclut une interface de communication pour communication directe avec le dispositif utilisateur d'un utilisateur de vérification d'entrée, dans lequel le dispositif utilisateur et le dispositif de salle concerné sont configurés de telle sorte que, pendant la vérification d'entrée, ce qui suit se produit :
- génération du paquet de données de vérification d'entrée (400, 420, 430) par le dispositif utilisateur, dans lequel le paquet de données de vérification d'entrée code pour l'ID de suivi (406) et de préférence également les données d'ID chiffrées (408) ;
- transmission du paquet de données de vérification d'entrée depuis le dispositif utilisateur au dispositif de salle par l'intermédiaire de l'interface de communication directe ;
- décodage du paquet de données de vérification d'entrée reçu par le dispositif de salle pour obtenir l'ID de suivi et de préférence également les données d'ID (408) de l'utilisateur effectuant la vérification d'entrée et le stockage de ceux-ci sur le serveur (202) liée à l'ID de salle de la salle à laquelle le dispositif de salle est attribué ;
- dans lequel le lien de communication directe est de préférence un lien de communication de champ proche ou une transmission de données optiques, dans lequel la transmission de données optiques comprend la détection par un capteur optique du dispositif de salle d'un code graphique codant pour le paquet de données de vérification d'entrée et affiché sur un affichage du dispositif utilisateur.

9. Procédé ou système électronique selon l'une quelconque des revendications précédentes, comprenant en outre au moins un objet de support avec un code de salle graphique de l'une des salles, dans lequel le code de salle code pour un ID de salle sous lequel ladite salle est enregistrée auprès du serveur (202), dans lequel l'utilisateur effectue une vérification d'entrée pour ladite salle, dans lequel le dispositif utilisateur est configuré de telle sorte que ce qui suit se produit pendant la vérification d'entrée :
- capture du code de salle graphique avec une caméra du dispositif utilisateur ;
- décodage du code de salle par le dispositif utilisateur pour obtenir l'ID de salle de la salle ;
- dans lequel l'ID de suivi et de préférence également les données d'identification (408) de l'utilisateur effectuant la vérification d'entrée sont transmis au serveur par le dispositif utilisateur, dans lequel l'ID de salle décodé lié à l'ID de suivi est également stocké sur le serveur (202) ;
- dans lequel le code de salle est de préférence un QR code ou un code-barres qui est visible sur une étiquette ou un affichage numérique, et/ou dans lequel l'objet de support est de préférence un affichage de table ou un panneau de porte pour un véhicule, un compartiment de véhicule, un bâtiment ou une pièce.

10. Procédé ou système électronique selon l'une quelconque des revendications précédentes, dans lequel le système électronique est configuré de telle sorte qu'une date et une heure de vérification de sortie sont transmises au serveur depuis le dispositif utilisateur de l'un des utilisateurs ou depuis le dispositif de salle lorsque l'utilisateur quitte la salle, dans lequel la date et l'heure de vérification de sortie sont attribuées à l'ID de suivi précédemment reçu pour cet utilisateur, de telle sorte que la période de temps passé par l'utilisateur au niveau de la salle est stockée.

11. Procédé ou système électronique selon l'une quelconque des revendications précédentes, dans lequel le système est configuré pour :
- faire générer un objet de transfert de secret de suivi par le dispositif utilisateur, dans lequel l'objet de transfert de secret de suivi contient au moins l'ID utilisateur et la clé de déchiffrement de données utilisateur de l'utilisateur auquel le dispositif utilisateur est attribué ;
- faire chiffrer l'objet de transfert de secret de suivi avec une clé publique (332) de l'entité par le dispositif utilisateur, dans lequel la clé publique est en particulier la plus récente d'une série de clés d'entité publiques générées de manière consécutive ;
- transmettre l'objet de transfert de secret de suivi chiffré par l'intermédiaire d'un réseau depuis le dispositif utilisateur au serveur ;
- en réponse à la réception de l'objet de transfert de secret de suivi chiffré, faire générer un TAN et stocker le TAN lié à l'objet de transfert de secret de suivi chiffré par le serveur, et délivrer le TAN à l'utilisateur ;
- recevoir le TAN de la part de l'utilisateur par l'intermédiaire d'un dispositif d'entité (208) attribué à l'entité ;
- faire utiliser le TAN par le dispositif d'entité pour recevoir et déchiffrer les données utilisateur chiffrées de l'utilisateur en interfonctionnement avec le serveur.

12. Procédé ou système électronique selon la
revendication 11, dans lequel l'étape consistant à utiliser le TAN comprend les étapes consistant à :
- faire envoyer une première requête de données utilisateur contenant le TAN au serveur (202) par le dispositif d'entité (208) ;
- faire recevoir une première requête de données utilisateur contenant le TAN par le serveur (202) depuis le dispositif d'entité ;
- en réponse à l'étape consistant à faire recevoir la requête de données utilisateur, envoyer l'objet de transfert de secret de suivi chiffré stocké en association avec le TAN depuis le serveur au dispositif d'entité ;
- faire déchiffrer l'objet de transfert de secret de suivi avec la clé d'entité privée correspondant à la clé publique (332) de l'entité pour obtenir l'ID utilisateur et la clé de déchiffrement de données utilisateur (310) exigés pour déchiffrer les données utilisateur chiffrées de l'utilisateur, par le dispositif d'entité ; et
- utiliser l'ID utilisateur déchiffré pour générer une seconde requête de données utilisateur contenant l'ID utilisateur et l'envoyer depuis le dispositif d'entité au serveur ; et
- faire recevoir les données utilisateur chiffrées de l'utilisateur depuis le serveur par le dispositif d'entité en réponse à la seconde requête ; et
- faire déchiffrer les données utilisateur chiffrées au moyen de la clé de déchiffrement de données utilisateur contenue dans l'objet de transfert de secret de suivi par le dispositif d'entité.

13. Procédé ou système électronique selon l'une quelconque des revendications précédentes, dans lequel les ID de suivi ne contiennent pas l'ID utilisateur en texte clair, et dans lequel les ID de suivi sont générés d'une manière telle qu'il ne peut pas être déterminé sur la base d'une analyse des ID de suivi stockés sur le serveur seul si deux ID de suivi représentent le même ID utilisateur ou des ID utilisateur différents.

14. Procédé ou système électronique selon l'une quelconque des revendications précédentes,
- dans lequel le dispositif utilisateur est configuré pour détecter sa propre position actuelle pendant le processus de vérification d'entrée ;
- dans lequel le système est configuré pour vérifier pendant la vérification d'entrée si le dispositif utilisateur est à moins d'une distance maximale de la position de la salle et pour réaliser la vérification d'entrée uniquement si le dispositif utilisateur est à moins de la distance maximale depuis la position de la salle.

15. Procédé ou système électronique selon la
revendication 14, dans lequel la détection de la position actuelle du dispositif utilisateur est choisie dans son groupe comprenant :
- capturer des données GPS depuis le dispositif utilisateur en utilisant un capteur GPS sur le dispositif utilisateur, moyennant quoi les données GPS déterminées par le dispositif utilisateur sont comparées à des données de position provenant de la salle pour déterminer si le dispositif utilisateur est à moins de la distance maximale depuis la salle au moment de la vérification d'entrée ;
- capturer un identifiant WLAN d'un réseau WLAN disponible localement, dans lequel l'identifiant WLAN déterminé par le dispositif utilisateur sert de preuve pendant la vérification lors de l'étape consistant à déterminer si le dispositif utilisateur est à moins de la distance maximale depuis la salle pendant la vérification d'entrée ;
- détecter un signal de champ proche depuis un émetteur de signal de champ proche de la salle, dans lequel le signal de champ proche code pour un identifiant de la salle, et dans lequel l'identifiant de la salle déterminé par le dispositif utilisateur par l'intermédiaire du signal de champ proche sert de preuve pendant la vérification lorsque le dispositif utilisateur est à moins de la distance maximale depuis la salle pendant la vérification d'entrée.

16. Programme informatique, produit de programme informatique ou support de stockage numérique avec des instructions de programme exécutables par un processeur d'un dispositif de traitement de données destiné à réaliser les étapes pour lesquels un ou plusieurs constituants du système électronique selon l'une quelconque des revendications 8 à 15 précédentes sont configurés, dans lequel les constituants de système sont le dispositif utilisateur ou le serveur (202) ou le dispositif de salle ou le dispositif d'entité.
